# EUROPEAN PATENT SPECIFICATION

(11) **EP 0 311 378 B1**
(45) Date of publication and mention of the grant of the patent: **27.04.1994**
(21) Application number: 88309284.3
(22) Date of filing: 05.10.1988
(51) Int. Cl.: C07D 241/44, C07D 403/12, A61K 31/495

(54) **6-Substituted alkoxy-2-oxo-1, 2-dihydroquinoxaline derivatives**
6-Substituierte Alkoxy-2-oxo-1,2-dihydro-chinoxalin-Derivate
Dérivés de 2-oxo-1,2-dihydro-quinoxaline substitués en position 6 par alkoxy substitué

(30) Priority: 05.10.1987 JP 251264/87; 24.08.1988 JP 210346/88
(43) Date of publication of application: 12.04.1989
(73) Proprietor: ASAHI KASEI KOGYO KABUSHIKI KAISHA, Osaka (JP)
(72) Inventor: Suzuki, Yukio, Tagata-gun Shizuoka-ken (JP); Yaso, Masao, Tagata-gun Shizuoka-ken (JP); Nishimura, Katumi, Mishima-shi Shizuoka-ken (JP); Saeki, Kenji, Tagata-gun Shizuoka-ken (JP); Takayanagi, Noriyasu, Sunto-gun Shizuoka-ken (JP); Saito, Tetsu, Mishima-shi Shizuoka-ken (JP); Hayashi, Eiichi, Shizuoka-shi Shizuoka-ken (JP)
(74) Representative: Woods, Geoffrey Corlett

(56) References cited:
- EP-A- 0 019 918

## Description

This invention relates to compounds having platelet aggregation inhibitory activity and/or cyclic AMP/phosphodiesterase inhibitory activity which are useful in the treatment of thrombosis or a circulatory condition, to processes for their preparation, to compositions containing them, to such compounds for use in a method of therapeutic treatment and to their use in the manufacture of a medicament.

Known quinoxaline derivatives having antithrombotic activity are: 1-diethylaminoethyl-2-oxo-3-(benzyl or substituted benzyl)-1,2-dihydroxyquinoxaline (JP-B-46-11183 and JP-A-56-97226); 1-non-substituted or lower alkyl-3-substituted carbamoyloxymethyl-2-oxo-1,2-dihydroquinoxaline (JP-A-49-24981); and N²⁻(2,3-diox- oquinoxaline-6-yl)sulfonyl-L-arginineamide derivatives (JP-A-54-100342).

Among pharmacologically active 2-oxoquinoxaline derivatives, in which the benzene ring side is substituted by substituted alkoxy, which are useful in the treatment of cardiac or circulatory diseases, is 5-or 8-[2-hydroxy-3-(substituted alkylamino- or substituted piperidino-)-propoxy]-3-nonsubstituted-, methyl- or hydroxymethyl-2-oxo-1,2-dihydroquinoxaline (JP-A-55-162783).

It is of great importance to find excellent pharmaceuticals having more potential activities.

We have found certain 3-non-substituted, alkyl or phenyl-6-substituted alkoxy-2-oxo-1,2-dihydroquinox- alines having platelet aggregation inhibitory activity and/or phosphodiesterase inhibitory activity with pharmaceutically interesting properties.

The present invention therefore provides a compound which is a 6-substituted alkoxy-2-oxo-1,2-dihydroquinoxaline derivative of formula (I): 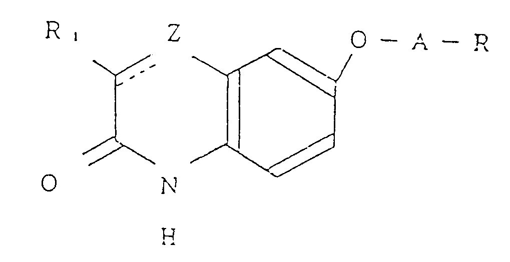 wherein
Z is N and --- is a double bond or Z is NH and --- is a single bond,
R₁ is hydrogen, branched or unbranched C₁ -₂₀ alkyl, unsubstituted phenyl or phenyl substituted by at least one substituent selected from C₁₋₃ alkyl, halogen, nitro and C₁-C₆ alkoxy,
A is C₁-C₆ alkylene, and
R is carboxyl, (C1-C6 alkoxy)carbonyl, 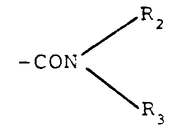 or 1-cycloalkyl-tetrazole-5-yl, wherein R₂ is C₁-C₆ alkyl, hydroxy-C₁-C₆ alkyl or phenyl-C₁-C₆ alkyl, in which the phenyl is unsubstituted or substituted by at least one substituent selected from C₁-C₃ alkyl, halogen, nitro and C₁-C₆ alkoxy, and R₃ is C₁-C₆ alkyl or C₅-C₇ cycloalkyl, or R₂ and R₃, together with the nitrogen to which they are attached, form 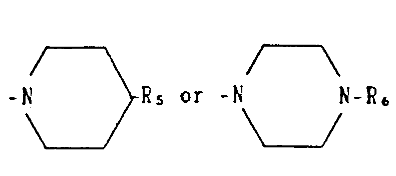 wherein R₅ and R₆ are hydrogen, phenyl or phenyl substituted by at least one substituent selected from C₁-C₃ alkyl, halogen, nitro and C₁-C₆ alkoxy,

or a pharmacologically acceptable non-toxic salt thereof.

The present invention also provides a process for the preparation of a compound of formula (I), or a pharmacologically acceptable non-toxic salt thereof, which comprises:
i) when Z is NH, --- is a single bond and R is (C₁-C₆ alkoxy)carbonyl or 1-cycloalkyl-tetrazole-5-yl, reducing a compound of formula [2]: 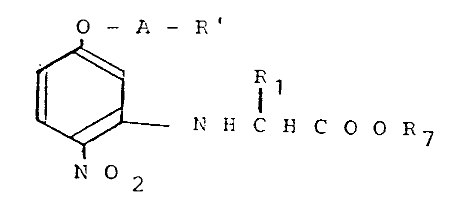 wherein R' is (C₁-C₆ alkoxy)carbonyl or 1-cycloalkyl-tetrazole-5-yl, A and R₁ are as defined above and R₇ is C₁-C₆ alkyl, to convert the nitro group thereof to an amino group, and cyclizing the product thus obtained;
ii) when Z is N, --- is a double bond and R is (Cₗ-C₆alkoxy)carbonyl or 1-cycloalkyl-tetrazole-5-yl, oxidizing a compound of formula [3]: 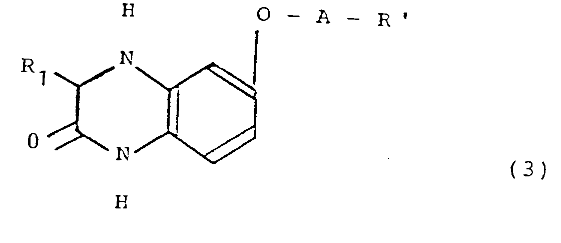 wherein R' is (C₁-C₆alkoxy)carbonyl or 1-cycloalkyl-tetrazole-5-yl and A and R₁ are as defined above;
iii) when Z is N, --- is a double bond and R is carboxyl, de-esterifying a compound of formula [1 a']: 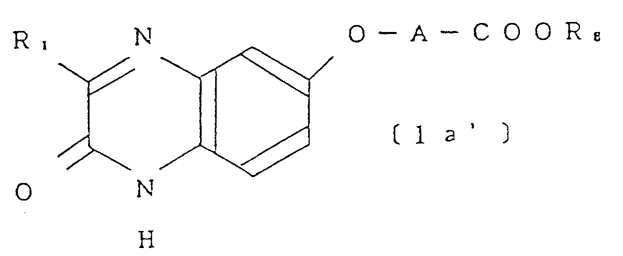 wherein R₈ is C₁-C₆alkyl and A and R₁ are as defined above;
iv) when Z is N, --- is a double bond and R is 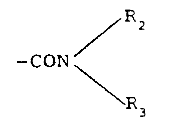 wherein R₂ and R₃ are as defined above, forming an acid halide or acid anhydride of a compound or formula [1 b]: 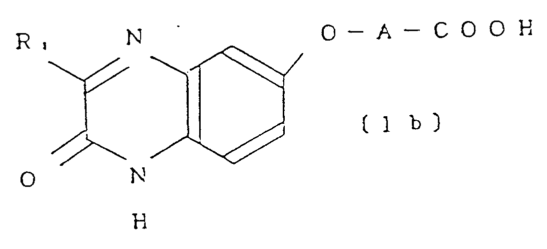 wherein A and R₁ are as defined above and reacting the compound thus formed with an amine of formula [6]: 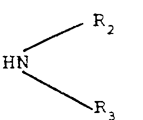 wherein R₂ and R₃ are as defined above; or reducing a compound of formula [7]: 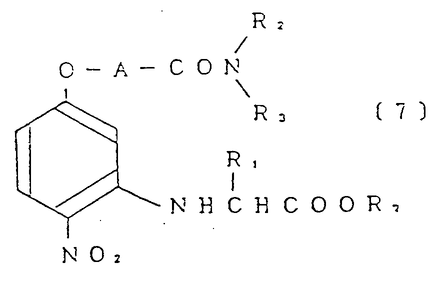 wherein A, R₁ , R₂ and R₃ are as defined above and R₇ is C₁-C₆ alkyl, cyclizing the product thus obtained and oxidizing the product obtained by the cyclization;
v) when Z is NH, --- is a single bond and R is carboxyl, de-esterifying a compound of formula [1d']: 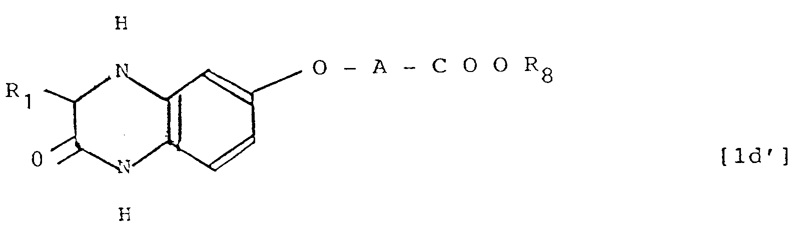 wherein A and R₁ are as defined above and R₈ is C₁-C₆ alkyl; or
vi) when Z is NH, --- is a single bond and R is 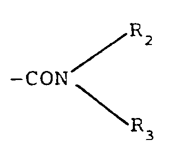 wherein R₂ and R₃ are as defined above, forming an acid halide or acid anhydride of a compound of formula [1 e]: 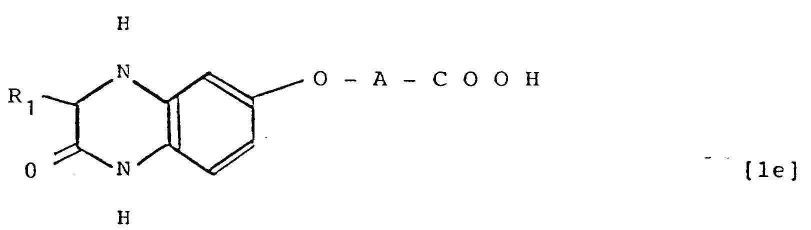 wherein A and R₁ are as defined above and reacting the compound thus formed with an amine of formula [6]: 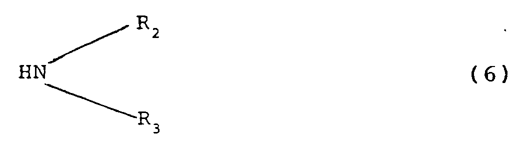 wherein R₂ and R₃ are as defined above; or
   reducing a compound of formula [7] as defined above and cyclizing the product thus obtained; and if desired converting the resultant compound of formula [I] obtained by any of processes i) to vi) into a pharmacologically acceptable non-toxic salt thereof.

The present invention additionally provides a pharmaceutical composition comprising a compound of formula (I), or a pharmacologically acceptable non-toxic salt thereof, and a pharmaceutically acceptable carrier or diluent.

The present invention further provides a compound of formula (I), or a pharmacologically acceptable non-toxic salt thereof, for use in a method of treatment of the human or animal body by therapy, in particular for use in a method of treatment of thrombosis or a circulatory condition.

The present invention yet further provides the use of a compound of formula (I), or a pharmacologically acceptable non-toxic salt thereof, in the manufacture of a medicament for the treatment of thrombosis or a circulatory condition.

Examples of pharmacologically acceptable non-toxic salts are salts of inorganic acids such as hydrochloride, sulfate or phosphate salts, and salts of organic acids such as acetate, propionate, butyrate, glycolate, gluconate, malate, tartrate, succinate, mandelate, glutamate, aspartate, methanesulfonate or toluenesulfonate salts.

A C₁ -C₆ group has from 1 to 6 carbon atoms, preferably from 1 to 4 carbon atoms.

The compound (1) can be produced by the following process.

### Process A:

A process for production of compound (1) wherein Z is N, ------ is a double bond and R is (C₁-C₆ alkoxy)carbonyl or 1-cycloalkyl-tetrazole-5-yl (hereinafter designated a compound [1a]) of the formula 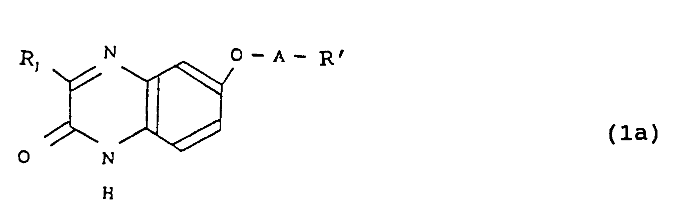 wherein R' is (Cᵢ -C₆ alkoxy) carbonyl or 1-cycloalkyl-tetrazole-5-yl:

The compound [1 a] can be produced by reducing and cyclizing a compound of formula (2): 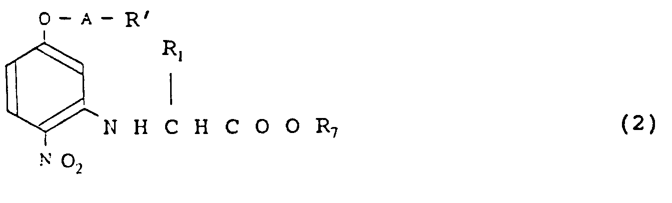 wherein R₇ is C₁-C₆ alkyl, and A, R₁ and R' are as defined above to obtain a compound of the formula 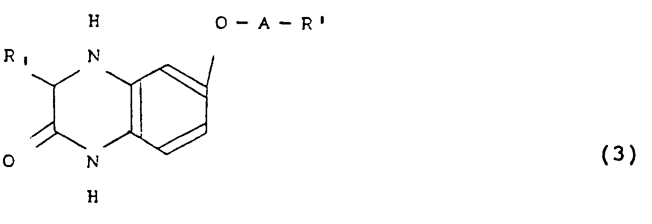 wherein Ri, R' and A are as defined above, which is then oxidized with an oxidizing agent.

In the compound (2) above, R₁ is hydrogen, branched or unbranched C₁ -₂o alkyl (eg methyl, ethyl, propyl, isopropyl, butyl, isobutyl, sec-butyl, t-butyl, pentyl, isopentyl, hexyl, heptyl, octyl, nonyl, decyl, undecyl, dodecyl, tridecyl, tetradecyl, pentadecyl or hexadecyl), unsubstituted phenyl or phenyl substituted by at least one substituent selected from C₁₋₃ alkyl, halogen, nitro and C₁-C₆ alkoxy.

A is C₁ -₆ alkylene, which is optionally branched. Examples are methylene, ethylene, methylmethylene, propylene, isobutylene or pentylene.

In the compound (2), R¹ is (Cₗ-C₆ alkoxy)-carbonyl or 1-cycloalkyl-tetrazole-5-yl. The C₁-C₆ alkoxy can be C₁ -₄ alkoxy such as methoxy, ethoxy, propoxy, isopropoxy, butoxy, isobutoxy, sec-butoxy or t-butoxy. The C₅-₇ cycloalkyl is preferably cyclohexyl. R₇ is C₁-C₆ alkyl, preferably an optionally branched C1-4 alkyl such as methyl or ethyl.

The compound (2) is a novel compound and can be produced by O-alkylating a compound of the formula 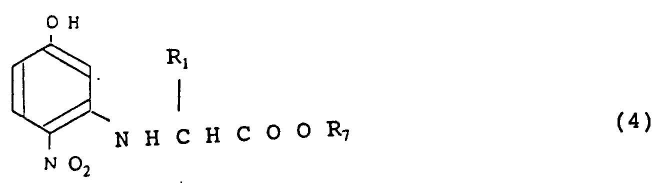 wherein R₁ and R₇ are as hereinbefore defined, with a halide of the formula 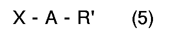 wherein X is halogen, and A and R' are as hereinbefore defined, in a reaction medium.

The compound (4) may be prepared by a process in which a commercially available 3-fluoro-4-nitrophenol is reacted with an a-amino acid of the formula 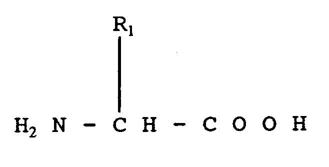 wherein R₁ is as hereinbefore defined, in the presence of an alkali, for example an alkaline hydrogen carbonate such as sodium hydrogen carbonate or an alkaline carbonate such as sodium carbonate or potassium carbonate, under reflux with water containing a Ci-Ce alkanol such as methanol or ethanol. Thereafter the reaction mixture is neutralized with hydrochloric acid to remove the reaction solvent, dried, a C₁ -C₆ alkanol is added thereto, and esterified by adding thionyl chloride.
Isolation of the product (4) can be performed by filtering out insoluble material, removing the reaction solvent, thereafter dissolving the residue with a water immiscible organic solvent such as chloroform, drying, removing the solvent and further purifying by column chromatography using silica-gel. The thus obtained compound (4) is a novel compound.

A and R' in the halide (5) are as hereinbefore defined. X in formula (5) is a halogen such as chlorine or bromine.

O-alkylation of the compound (4) with the halide (5) is preferably performed by dissolving the compound (4) in an alkanol solution, such as a derivative ethanol with an alkaline metal such as metallic sodium, and distilling off the ethanol to activate the hydroxyl group.

An example of a reaction solvent for the O-alkylation is dimethyl formamide.

The O-alkylation reaction preferably proceeds under heating. The reaction can be terminated when the maximum production of the compound (2) is achieved.

Isolation of the product (2) can be performed by distilling the reaction solvent, dissolving the residue in a water immiscible organic solvent such as chloroform, washing with diluted aqueous alkali, drying the organic layer and distilling off the solvent. Further purification can be performed by silica-gel chromatography using an elution solvent mixture such as benzene-ethyl acetate.

Reduction and cyclization of the nitrobenzene derivative (2) can be performed by dissolving the compound (2) in a reaction solvent, for example a C₁-C₆ alkanol such as ethanol, adding therein a reducing agent, which reduces nitro group to amino, such as excess amount of iron powder, and adding hydrogen chloride/Ci -C₆ alkanol under heating to complete the intramolecular cyclization.

Isolation of the product (3) can be performed by removing insoluble material, if any, by filtration, distilling off the reaction solvent, dissolving the residue in a water immiscible organic solvent such as chloroform, washing with a dilute aqueous alkaline, and removing the solvent after drying. In the above isolation procedure, part of the product (3) is oxidized and compound (1 a) thus obtained is mixed in the product. A product (3) can be isolated and purified by silica-gel chromatography using an elution solvent such as chloroform-methanol. However, in order to obtain the compound (1a), product (3) mixed with the objective compound (1 a) can be treated with a preferred oxidizing agent to obtain the product (1 a) without any purification procedure. For example, an impure product (3) dissolved in benzene is heated, after adding dichlorodicyano benzoquinone, until the product (3) is consumed, and if required an insoluble material is performed under heating, then solvent is removed off and the residue is isolated and purified by silica-gel chromatography using an elution solvent of chloroform-methanol to obtain the product (1 a).

Another method of oxidation of the product (3) is oxidation by aqueous hydrogen peroxide, in which the product (3) is oxidized in an ethanol solution.

In the above reduction-cyclization reaction, when the reaction mixture is filtered using a filter-aid and the concentrated filtrate solution is left as it is, ferric chloride generated by a reaction of iron powder and hydrogen chloride acts as an oxidizing agent for the product (3), then all the product (3) is converted to the product (1 a), which can be purified by the same procedure as defined hereinabove.

Another method of reduction and cyclization is that a compound (2) dissolved in a C₁ -C₆ alkanol such as ethanol is subjected to catalytic hydrogenation with a Pd/C catalyst. The reaction proceeds in general at room temperature. The product (3) can be obtained by removing the catalyst and reaction solvent, thereafter purifying by the same procedure as defined above. The product (1a) can be obtained by oxidizing the product (3).

### Process B:

A process for production of compound (1) wherein Z is N, ------ is double bond, and R is carboxyl, of the formula (1 b): 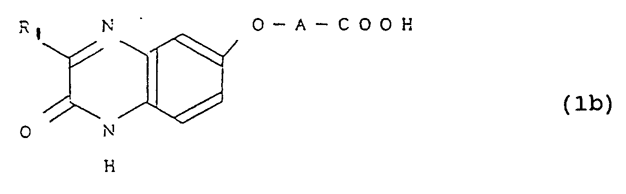 wherein R₁ and A are as defined above.

The compound (1 b) can be obtained by deesterifying a compound of formula (1 a) in which R' is (C₁-C₆ alkoxy)carbonyl (hereinafter designated compound (1a')) of formula: 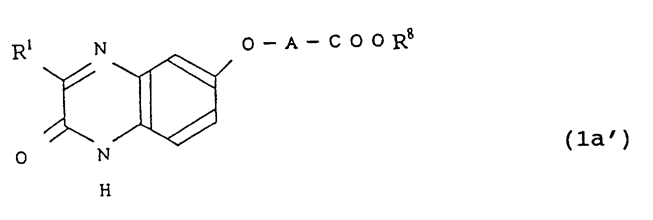 wherein R₈ is C₁ -C₆ alkyl and R₁ and A are as defined above.

In the above de-esterification the compound (1 a') dissolved in a C₁-C₆ alkanol is treated with an alkaline hydroxide such as KOH or NaOH. The reaction proceeds at room temperature and can be terminated when the compound (1 a') has been consumed.

Isolation of the product (1b) can be made by adding water to the reaction mixture, acidifying with hydrochloric acid, filtering the precipitated crystals, washing with water and drying.

### Process C:

A process for production of compound (1) wherein Z is N, ------- is double bond, and R is 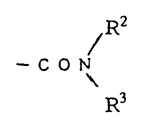 of the formula (1 c): 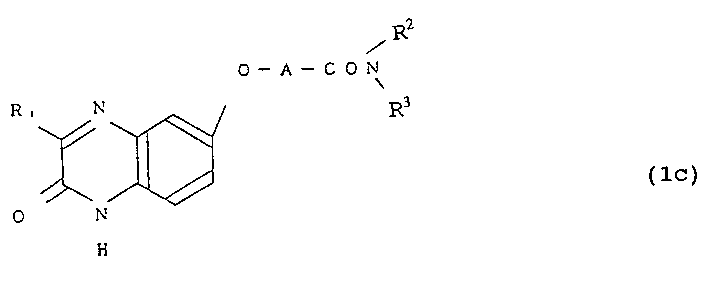 wherein Rᵢ, R₂, R₃ and A are as defined above.

The compound (1 c) can be produced by forming an acid halide or anhydride of the compound (1 b) and reacting the compound thus formed with an amine of the formula 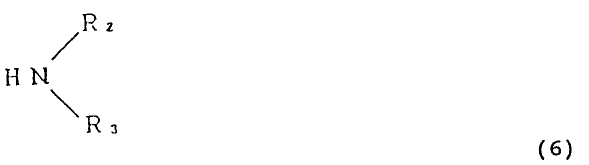 wherein R₂ and R₃ are as defined above.

R₂ in the amine (6) is a C₁-C₆ alkyl, hydroxy C₁-C₆ alkyl or unsubstituted or substituted phenyl-C₁-C₆ alkyl, and R₃ is C₁-C₆ alkyl or C₅-C₇ cycloalkyl, or R₂ and R₃, together with the nitrogen to which they are attached, form 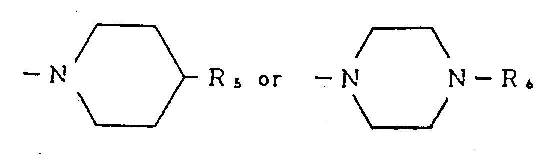 wherein R₅ and R₆ are hydrogen or unsubstituted or substituted phenyl.

Examples of C₁-C₆ alkyl are optionally branched C₁₋₄ alkyl such as methyl, ethyl, propyl, isopropyl, butyl, isobutyl, sec-butyl or t-butyl. Example of hydroxy C₁-C₆ alkyl is a hydroxy alkyl in which the alkyl is optionally substituted C₁₋₄ alkyl, such as hydroxymethyl, 2-hydroxyethyl, 1-hydroxyethyl, 3-hydroxypropyl, 2-hydroxypropyl or 1-hydroxypropyl. The substituted phenyl in the phenyl-C₁-C₆ alkyl is a phenyl which has at least one substituent selected from C₁₋₃ alkyl, halogen, nitro and C₁-C₆ alkoxy.

The substituted phenyl represented by R₅ and R₆ is a phenyl which has at least one substituent selected from C₁₋₃ alkyl, halogen, nitro and C₁-C₆ alkoxy.

Examples of the amine (6) are diethylamine, N-methyl-cyclohexylamine, N-ethyl-cyclohexylamine, N-2-hydroxyethyl-cyclohexylamine, N-benzyl-cyclohexylamine, piperidine, 4-phenylpiperidine, piperazine, 4-phenyl-piperazine or 4-(o-, m- or p-methoxyphenyl) piperazine.

An acid halide of the compound (1 b) can be produced by a known halogenation with known halogenating agent. Examples of a halogenating agent are SOCI₂, PCls, POCI₃ or SOBr₂. The halogenation can be performed in a reaction solvent such as chloroform.

An acid anhydride of a compound (1b) can be produced by a reacting a compound (1b) with a carboxylic acid halide such as pivaloyl chloride in a reaction solvent such as tetrahydrofuran or dimenthyl- formamide in the presence of a tertiary organic amine such as triethylamine to form a mixed anhydride with a compound (1 b) and a carboxylic acid.

The thus formed compound (1 b) is converted to a reactive derivative and said derivative is reacted with the above amine (6). The reaction proceeds in general under ice-cooling or at room temperature. Isolation of the product can be made by mixing with a water immiscible or organic solvent, washing with a dilute alkaline solution, drying, removing the solvent and subjecting the residue to silica-gel column chromatography using an elution system of chloroform-methanol.

Another method of synthesis of the compound (1 c) is as follows.

A compound of formula (7): 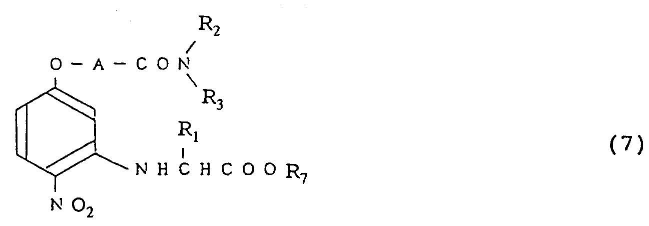 wherein Rᵢ, R₂, R₃, R₇ and A are as hereinbefore defined is reduced, subjected to cyclization, and then oxidized with an oxidizing agent.

The compound (7) can be produced by O-alkylating the compound (4) with a halide of formula (8): 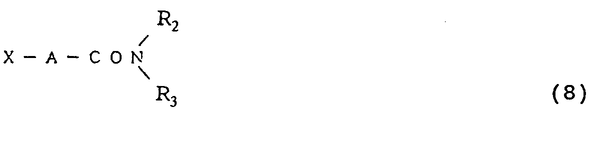 wherein R₂, R₃, A and X are as hereinbefore defined, in a reaction medium.

The above reduction, cyclization and oxidation can be made by the same way as in process (A).

### Process D:

A process for production of the compound (1) wherein Z is NH, ------ is a single bond and R is (C₁-C₆ alkoxy)carbonyl or 1-cycloalkyl-tetrazole-5-yl, of the formula (1d): 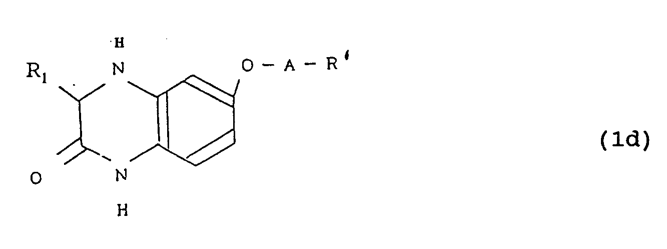 wherein R' is (C₁-C₆ alkoxy)carbonyl or 1-cycloalkyl-tetrazol-5-yl, and R₁ and A are as hereinbefore defined.

The above compound (1 d) can be obtained by isolating and purifying the compound (3).

### Process E:

A process for production of the compound (1) wherein Z is NH, ----- is a single bond and R is carboxyl, of the formula (1e): 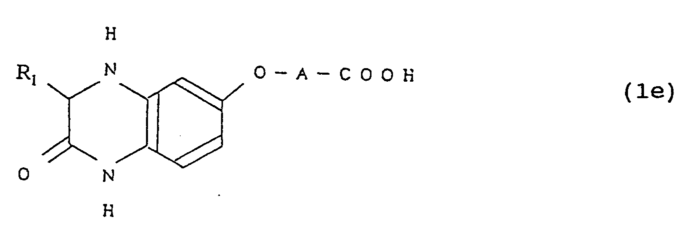 wherein R₁ and A are as hereinbefore defined.

The above compound (1e) can be obtained by de-esterifying a compound of formula (1d) in which R' is a (Ci -C₆ alkoxy)carbonyl. This is represented by the following compound (1 d'): 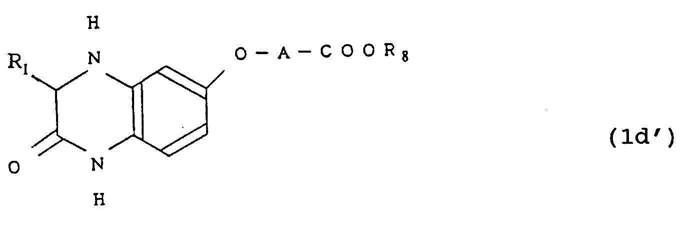 wherein R₈, R₁ and A are as hereinbefore defined.

The above de-esterification is performed by treating the compound (1 d') dissolved in a C₁-C₆ alkanol with an alkaline hydroxide such as KOH or NaOH. The reaction proceeds at room temperature and can be terminated when the compound (1d') has been consumed.

Isolation of the product (1e) can be made by adding water to the reaction mixture, acidifying with hydrochloric acid, filtering the precipitated crystals, washing with water and drying.

### Process F:

A process for production of compound (1) wherein Z is NH, ----- is a single bond, and R is 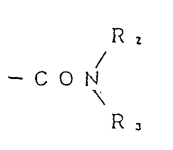 of formula (1f): 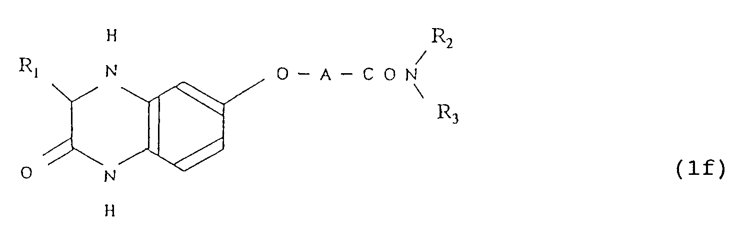 wherein Rᵢ, R₂, R₃ and A are as hereinbefore defined:

A compound (1f) can be produced by forming an acid halide or anhydride of the compound (1e) and reacting the compound thus formed with an amine (6).

The reaction conditions and isolation of the compound (1f) can be the same as in process (C). Another method of production of the compound (1f) is reducing the compound (7) and subjecting the compound thus obtained to cyclization.

The reduction and cyclization can be the same as in process (A).

Examples of the compound of the present invention are illustrated in Table 3.

The pharmacological activity of the compound (1) is illustrated below.

### (1) Inhibitory action on cyclic AMP/phosphodiesterase:

A mixture of 700µI 2mM MgC1₂/40mM Tris-buffer (pH 7.5), 100µl enzyme solution (a crude enzyme fraction obtained from sonicated rabbit platelets), and 100µl test sample solution (compound dissolved in 50% aqueous methanol) is incubated at 30 ° C for 5 minutes. 100µl (50µg) 3', 5'-cyclic AMP is added thereto and further incubated for 40 minutes. The reaction is stopped by heating at 100_{°}C for 5 minutes. The amount of 5'-AMP in the supernatant obtained at 3000 rpm for 5 minutes is measured using HPLC.

The results are shown in Table 1. As shown in the table, the compounds of the present invention have cyclic AMP/phosphodiesterase inhibitory activity at 1 - 10µg/ml.

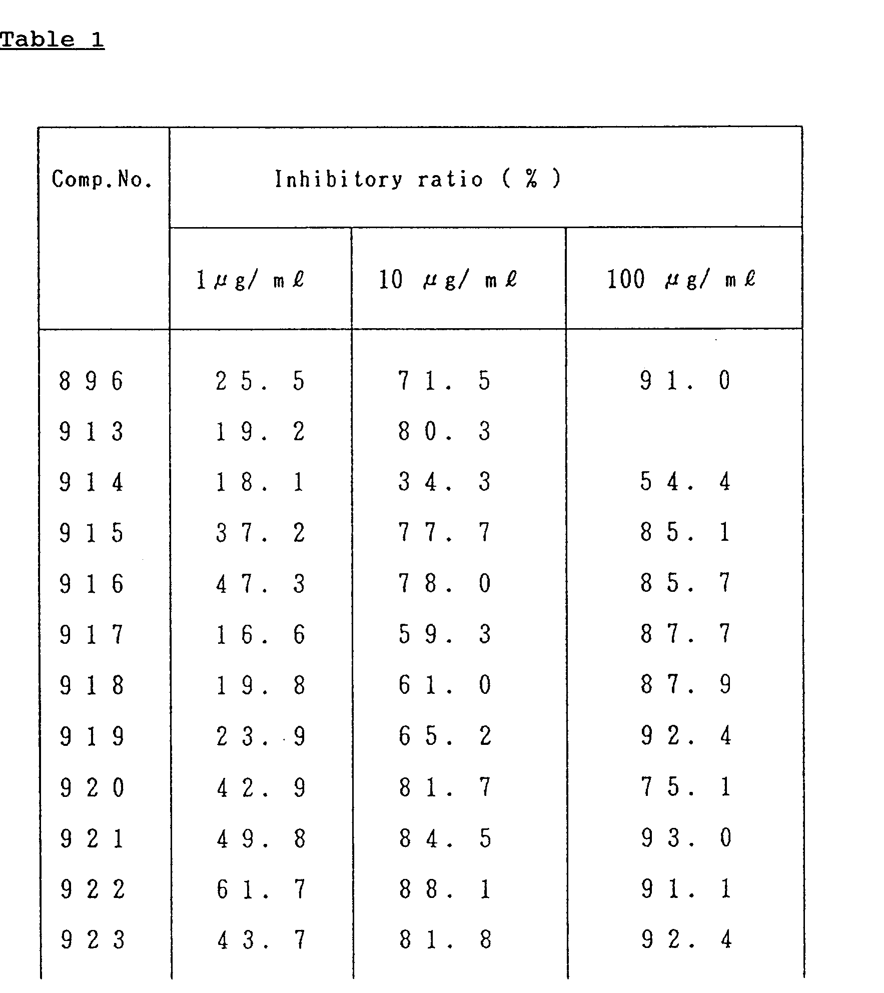

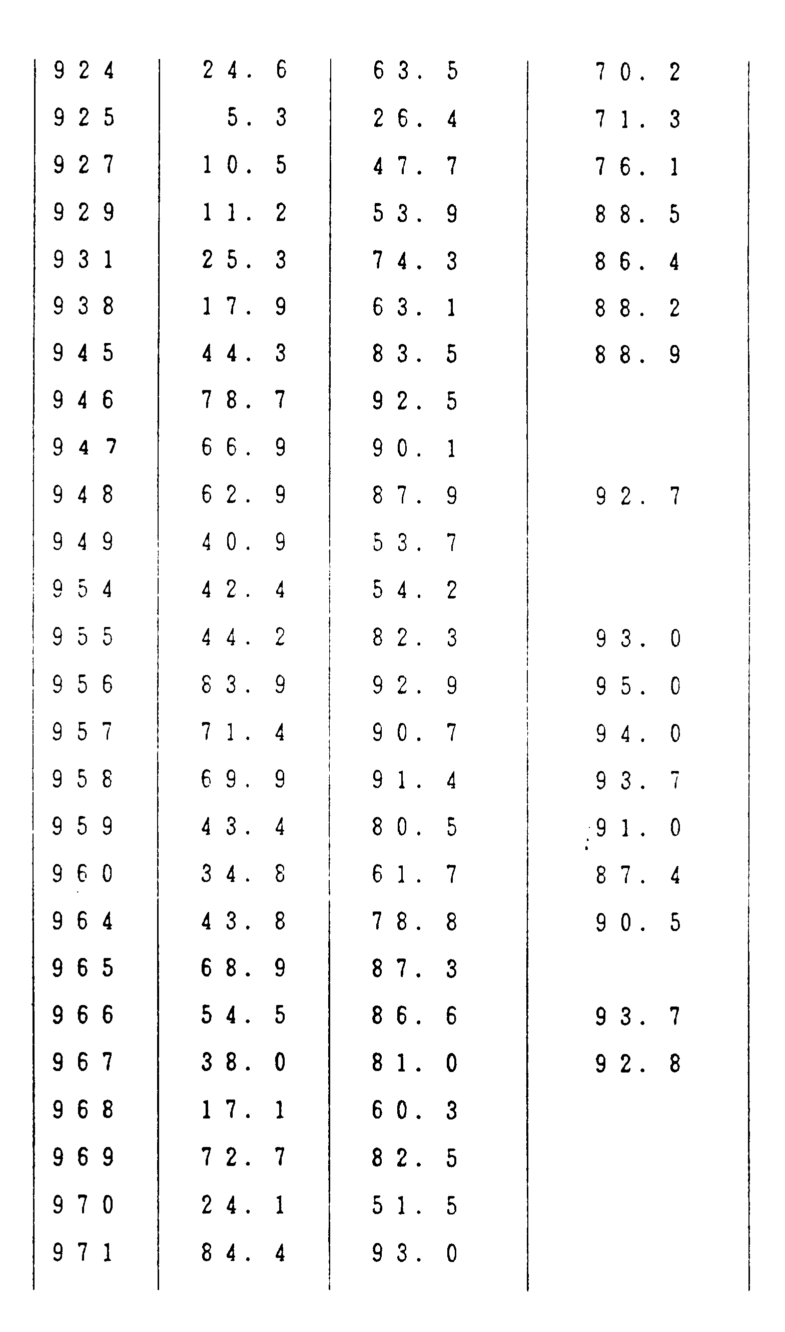

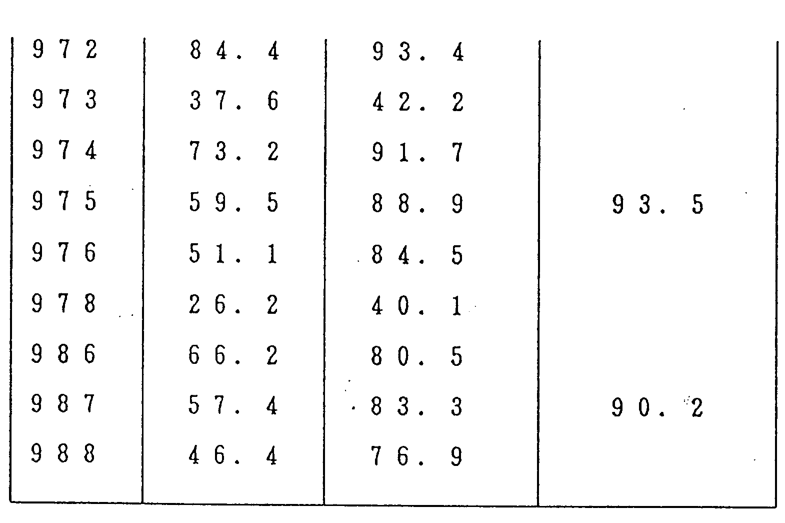

### (2) Platelet aggregation inhibition:

A rabbit platelet rich plasma (5 X 10⁵/mm³), to which was added 1/10 volume of a 3.8% sodium citrate solution (216µℓ) is incubated in an aggregometer at 37°C, 1000 rpm, for 3 minutes. l2µℓ test sample solution (final concentration 10µ.M) is added thereto and further incubated for 3 minutes, and 2.5µg/mℓ collagen (aggregation agent), 2.5 µM ADP, 0.05µg/mℓ platelet activating factor (PAF) or 12µl 0.25 mM arachidonic acid (A.A.) is added thereto as an aggregating agent, and the platelet aggregation activity is measured.

The results are shown in Table 2. As shown in the table, the compounds of the present invention have clear platelet aggregation inhibitory activity at 10µM.

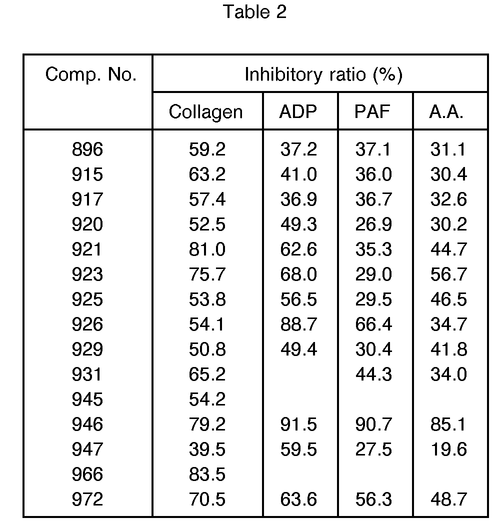

As explained hereinabove a compound (1) of the present invention or its salt has a platelet aggregation inhibitory activity and/or cyclic AMP/phosphodiesterase inhibitory action, and is useful as an antithrombotic drug and circulatory drug.

The following Examples further illustrate the present invention:

### Examples:

In the examples, the Rf of the silica-gel thin layer chromatography (TLC) is, if not specified, measured using the following carrier and developing solvent.
Carrier: silica-gel, Kieselgel 60 F₂s₄, Art 5715 (Merck)
Developer:
   a; chloroform - methanol (20 : 1)
   b; chloroform - methanol (10:1)
   c; benzene - ethyl acetate (5 : 1 )

The physical properties of the compounds (1) obtained in the following examples are shown in Tables 12 and 13.

### Referential examples 1 - 10:

### N-(5-hydroxy-2-nitrophenyl)-amino acid ethyl ester:

30 mM 3-fluoro-4-nitrophenol, 50 mM a-amino acid and 100 mM sodium hydrogen carbonate were added to a mixture of 80 m1 ethanol and 20 m1 water and refluxed. The reaction was terminated by checking the disappearance of a spot of phenol compound on silica-gel thin layer chromatography (TLC). The reaction mixture was acidified by adding 6N HCI, concentrated in vacuo, and then the residue was dried completely. 100 m ethanol was added thereto, 5 ml thionyl chloride was added dropwise under ice-cooling, then the mixture was stirred at room temperature overnight. Insoluble material was removed by filtration and the filtrate was concentrated in vacuo. The residue, dissolved in chloroform, was washed with water, dried by adding anhydrous magnesium sulfate and concentrated in vacuo. The residue was charged on a column of silica-gel (WAKO Pure Chem. Co., Wakogel C-200) packed with benzene and eluted with benzene-ethyl acetate (50 : 1) to obtain N-(5-hydroxy-2-nitrophenyl)-amino acid ethyl ester.

The reaction scale (based on 3-fluoro-4-nitrophenol), amino acid used, reflux time and amount and yield of the product are shown in Table 4.

The physical properties of N-(5-hydroxy-2-nitrophenyl)-amino acid ethyl ester are shown in Table 5.

### Referential examples 11 - 20:

### N-(5-(3-ethoxycarbonyl) propoxy-2-nitrophenyl)--amino acid ethyl ester:

10 mM N-(5-hydroxy-2-nitrophenyl)-amino acid ethyl ester obtained in Referential examples 1-10 was added to and dissolved in 10 mM metallic sodium (0.23 g) dissolved in 50 m1 ethanol, and the solvent was distilled off in vacuo. 100 m dimethylformamide and 1.95g y-bromobutyric acid ethyl ester were added to the residue, and stirred at 100 ° C. The solvent was distilled off in vacuo and the residue was dissolved in chloroform. The chloroform solution was washed with dilute aqueous sodium carbonate. The aqueous layer was extracted with chloroform. The chloroform layers were combined, dried by adding anhydrous sodium sulfate and concentrated in vacuo. The residue was charged on a column of silica-gel (C-200) packed with benzene, and eluted with benzene-ethyl acetate (50 : 1) to obtain the product.

The reaction scale (based on N-(5-hydroxy-2-nitrophenol)-amino acid used), reflux time, amount of silica-gel used,and amount and yield of the product are shown in Table 6.

The physical properties of N-(5-(3-ethoxycarbonyl) propoxy-2-nitrophenyl)-amino acid ethyl ester are shown in Table 7.

### Examples 1 - 10:

### 3-substituted-6-(3-ethyoxycarbonylpropoxy)-2-oxo-1,2-dihydroquinoxaline:

Iron powder (4-5 equivalents amount) was added to N-(5-(3-ethyoxycarbonyl)propoxy-2-nitrophenyl)-amino acid ethyl ester (a starting material) dissolved in ethanol. Approx. 5-N HCI/ethanol was added dropwise slowly under reflux conditions and reacted for 3 hours. Insoluble material was removed by filtration, and the filtrate was washed with ethanol. The combined solution (filtrate and washing) was concentrated in vacuo. The residue, dissolved in chloroform, was washed with water, and the aqueous layer was again extracted with chloroform. The combined chloroform layer was washed with dilute aqueous sodium carbonate, dried with anhydrous sodium sulfate and concentrated in vacuo to obtain a residue which was a mixture of the above product and 3-substituted-6-(3-ethoxycarbonyl-propoxy)-2-oxo-1,2,3,4-tetrahydroquinoxaline. An equimolar amount of dichlorodicyano benzoquinone (DDQ) was added to the residue dissolved in benzene, and refluxed for 3-4 hours. The reaction mixture was filtered hot to remove insoluble materials, the filtered material was completely washed with benzene, and then concentrated in vacuo. The residue was charged on a column of silica-gel (C-200) packed with chloroform, and purified by eluting with chloroform-methanol (20 : 1) to obtain the product.

In Table 8, the amount of starting material, ethanol used, iron powder used, HCI/ethanol used, DDQ used, silica-gel used, and the compound No. of the product and its yield are given.

### Examples 11-20:

### 3-substituted-6-(3-carboxypropoxy)-2-oxo-1,2-dihydroquinoxaline:

2N-NaOH was added to a suspension of 10 mM of the ester obtained in Referential example 1-10 in 10mt ethanol. 2N-NaOH was added and stirred at room temperature overnight. 30 m water was added to the reaction mixture, which was then adjusted to approximately pH 2 by adding 6N-HCI. The precipitated crystals were filtered, completely washed with water and dried to obtain the product.

In Table 9, the compound No. of the above ester, its amount used, 2N-NaOH used, product No., amount obtained and yield are given.

### Examples 21-47:

### 3-substituted-6-(3-N,N-di-substituted-aminocarbonyl)propoxy)-2-oxo-1, 2-dihydroquinoxaline:

2 mM carboxylic acid obtained in Examples 11-20 was added to 20 mî tetrahydrofuran. An equimolar amount of triethylamine was added thereto, an equimolar amount of pivaloyl chloride was further added dropwise at -10 ° C and stirred at below -10 ° C for 1 hour. An equimolar amount of an amine was added at once to the obtained residue, and stirred for 3 hours, gradually adjusting the temperature to room temperature. Chloroform was added to the reaction mixture, and washed with dilute aqueous potassium carbonate. The aqueous layer was further extracted with chloroform. The combined chloroform layer was dried by adding anhydrous sodium sulfate and concentrated in vacuo. The residue was charged on a column of silica-gel (C-200, 70 g) and eluted with chloroform-methanol to obtain the product. The No. of the carboxylic acid, its amount used, kind of amine, amount used, ratio of solvent system of chloroform-methanol, No. of compound obtained, amount obtained and its yield are shown in Table 10.

### Referential example 21:

### N-(5-hydroxy-2-nitrophenyl)-amino acetic acid methyl ester:

50 g 3-fluoro-4-nitrophenol (0.138 mole), 48 g glycine (0.637 mole) and 107 g sodium hydrogen carbonate (1.27 mole) were added to a mixture of 600 mî ethanol and 100 mî water and refluxed for 5 days. The solvent was distilled off in vacuo. The reaction mixture was acidified by adding dilute HCI and filtered. The filtrate material was washed with dilute HCI and water, then dried in vacuo to obtain N-(5-hydroxy-2-nitrophenyl)-amino acetic acid as a yellow solid. Yield: 66.4 g (yield: 98%).

30.84g thionyl chloride (260 mM) was added dropwise under ice-cooling to 45.78 g N-(hydroxy-2-nitrophenyl)-amino acetic acid (216 moles) dissolved in 500 mî methanol for 30 minutes, then stirred at room temperature for 22 hours. The reaction mixture was filtered, the filtered material was washed with methanol and dried to obtain a yellow solid product. The above filtrate was concentrated in vacuo and the precipitated material was washed with chloroform and dried. The solid material thus obtained was combined to obtain the product 47.5 g (Yield: 97%).
NMR (DMSO - d₆) 6 (ppm) ; 3.71 (s, 3H, CH₃), 4.20 (d, 2H, CH₂), 6.04 - 6.29 (m, 2H, phenyl proton), 8.01 (d, 1 H, phenyl proton), 8.50 (t, 1 H, NH)
Mass (CI) ; 227 (M⁺ + 1)
TLC ; Rf_{b} = 0.52

### Referential example 22:

### N-(5-(3-ethoxycarbonyl) propoxy-2-nitrophenyl)-amino acetic acid methyl ester:

8.31 N-(5-hydroxy-2-nitrophenyl)-amino acetate methyl ester (37 mM) was dissolved in metallic 0.85 g sodium (37 mM) dissolved in 100 mî methanol, and the solvent was distilled off in vacuo. The residue was dissolved in 80 mî dimethylformamide, 5.3 ml y- bromo butyric acid ethyl ester (37 mM) was added to the residue, and stirred at 60 ° C for 14 hours. The solvent was distilled off in vacuo. The residue was charged on a column of silica-gel (C-200) packed with chloroform and eluted with chloroform to obtain the product as yellow crystals.
Yield: 10.76 g (yield: 86%)
   NMR (CDCℓ₃) δ (ppm) ; 1.27 (t, 3H, CH3), 2.12 (q, 2H, CH2), 2.51 (t, 2H, CH2), 3.83 (s, 3H, CH3), 4.00 - 4.28 (m, 6H, 3 CH₃), 6.02 - 6.34 (m, 2H, phenyl proton), 8.16 (d, 1 H, phenyl proton), 8.60 (t, 1 H, NH)
   Mass (CI) ; 341 (M⁺ + 1)
   TLC ; Rf_{c} = 0.44

### Example 48:

### 6-(3-ethoxycarbonylpropoxy)-2-oxo-1,2,3,4-tetrahydroquinoxaline (compound 925):

2.42 g N-(5-(3-ethoxycarbonyl) propoxy-2-nitrophenyl) amino acetic acid methyl ester (7.1 mM) and 7.26 g iron powder were added to 100 m ethanol.

Approximately 5-N HCI/ethanol solution was added dropwise slowly under reflux. The yellow colour of the solution changed to colourless generating hydrogen gas. HCI/ethanol solution was added until the starting material was consumed (observed by noting the change of the reaction mixture to colourless). The reaction mixture was poured into chloroform-water by decantation. The organic layer was washed with aqueous sodium hydrogen carbonate and water, dried with anhydrous sodium sulfate and concentrated in vacuo. The residue was charged on a column of silica-gel (C-200) packed with chloroform, and purified by eluting with chloroform-methanol (100:1) to obtain the compound 925.
Yield: 1.4 g (yield : 70%)

### Example 49:

### 6-(3-ethoxycarbonylpropoxy)-2-oxo-1, 2-dihydroquinoxaline (compound 914):

4.5 g dichlorodicyanobenzoquinone (DDQ) (19.8 mM) was added to the 5 g compound 925 (18 mM) dissolved in 300 mℓ benzene by heating, and refluxed for 3 hours. The reaction mixture was filtered hot and then the filtrate was concentrated in vacuo. The residue, dissolved in chloroform, was washed with water, dried by adding anhydrous sodium sulfate and concentrated in vacuo. The residue was charged on a column of silica-gel (C-200) packed with chloroform, and purified by eluting with chloroform-methanol (300 : 1), (200 : 1) and (100 : 1), in this order to obtain the compound 914 as colourless crystals.
Yield: 4 g (yield: 81 %)

### Example 50-54:

### 6-(3-(N,N-di-substituted-aminocarbonyl)-propoxy)-2-oxo-1,2-dihydro-quinoxaline:

6 mi 2N-NaOH was added to 3.6 mM compound 914 (1 g, a starting material) dissolved in 50 mℓ 50% aqueous ethanol and stirred at room temperature for 1-1.5 hours. Dilute HCI was added to the reaction mixture to adjust the pH to acidic, and the solvent was distilled off in vacuo. The residue was dried in vacuo to obtain crude 6-(3-carboxypropoxy)-2-oxo-1,2,-dihydroquinoxaline (compound 938).

50 mℓ dimethylformamide and 1 mℓ triethylamine were added thereto, 0.45 mℓ pivaloyl chloride was added dropwise and stirred at room temperature for 2 hours. 4.3 mM amine was added to the reaction mixture and stirred at room temperature for 15 hours. The reaction mixture was concentrated in vacuo and chloroform was added thereto. Insoluble material was removed by filtration and the filtrate was concentrated in vacuo. The residue was charged on a column of silica-gel (C-200) packed with chloroform and eluted with chloroform-methanol (100 : 1) and (50 : 1) in this order to obtain the product.

The starting material, kind of amine, No. of compound obtained, amount obtained and its yield are shown in Table 11.

### Referential example 23:

### N-(5-(3-ethoxycarbonyl)butoxy-2-nitrophenyl)-amino acetic acid methyl ester:

15 g N-(5-hydroxy-2-nitrophenyl)-amino acetic acid methyl ester (66.4 mM) was dissolved in 1.83 g metallic sodium (797 mM) dissolved in 100 mî methanol, and the solvent was distilled off in vacuo. The residue was dissolved in 150 mℓ dimethylformamide and 12.6 mℓ y-bromo valeric acid ethyl ester (79.7 mM) was added thereto and stirred at 60 ° C for 20 hours. The solvent was distilled off in vacuo. The residue dissolved in chloroform was filtered through Celite (Tradename) to remove insoluble materials and concentrated in vacuo. The residue was charged on a column of silica-gel (C-200) packed with chloroform and eluted with chloroform to obtain the product as yellow crystals.
Yield: 22.76 g (yield: 97%)
   NMR (CDCℓ₃) 6 (ppm) ; 1.26 (t, 3H, CH₃), 1.6 - 2.0 (m, 4H, 2 CH₂), 2.39 (t, 2H, CH₂), 3.83 (s, 3H, CH₃), 3.9 - 4.3 (m, 6H 3 CH₂), 6.00 (d, 1 H, phenyl proton), 6.27 (d.d, 1 H, phenyl proton), 8.15 (d, 1 H, phenyl proton),
   Mass (CI) ; 355 (M⁺ + 1)

### Example 55:

### 6-(4-ethoxycarbonylpropoxy)-2-oxo-1,2,3,4-tetrahydroquinoxaline (compound 927):

14.1 g N-(5-(4-ethoxycarbonyl) propoxy-2-nitrophenyl) amino acetic acid methyl ester (40 mM) and 42.3 g iron powder were added to 300 m ethanol.

Approximately 5-N HCI/ethanol solution was added dropwise under reflux. The yellow colour of the solution changed to colourless generating hydrogen gas. HCI/ethanol solution was added until the starting material was consumed (observed by the reaction mixture changing to colourless). The reaction mixture was poured into chloroform-water by decantation. The separated organic layer was washed with aqueous sodium hydrogen carbonate and water, dried with anhydrous sodium sulfate and concentrated in vacuo. The residue was charged on a column of silica-gel (C-200) packed with chloroform, and purified by eluting with chloroform-methanol (100 : 1) to obtain compound 927 (colourless crystals).
Yield: 9.8 g (yield: 84%)

### Example 56:

### 6-(4-ethoxycarbonylpropoxy)-2-oxo-1 ,2-dihydroquinoxaline (Compound 919)

7.69 g Dichlorodicyanobenzoquinone (DDQ) (33.9 mM) was added to 9 g compound 927 (30.8 mM) dissolved in 600 m benzene with heating, and refluxed for 3 hours. The reaction mixture was filtered hot, then the filtrate was concentrated in vacuo. The residue, dissolved in chloroform, was washed with water, dried by adding anhydrous sodium sulfate and concentrated in vacuo. The residue was charged on a column of silica-gel (C-200) packed with chloroform, and purified by eluting with chloroform-methanol (300 : 1), (200 : 1) and (100 : 1), in this order to obtain compound 919 as colourless crystals.
Yield: 7.4 g (yield: 83%)

### Examples 57-61:

### 6-(4-(N,N-di-substituted-aminocarbonyl)butoxy)-2-oxo-1,2-dihydro-quinoxaline:

6 mi 2N-NaOH was added to 500 mg compound 919 (1.72 mM, a starting material) dissolved in 50 mℓ 50% aqueous ethanol and stirred at room temperature for 1-1.5 hours. Dilute HCI was added to the reaction mixture to adjust the pH to acidic, and the solvent was distilled off in vacuo. The residue was dried in vacuo to obtain crude 6-(4-carboxybutoxy)-2-oxo-1,2-dihydroquinoxaline.

50 mℓ dimethylformamide and 1 mℓ triethylamine were added thereto, 0.45 mℓ pivaloyl chloride was added dropwise and stirred at room temperature for 2 hours. 4.3 mM amine was added to the reaction mixture and stirred at room temperature for 15 hours. The reaction mixture was concentrated in vacuo and chloroform was added thereto. Insoluble material was removed by filtration and the filtrate was concentrated in vacuo.

The residue was charged on a column of silica-gel (C-200) packed with chloroform and eluted with chloroform-methanol (100 : 1) and (50 :1) in this order to obtain the product.

The starting material, kind of amine, No. of compound obtained, amount and its yield are shown in Table 11.

### Referential example 24:

### N-(5-[3-(N-cyclohexyl-N-methyl-aminocarbonyl)-propoxy-2-nitrophenyl)-amino acetic acid methyl ester:

10 g N-(5-hydroxy-2-nitrophenyl)-amino acetic acid methyl ester (44 mM) was added to 1.02 g metallic sodium (44 mM) dissolved in 100 mî methanol, and the solvent was distilled off in vacuo. The residue was dissolved in 100 mî dimethylformamide and 9.6 g N-cyclohexyl-N-methyl-4-chlorobutaneamide (44 mM) was added thereto and stirred at 100 ° C overnight. The reaction mixture was concentrated in vacuo. The residue, dissolved in chloroform, was washed with water, dried with anhydrous magnesium sulfate and concentrated in vacuo. The residue was charged on a column of silica-gel (C-200) packed with benzene and eluted with benzene-ethyl acetate (5 : 1) and (3 : 1) to obtain the product as yellow crystals.
Yield: 10.3 g (yield: 58%)
   NMR (CDCℓ₃) 6 (ppm) ; 1.0 - 2.0 (m, 10H, 5 CH₂), 2.15 (q, 2H, CH₂), 2.52 (t, 2H, CH₂), 2.84 (s, 3H, CH₃), 3.83 (s, 3H, CH₃), 4.0 - 4.2 (m, 4H, 2 CH₂), 3.4 - 3.7, 4.2 - 4.6 (m, 1 H, CH), 6.10 (d, 1 H, phenyl proton), 6.29 (d.d, 1 H, phenyl proton), 8.16 (d, 1 H, phenyl proton), 8.60 (t, 1 H, NH)
   Mass (CI) ; 408 (M⁺ + 1)
   TLC ; Rf = 0.33 [benzene-ethyl acetate (1 : 1) ]

### Example 62:

### 6-(3-(N-cyclohexyl-N-methyl-aminocarbonyl)-propoxy)-2-oxo-1,2,3,4,-tetrahydro quinoxaline (compound 929):

9g N-(5-{3-(N-cyclohexyl-N-methyl-aminocarbonyl)-propoxy-2-nitrophenyl) amino acetic acid methyl ester (22 mM) and 27 g iron powder were added to 150 mî ethanol.

Approximately 5-N HCI/ethanol solution was added dropwise under reflux. The yellow colour of solution changed to colourless generating hydrogen gas. HCI/ethanol solution was added until the starting material was consumed (observed by the reaction mixture changing to colourless). The reaction mixture was poured into chloroform-aqueous sodium hydrogen carbonate by decantation. The separated organic layer was washed with aqueous sodium hydrogen carbonate and water, dried with anhydrous sodium sulfate and concentrated in vacuo. The residue was charged on a column of silica-gel (C-200) packed with chloroform, and purified by eluting with chloroform-methanol (100 : 1) to obtain compound 929 (colourless crystals). Yield: 7 g (yield: 91 %)

### Referential example 25:

### 5-(4-(1-cyclohexyl-tetrazole-5-yl)butoxy)-2-nitrophenyl-amino acetic acid methyl ester:

5 g N-(5-hydroxy-2-nitrophenyl)-amino acetic acid methyl ester (22 mM) was added to 509 mg metallic sodium (22mM) dissolved in 30 mî methanol, and the solvent was distilled off in vacuo. 100 mî dimethylformamide was added to the residue and 5.36 g 1-chloro-4-(1-cyclohexyl tetrazole-5-yl) butane (22 mM) was added thereto and stirred at 100 ° C for 3 hours. The reaction mixture was concentrated in vacuo and the residue was charged on a column of silica-gel (C-200) packed with benzene and eluted with benzene-ethyl acetate (15 : 1) to obtain the product as yellow crystals.
Yield: 2.9 g (yield: 30%)
   NMR (CDCℓ₃) δ (ppm) ; 1.1 - 2.3 (m, 14H, 7 CH₂), 2.93 (t, 2H, CH₂), 3.82 (s, 3H, CH₃), 3.9 - 4.4 ((m, 5H, 2 CH₂, CH), 5.99 (d, 1 H, phenyl proton), 6.24 (d.d, 1 H, phenyl proton), 8.11 (d, 1 H, phenyl proton), 8.59 (t, 1 H, NH)
   Mass (CI) ; 433 (M⁺ + 1)
   TLC ; Rf_{b} = 0.61 Rf_{c} = 0.05

### Example 63:

### 6-(4-(1-cyclohexyl tetrazole-5-yl)-butoxy)-2-oxo-1,2,3,4-tetrahydro quinoxaline (compound 931

1.9 g N-(5-{4-(1-cyclohexyl tetrazole-5-yl) butoxy}-2-nitrophenyl)amino acetic acid methyl ester (4.4 mM) and 1.9 g 10% Pd/catalyst were added to 500 mℓ ethanol.

The reaction mixture was stirred for 2 hours under bubbling hydrogen gas. The reaction mixture was filtered and the filtrate was concentrated in vacuo. The residue was charged on a column of silica-gel (C-200) packed with chloroform, and purified by eluting with chloroform-methanol (200 : 1) to obtain compound 931, a colourless solid.

### Example 64:

### 6-(4-(1-cyclohexyl-tetrazole-5-yl)-butoxy)-2-oxo-1,2-dihydro quinoxaline (compound 913):

2 g N-(5-{4-(1-cyclohexyl-tetrazole-5-yl) butoxy}-2-nitrophenyl)amino acetic acid methyl ester (4.6 mM) and 6 g iron powder were added to 100 mℓ ethanol.

Approximately 5-N HCI/ethanol solution was added dropwise under reflux until the starting material disappeared on TLC. The reaction mixture was filtered through Celite and the filtrate was concentrated in vacuo. The thus obtained residue was allowed to stand overnight. Compound 931 was converted to compound 913 by oxidation. The residue was charged on a column of silica-gel (C-200) packed with chloroform, and purified by eluting with chloroform-methanol (30 : 1) to obtain compound 913 (colourless crystals).
Yield: 854 mg (yield: 50%)

### Example 65:

### 3-methyl-6-(3-(N-cyclohexyl-N-ethylaminocarbonyl)propoxy)-2-oxo-1,2-dihydroquinoxaline (compound 1029):

2.0 mM 3-methyl-6-(3-carboxypropoxy)-2-oxo-1,2-dihydroquinoxaline obtained in Example 11 was added to 20 mℓ tetrahydrofuran. An equimolar amount of triethylamine was added thereto, an equimolar amount of pivaloyl chloride was added dropwise at -10 ° C and stirred at below -10 ° C for 1 hour. 0.3 mℓ cyclohexylethylamine was added at once to the obtained reaction mixture, and stirred for 3 hours, gradually adjusting the temperature to room temperature. Chloroform was added to the reaction mixture, and washed with dilute aqueous potassium carbonate. The aqueous layer was further extracted with chloroform. The combined chloroform layer was dried by adding anhydrous sodium sulfate and concentrated in vacuo. The residue was charged on a column of silica-gel (C-200, 70 g) and eluted with chloroform-methanol (50 : 1) to obtain compound 1029.
Yield: 0.47 g (yield: 63.3%)

### Referential example 26:

### 2-{N-(5-(4-ethoxycarbonyl)butoxy-2-nitrophenyl) -amino-propionic acid ethyl ester:

12.7 g 2-(N-(5-hydroxy-2-nitrophenyl))-amino propionic acid ethyl ester (50 mM) was added to 1.15 g metallic sodium (50 mM) dissolved in 150 mℓ ethanol, and dried in vacuo. The residue was dissolved in 300 mℓ dimethylformamide and 10.45 g δ-bromovaleric acid ethyl ester (50 mM) was added thereto and stirred overnight. The solvent was distilled off in vacuo. The residue, dissolved in chloroform, was washed with dilute aqueous potassium carbonate, dried with anhydrous sodium sulfate and concentrated in vacuo. The residue was charged on a column of silica-gel (C-200, 120 g) packed with benzene and eluted with benzene-ethyl acetate to obtain the product.
Yield: 17.13 g (yield: 93.1%)
   NMR (CDCℓ₃) δ (ppm) ; 1.27 (t, 3H, J=7), 1.28 (t, 3H, J = 7), 1.60 (d, 3H, J = 7), 1.6 - 2.0 (m, 4H), 2.39 (t, 2H, J = ₇), 3.8 - 4.4 (m, 7H), 6.04 (d, 1 H, J = ₂.₅), 6.25 (d.d, 1 H, J = 2.5, 10), 8.16 (1 H, J = 10), 8.54 (d, 1 H, J = 8)

### Referential example 27:

### 2-{N-(5-(4-ethoxycarbonyl)-butoxy-2-nitrophenyl)}-amino-butyric acid ethyl ester:

In referential example 26, 2-(N-(5-hydroxy-2-nitrophenyl))-aminopropionic acid ethyl ester was replaced by 2-(N-(5-hydroxy-2-nitro-phenyl))-aminobutyric ethyl ester (50 mM) to obtain the above compound.
Yield: 18.42 g (yield: 93.0%)
   NMR (CDCℓ₃) δ (ppm) ; 1.06 (t, 3H, J = 7), 1.26 (t, 3H, J = 7), 1.28 (t, 3H, J = 7), 1.6 - 2.2 (m, 6H), 2.39 (t, 2H, J = 7), 3.9 - 4.4 (m, 7H,), 6.06 (d, 1 H, J = 2.5), 6.25 (d.d, 1 H, J = 2.5, 10), 8.15 (d, 1 H, J = 10) 8.56 (d, 1 H, J = 8)

### Example 66:

### 3-methyl-6-(ethoxycarbonylbutoxy)-2-oxo-1,2-dihydroquinoxaline (compound 1036):

17.12 g 2-{N-(4-ethoxycarbonyl)butoxy}-2-nitrophenyl))amino propionic acid ethyl ester (44.84 mM) and 11.6 g iron powder were added to 350 m ethanol.

Approximately 5-N HCI/ethanol solution was added dropwise under reflux until the starting material disappeared. Insoluble material was removed by filtration and the filtrate was concentrated in vacuo. The residue, dissolved in chloroform, was washed with dilute hydrochloric acid and saturated sodium chloride solution, in this order, dried with anhydrous sodium sulfate and concentrated in vacuo. The residue was dissolved in 500 mℓ benzene, 10.2 g DDQ was added, and then the reaction mixture was refluxed for 2 hours.

Insoluble material was filtered off and the filtrate was concentrated in vacuo. The residue was charged on a column of silica-gel (C-200, 250 g) packed with chloroform, and purified by eluting with chloroform-methanol (200 : 1) to obtain compound 1036.
Yield: 8.99 g (yield: 66.0%)

### Example 67:

### 3-ethyl-4-(ethoxycarbonyl butoxy)-2-oxo-1,2-dihydroquinoxaline (compound 1012):

In example 66 2-{N-(5-(4-ethoxycarbonyl) butoxy-2-nitrophenyl))-amino-propionic acid ethyl ester was replaced by 18.40 g 2-{N-5-(4-ethoxycarbonyl) butoxy-2-nitrophenyl)}-amino-butyric acid ethyl ester (46.50 mM); 12.9 g iron powder and 11.1 g DDQ were used to obtain compound 1012.
Yield: 9.12 g (yield 61.7%)

### Example 68:

### 3-methyl-6-(4-carboxybutoxy)-2-oxo-1,2-dihydroquinoxaline (compound 1037):

29.6 mℓ 2N-NaOH solution was added to 8.99 g compound 1036 (29.6 mM) suspended in 29.6 mℓ methanol and stirred overnight. 88.8 mℓ water was added to the reaction mixture, and adjusted to pH 2 by adding 6N-HCI. Precipitated crystals were collected by filtration, washed completely with water and dried to obtain compound 1037.
Yield: 7.89 g (yield: 96.6%)

### Example 69:

### 3-ethyl-6-(4-carboxy butoxy)-2-oxo-1,2-dihydroquinoxaline (compound 1013):

28.0 mℓ 2N-NaOH solution was added to 8.90 g compound 1012 (28.9 mM) suspended in 28.0 mℓ methanol and stirred overnight. 84.0 mℓ water was added to the reaction mixture, and adjusted to pH 2 by adding 6N-HCI. Precipitated crystals were collected by filtration, washed completely with water and dried to obtain compound 1013.

### Example 70:

### 3-methyl-6-(4-(N-cyclohexyl-N-2-hydroxyethylaminocarbonyl)butoxy)-2-oxo-2,2-dihydroquinoxaline (compound 1034):

5.0 mM compound 1037 was added to 20 mî tetrahydrofuran. An equimolar amount of triethylamine was added thereto, an equimolar amount of chloroisobutyl formate was added dropwise at -10 ° C and stirred at -10 ° C for 1 hour. 0.73 g N-cyclohexyl 1-2-hydroxyethylamine was added at once, and stirred for 3 hours gradually adjusting the temperature to room temperature. Chloroform was added to the reaction mixture, and washed with dilute aqueous potassium carbonate. The aqueous layer was further extracted with chloroform. The combined chloroform layer was dried by adding anhydrous sodium sulfate and concentrated in vacuo. The residue was charged on a column of silica-gel (C200, 120 g) and eluted with chloroform-methanol (30 : 1) to obtain compound 1034.
Yield: 1.03 g (yield: 51.4%)

### Example 71:

### 3-ethyl-6-(4-(N-cyclohexyl-N-2-hydroxyethylaminocarbonyl) butoxy)-2-oxo-1,2-dihydroquinoxaline (compound 1015):

In example 70 compound 1037 was replaced by compound 13 to obtain compound 1015. Yield: 0.98 g (yield: 47.2%)

### Example 72:

### 3-ethyl-6-(4-(N-cyclohexyl-N-methyl-aminocarbonyl)butoxy)-2-oxo-1,2-dihydroquinoxaline (compound 1014):

5.0 mM compound 1013 was added to 20 mî tetrahydrofuran.

An equimolar amount of triethylamine was added thereto, an equimolar amount of chloroisobutyl formate was added dropwise at -10 ° C and stirred at -10 ° C for 1 hour. 0.68 m N-methylcyclohexylamine was added at once, and stirred for 3 hours gradually adjusting the temperature to room temperature. Chloroform was added to the reaction mixture, and washed with dilute aqueous potassium carbonate. The aqueous layer was further extracted with chloroform. The combined chloroform layer was dried by adding anhydrous sodium sulfate and concentrated in vacuo. The residue was charged on a column of silica-gel (C-200, 120 g) and eluted with chloroform-methanol (50 : 1) to obtain compound 1014.
Yield: 1.41 g (yield:73.2%)

### Example 73:

### 3-ethyl-6-(4-(N-cyclohexyl-N-methyl-aminocarbonyl)butoxy)-2-oxo-1,2-dihydroquinoxaline (compound 1035):

In example 72 compound 1013 was replaced by compound 37, and the N-methylcyclohexylamine was replaced by 0.77 mî N-ethylcyclohexylamine to obtain compound 1035.
Yield: 1.49 g (yield: 77.4%)

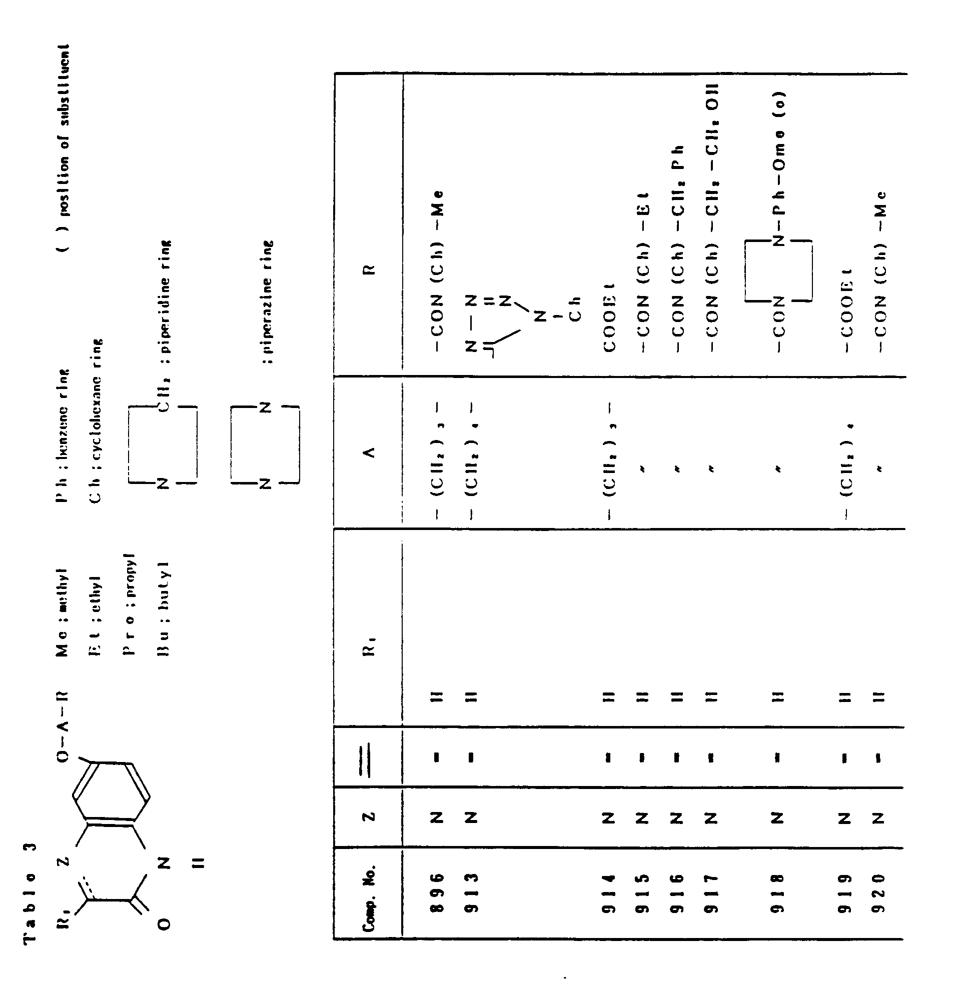

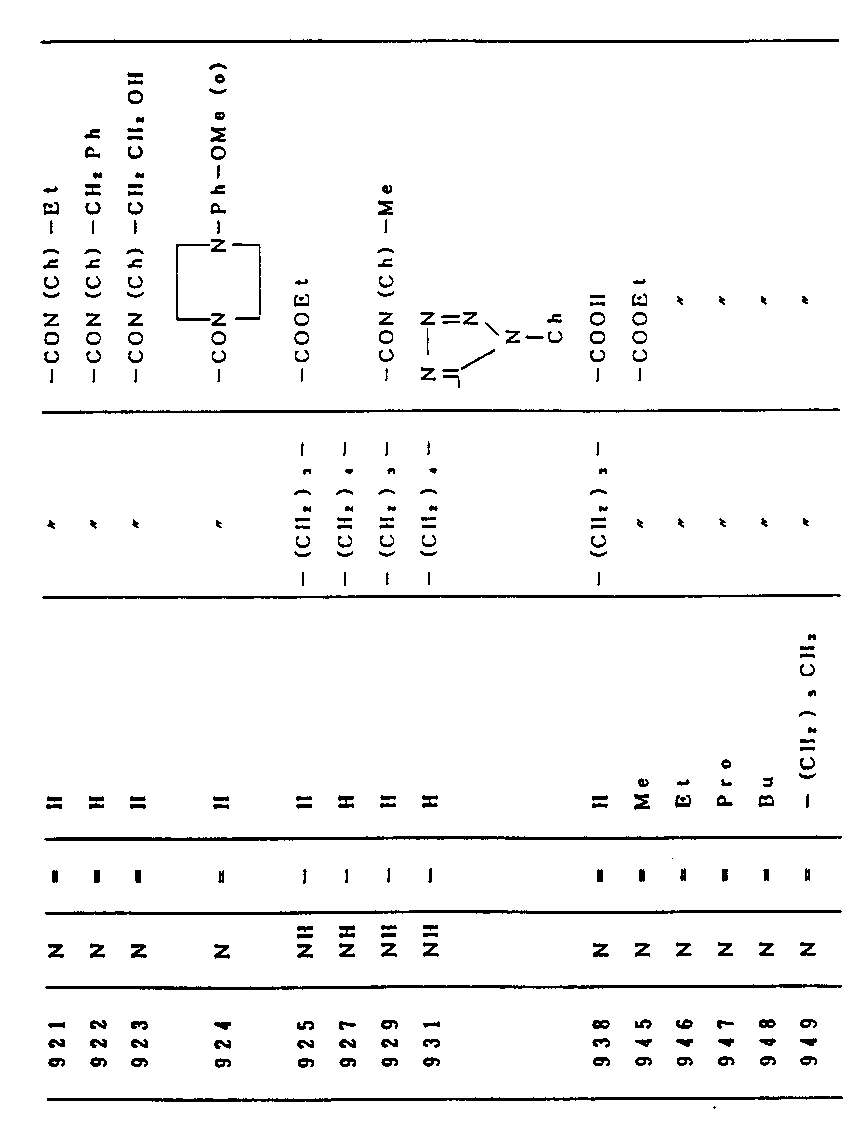

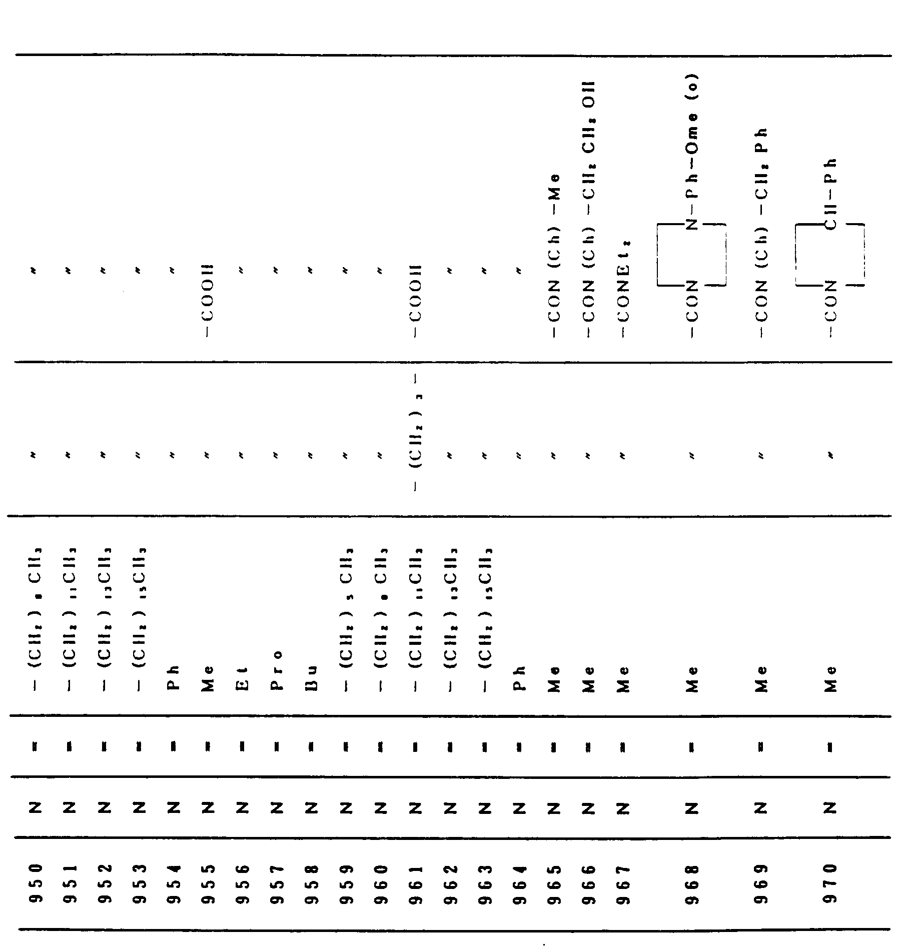

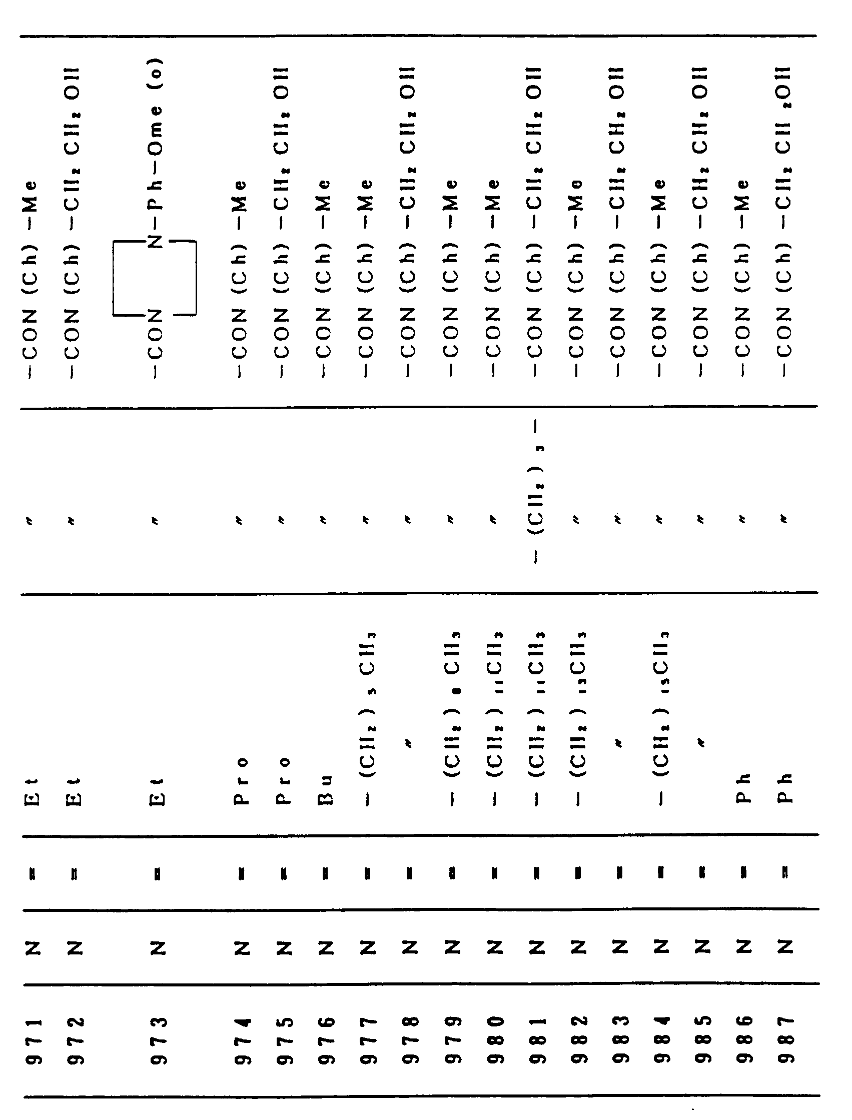

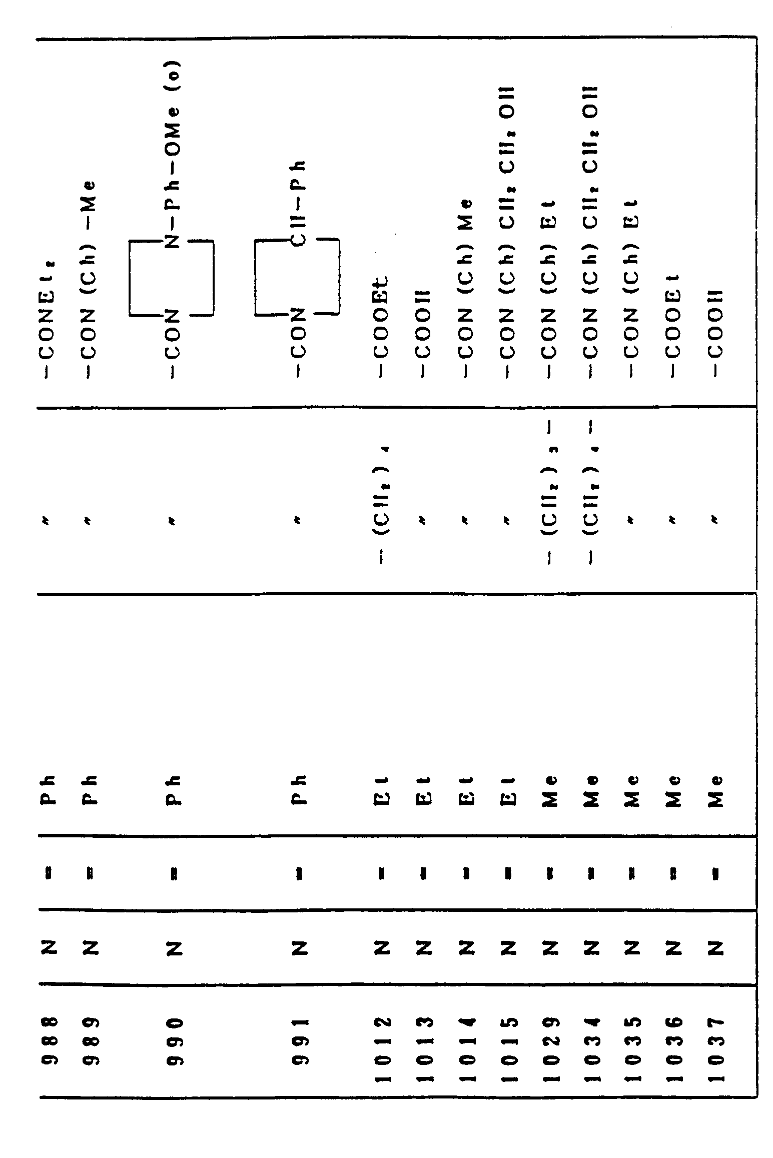

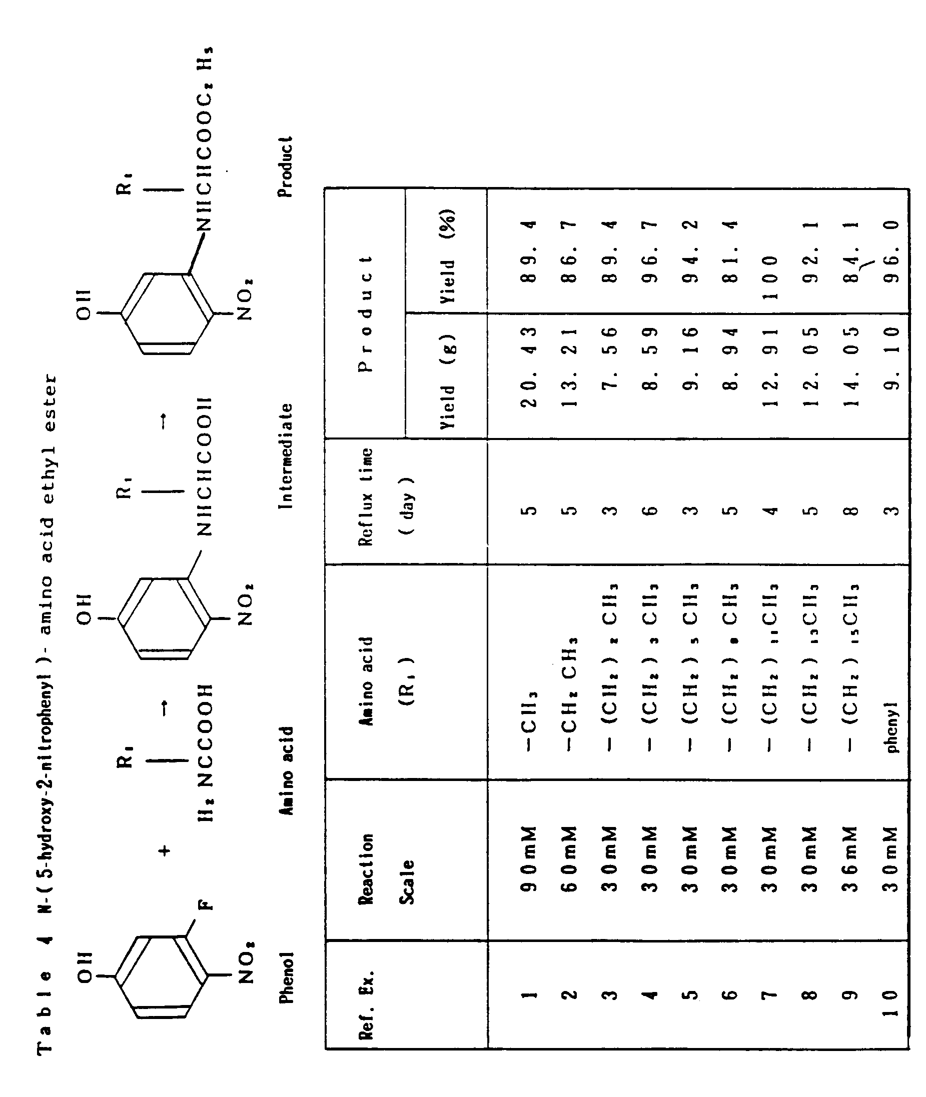

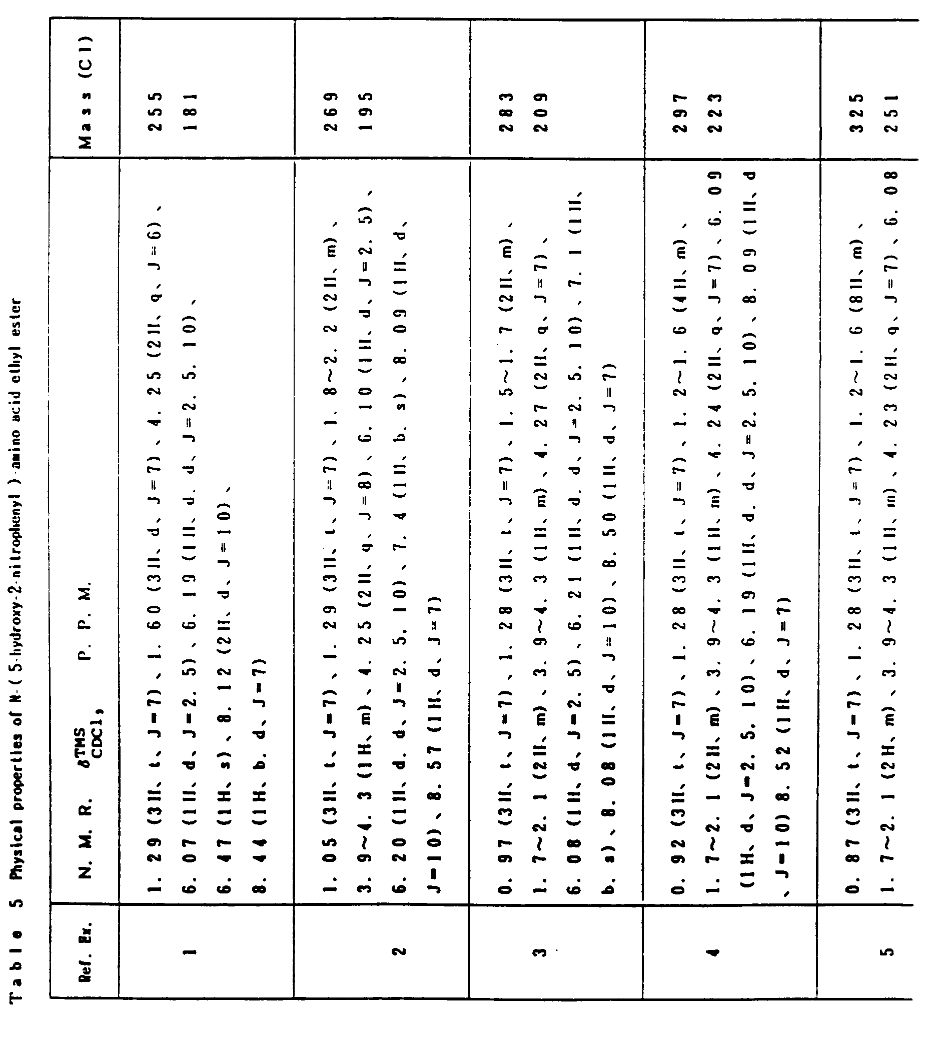

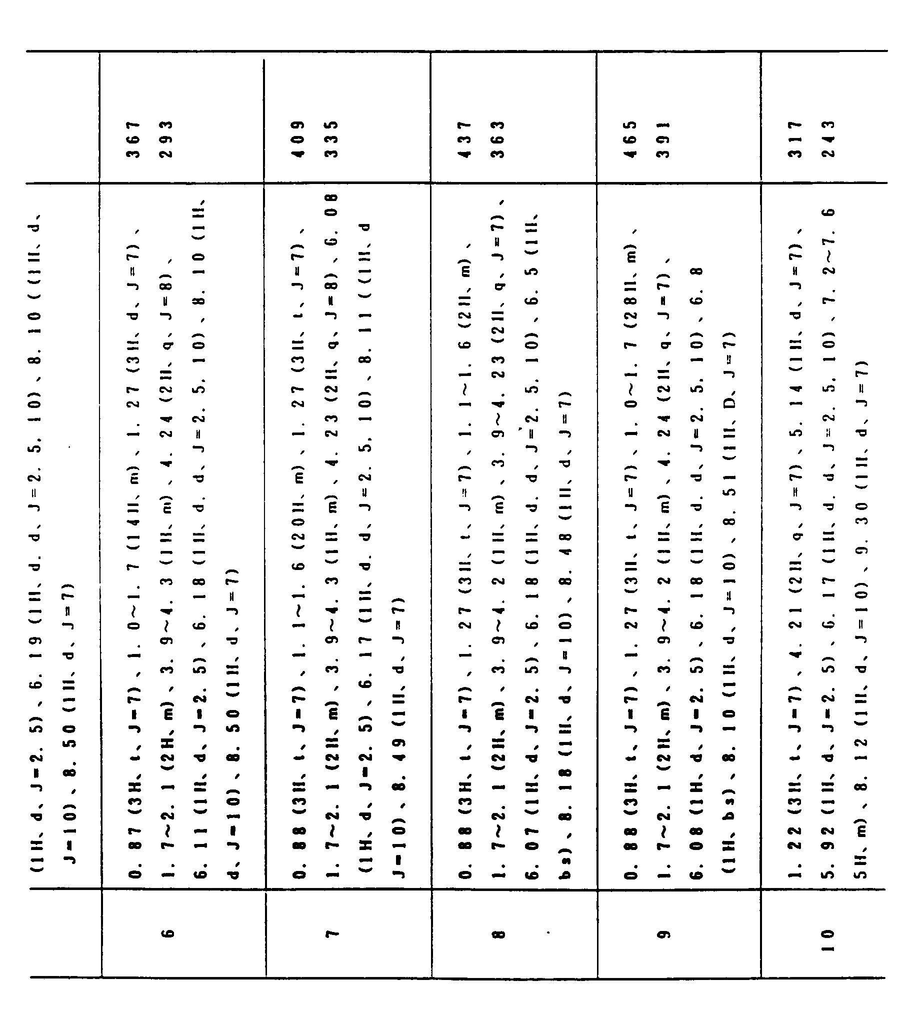

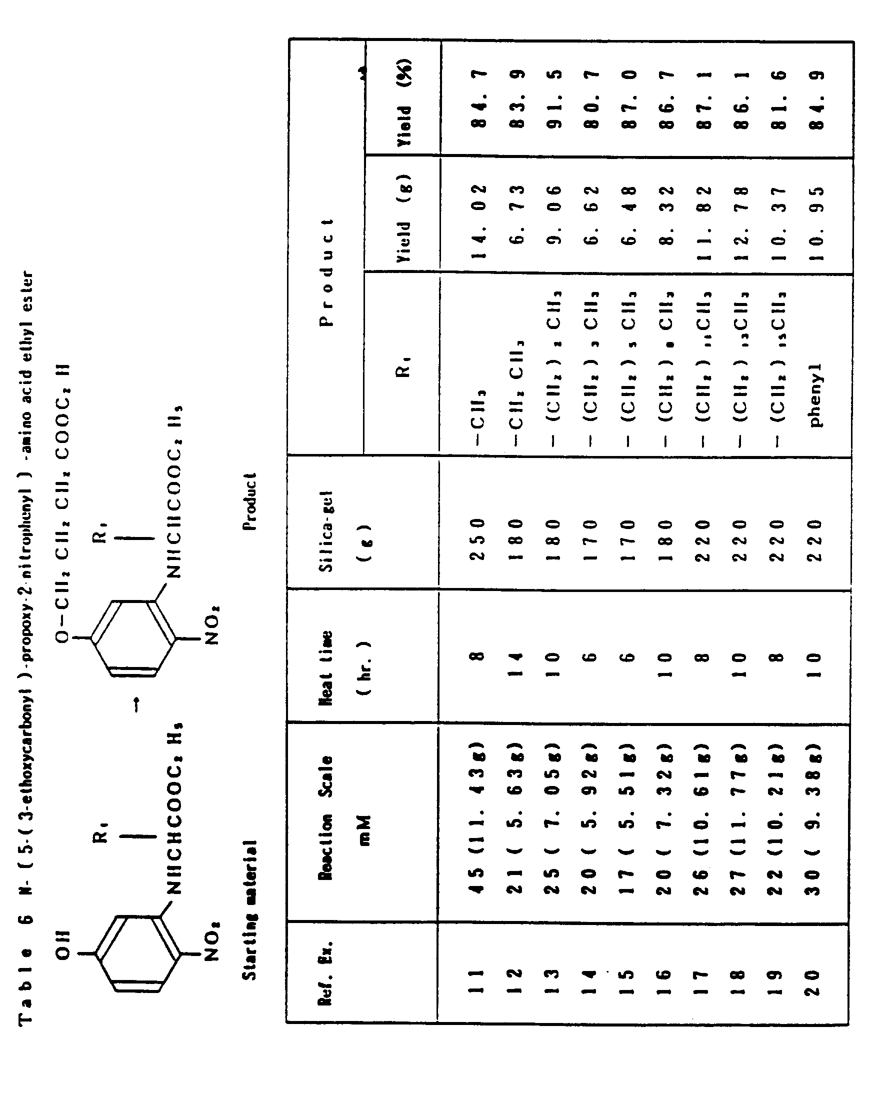

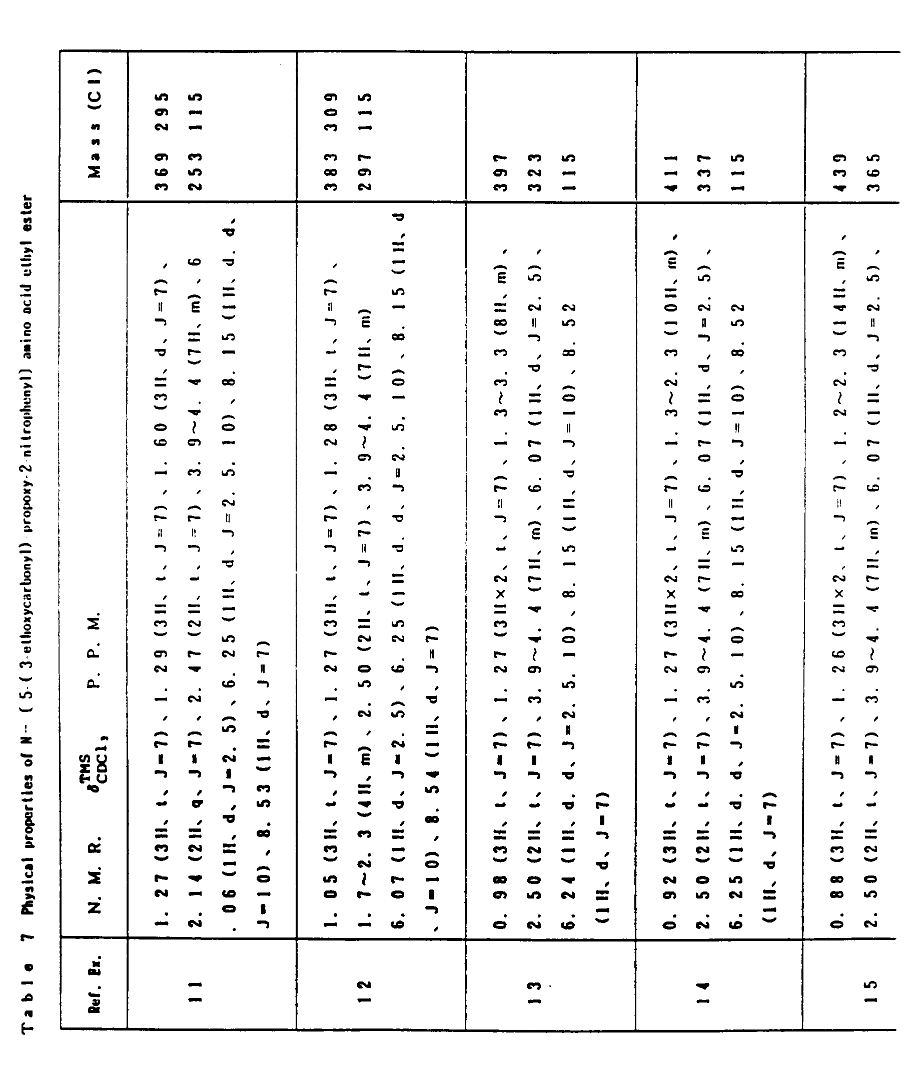

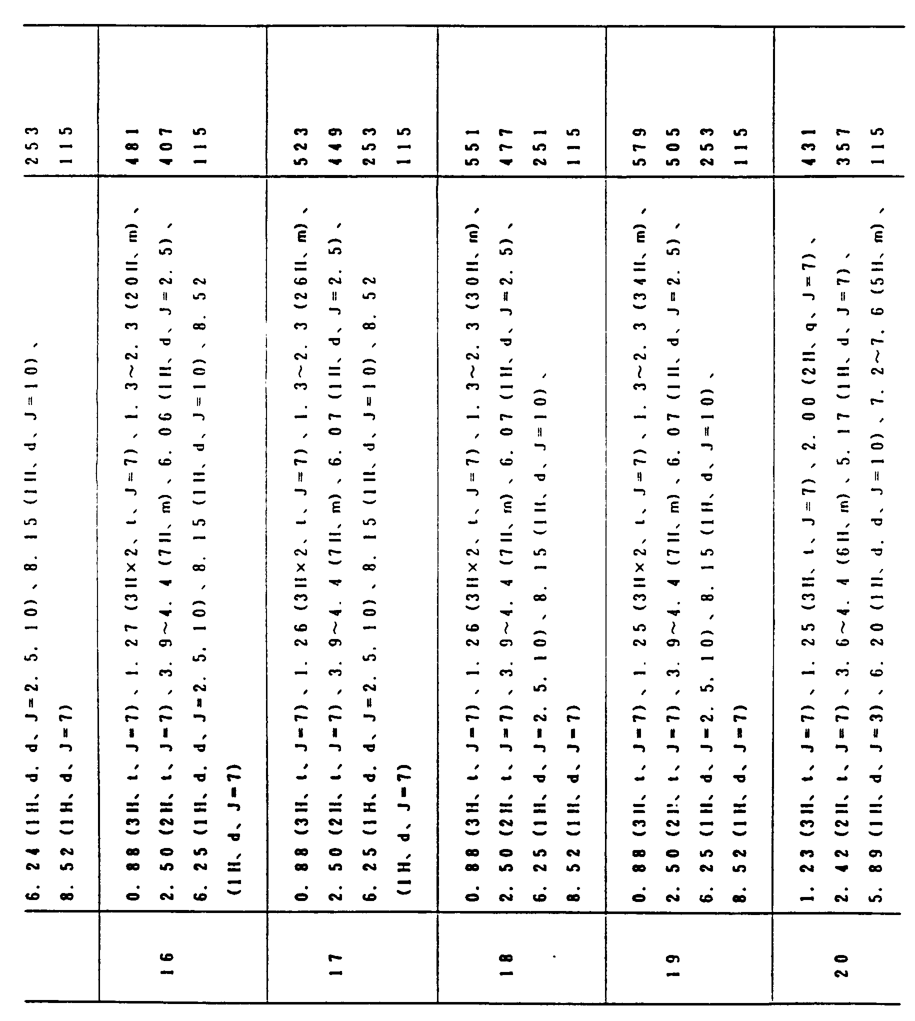

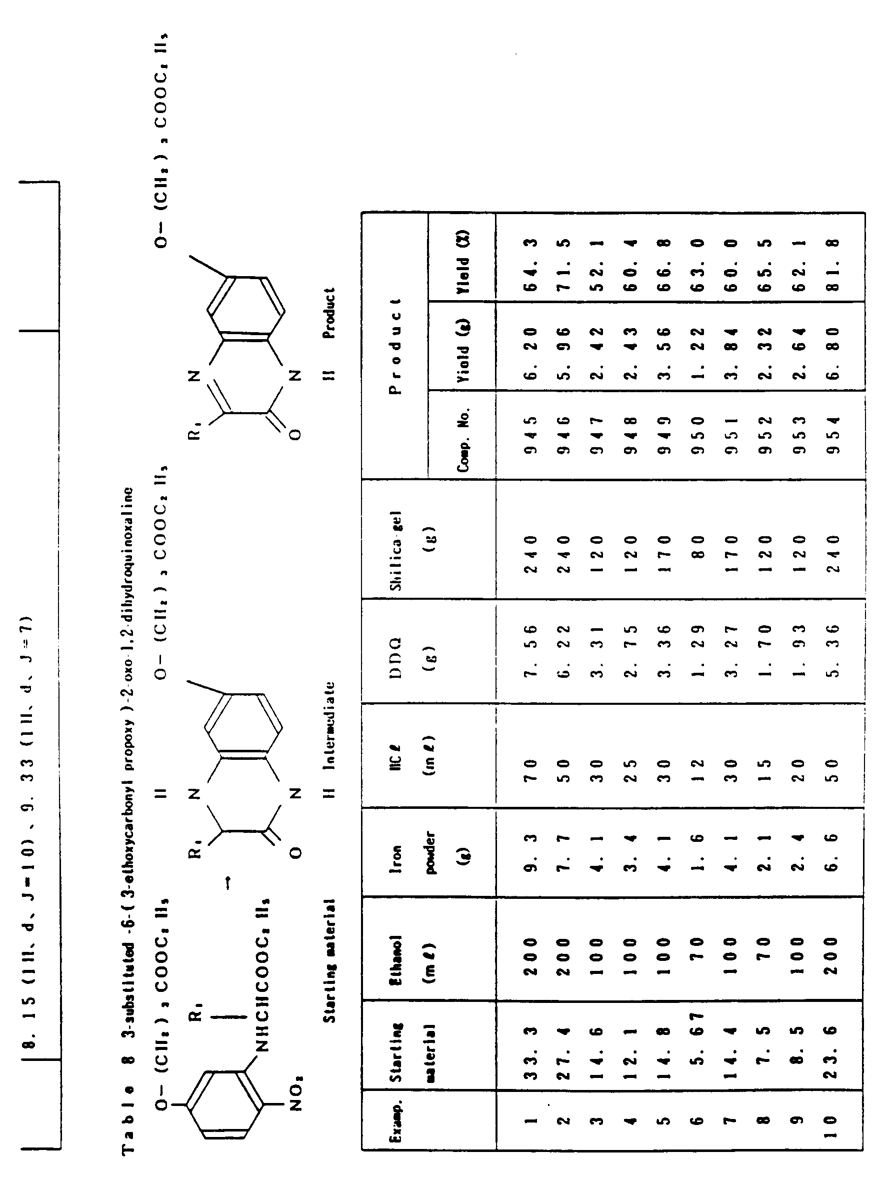

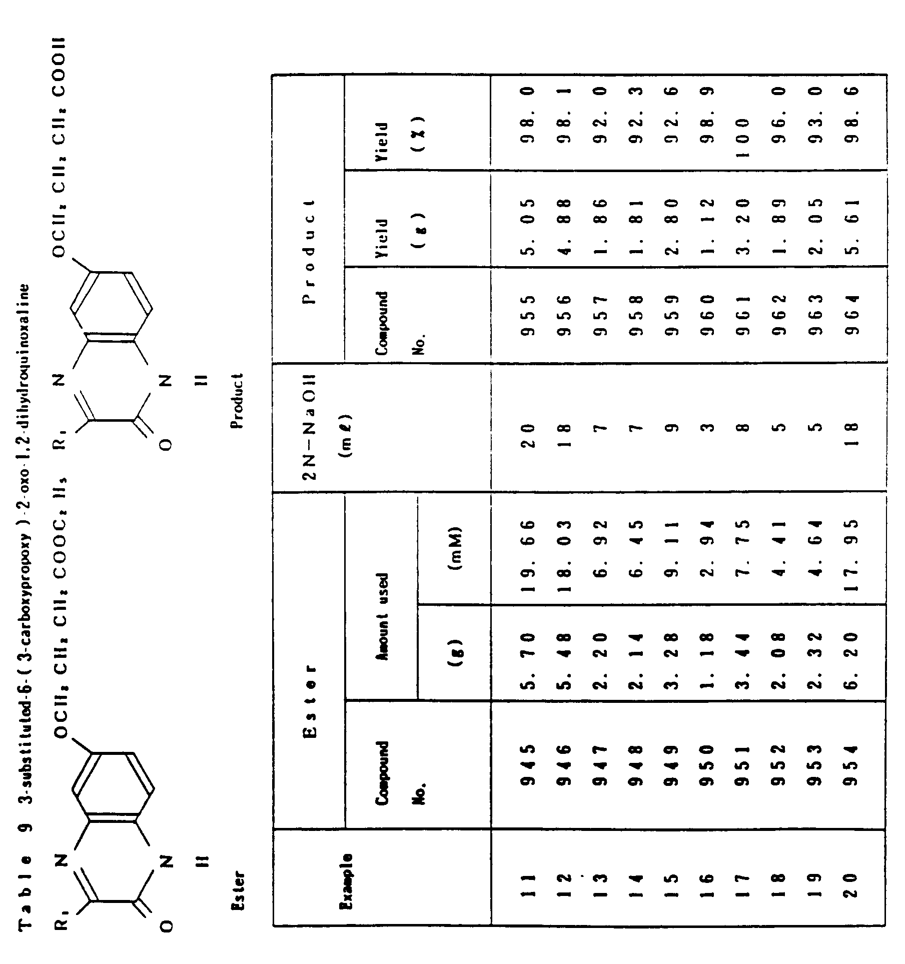

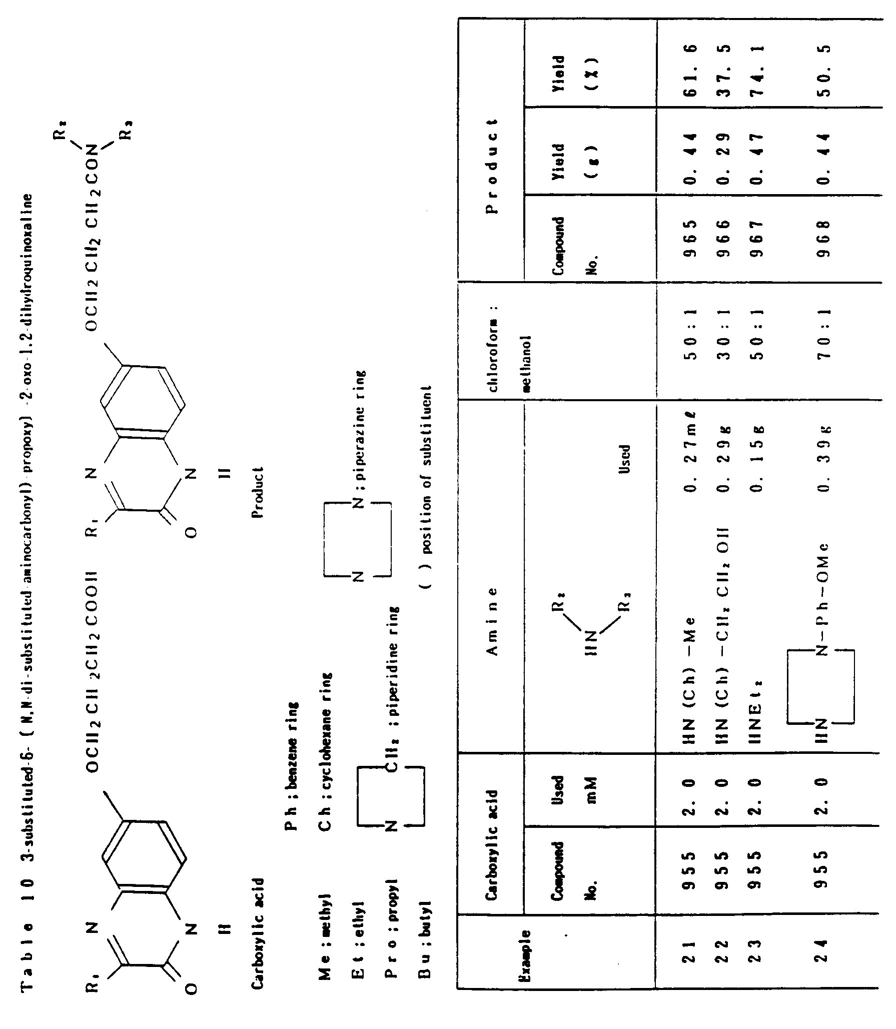

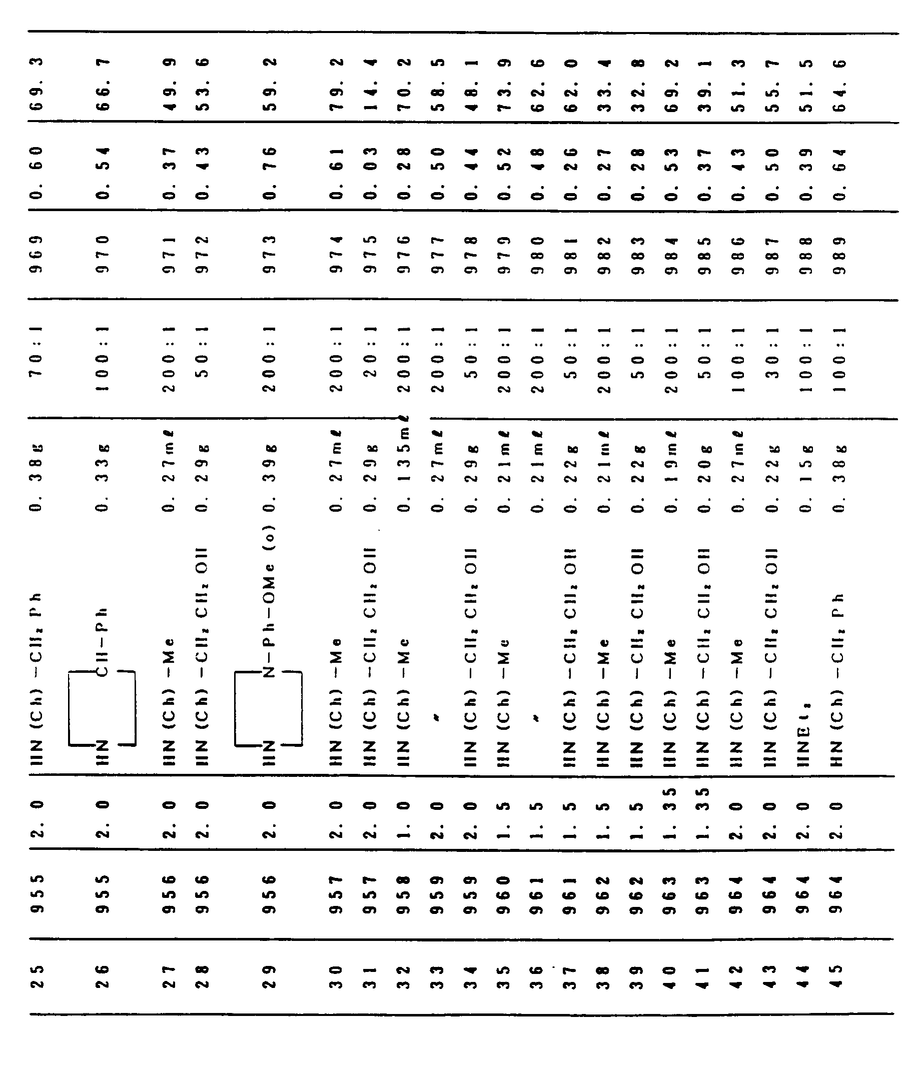

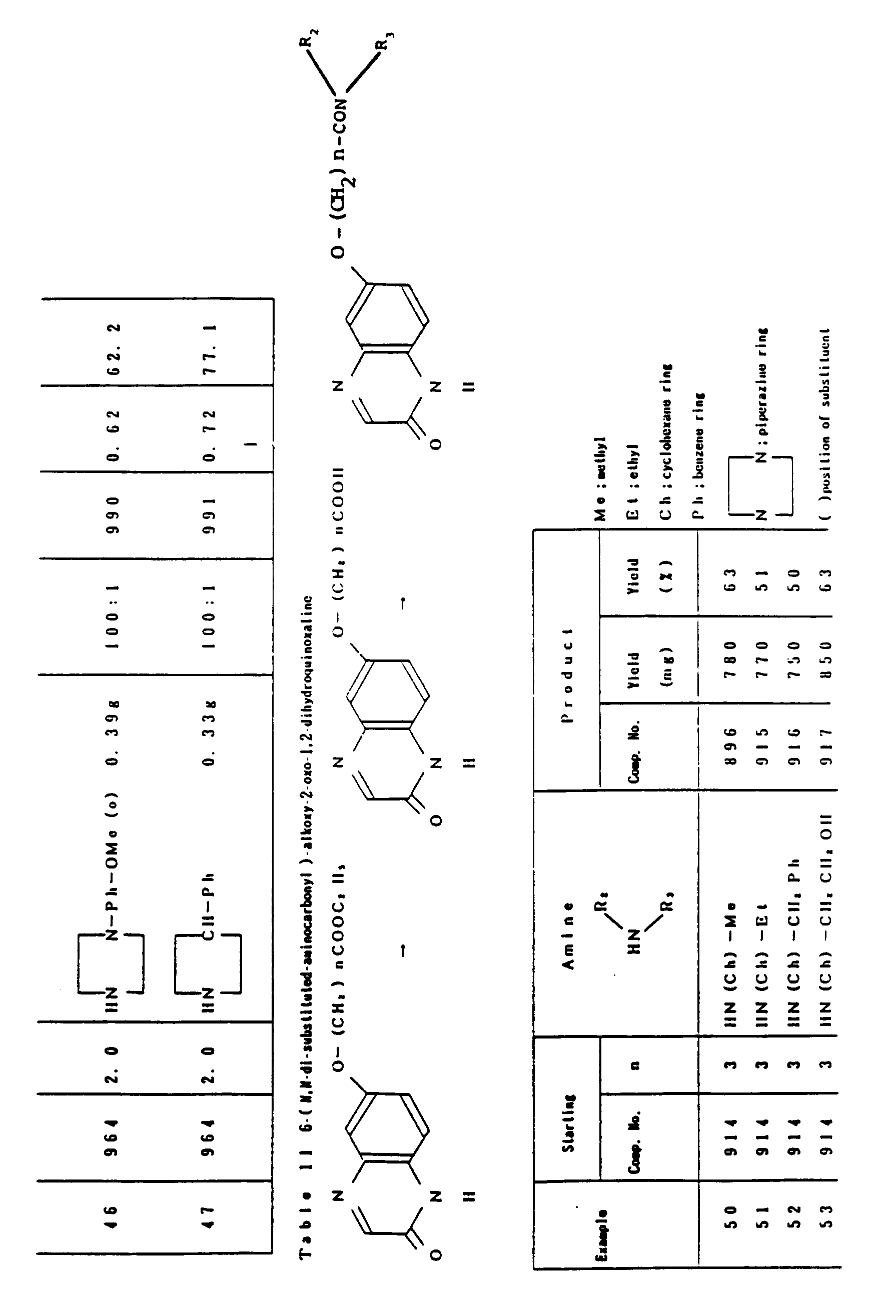

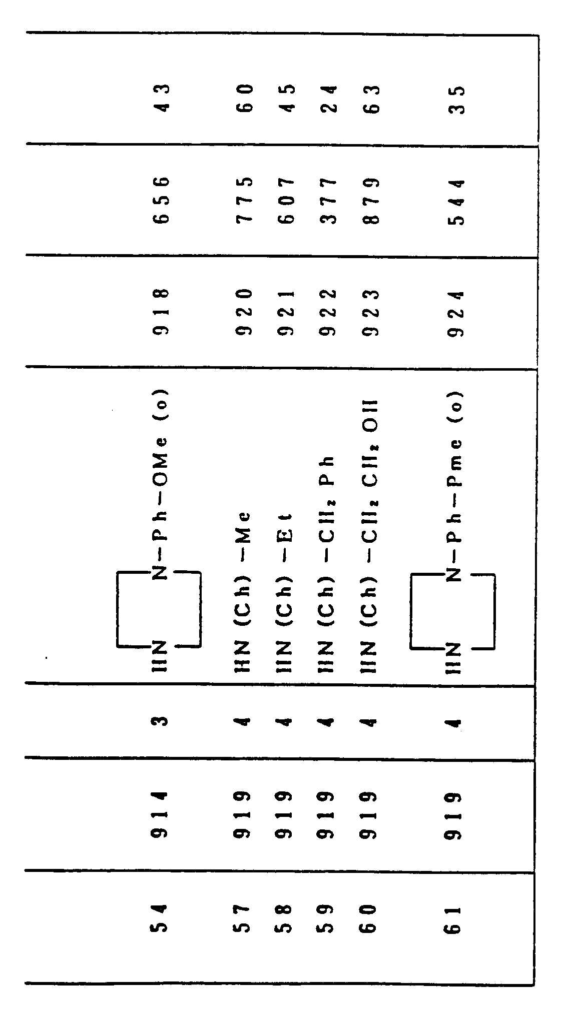

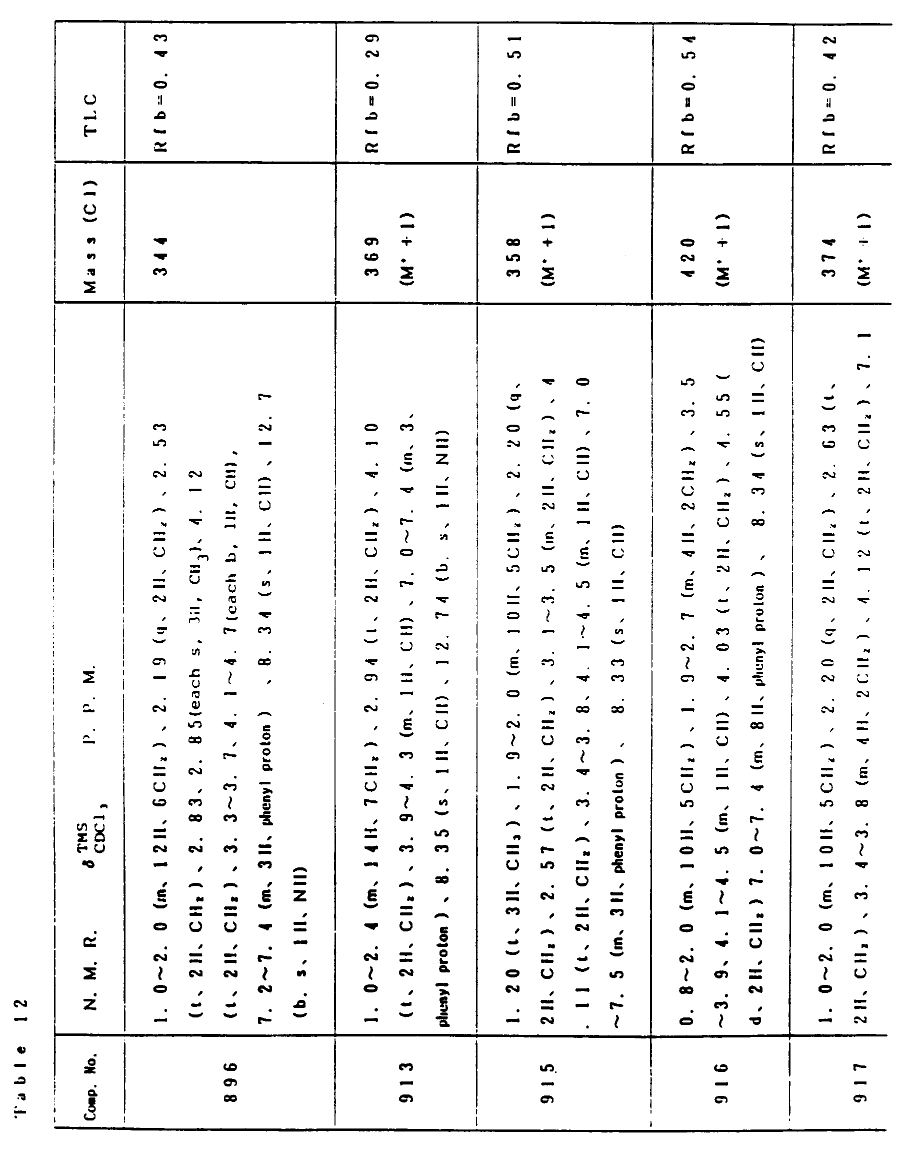

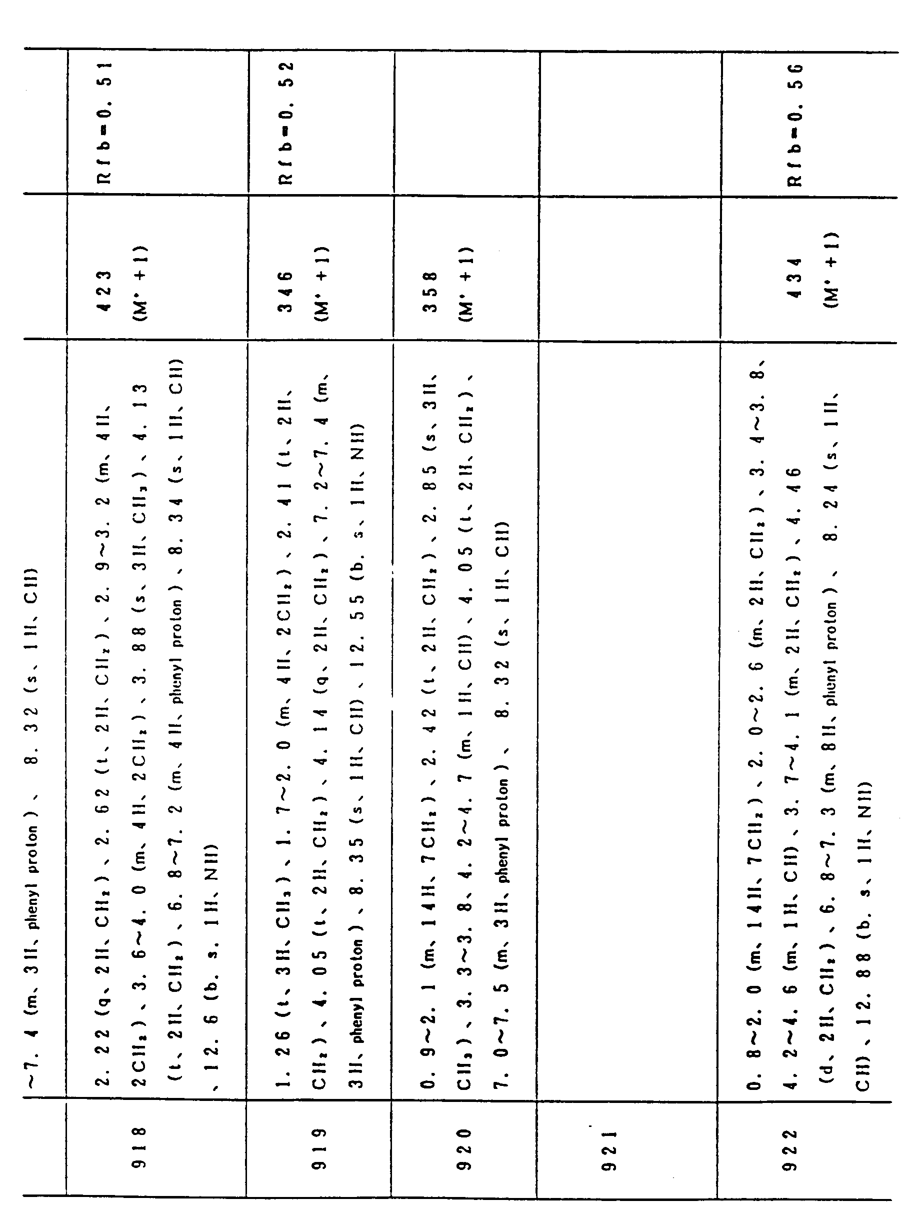

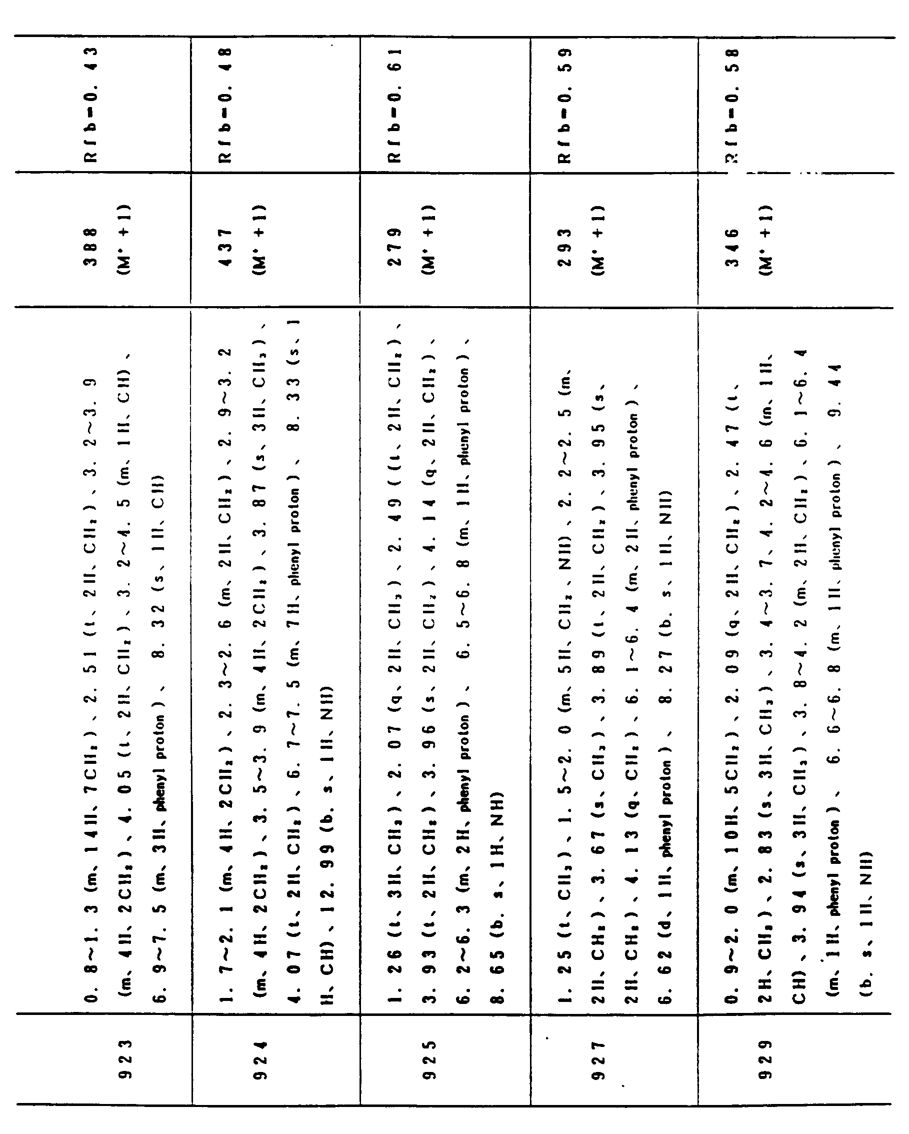

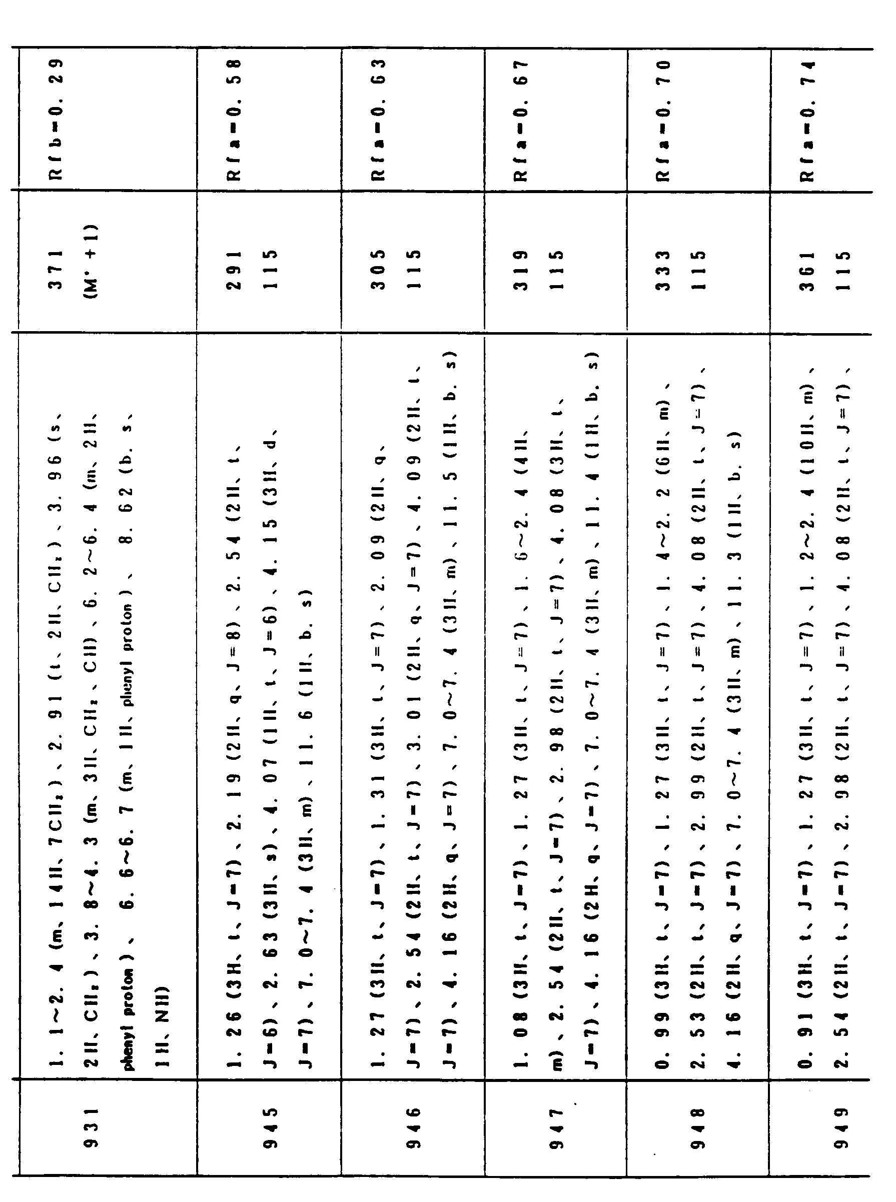

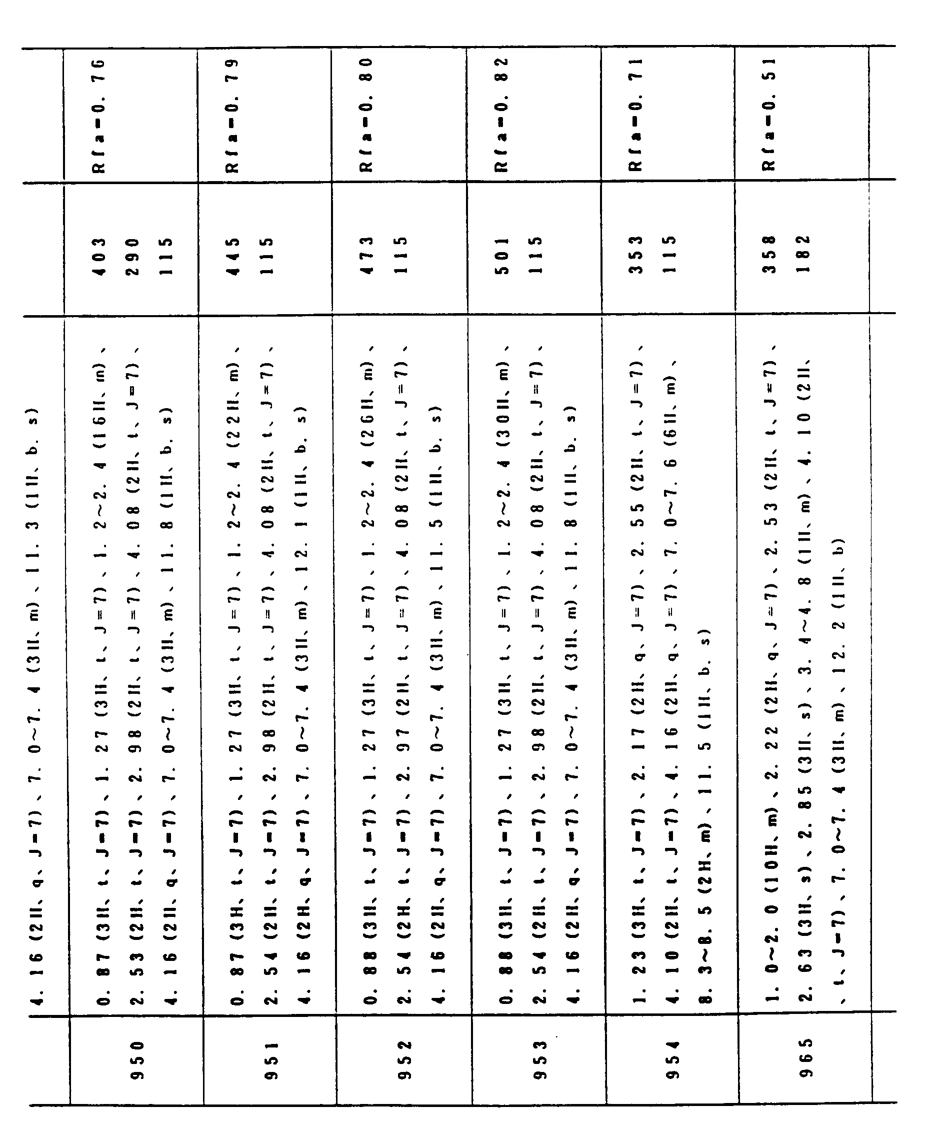

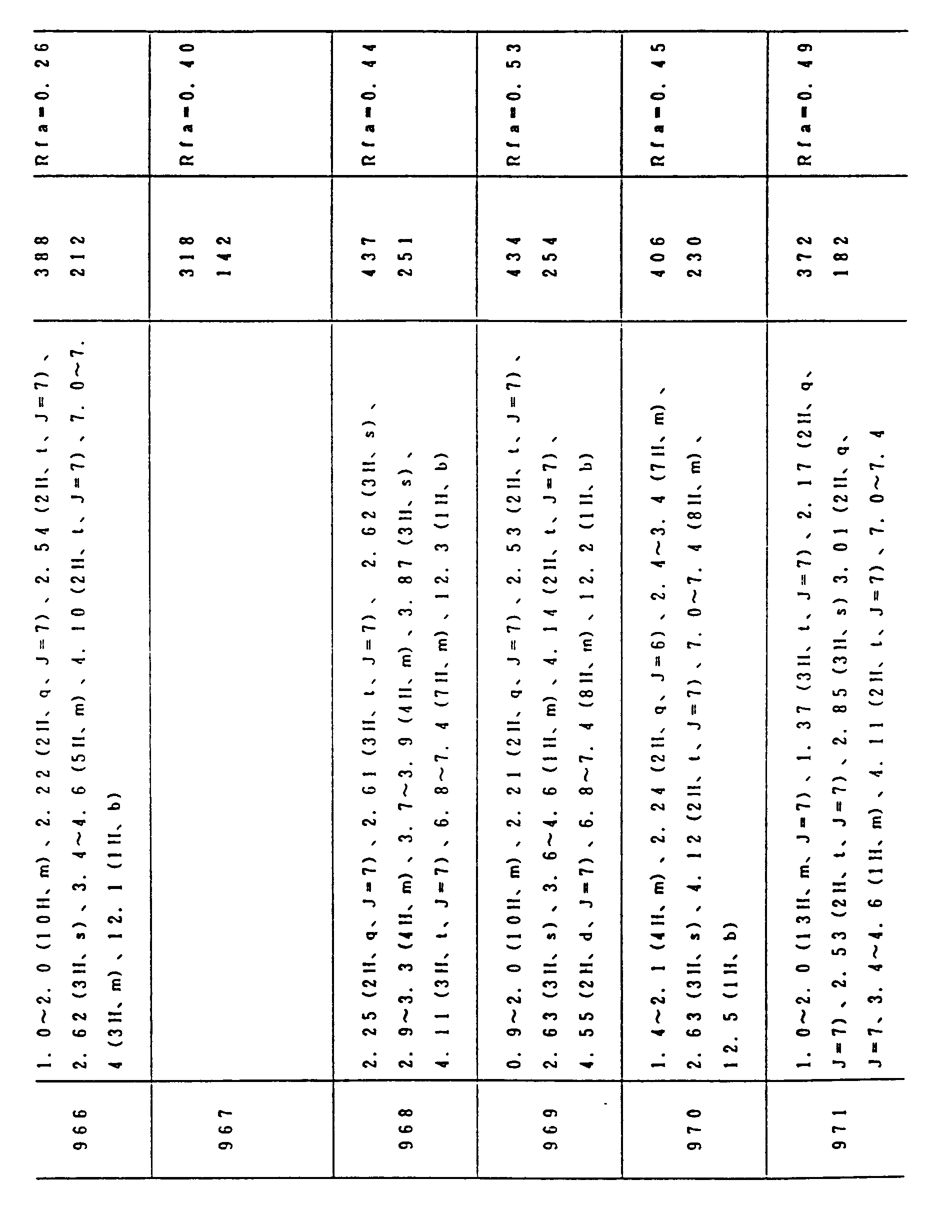

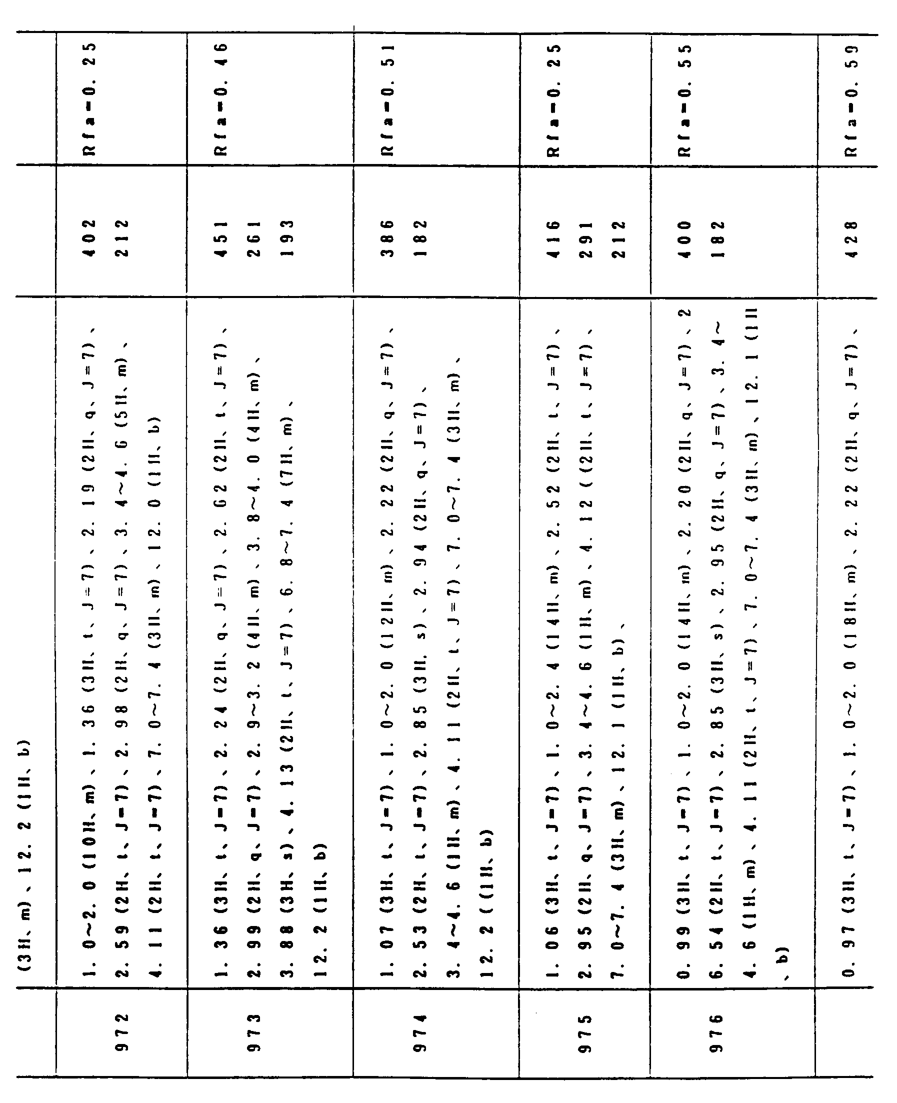

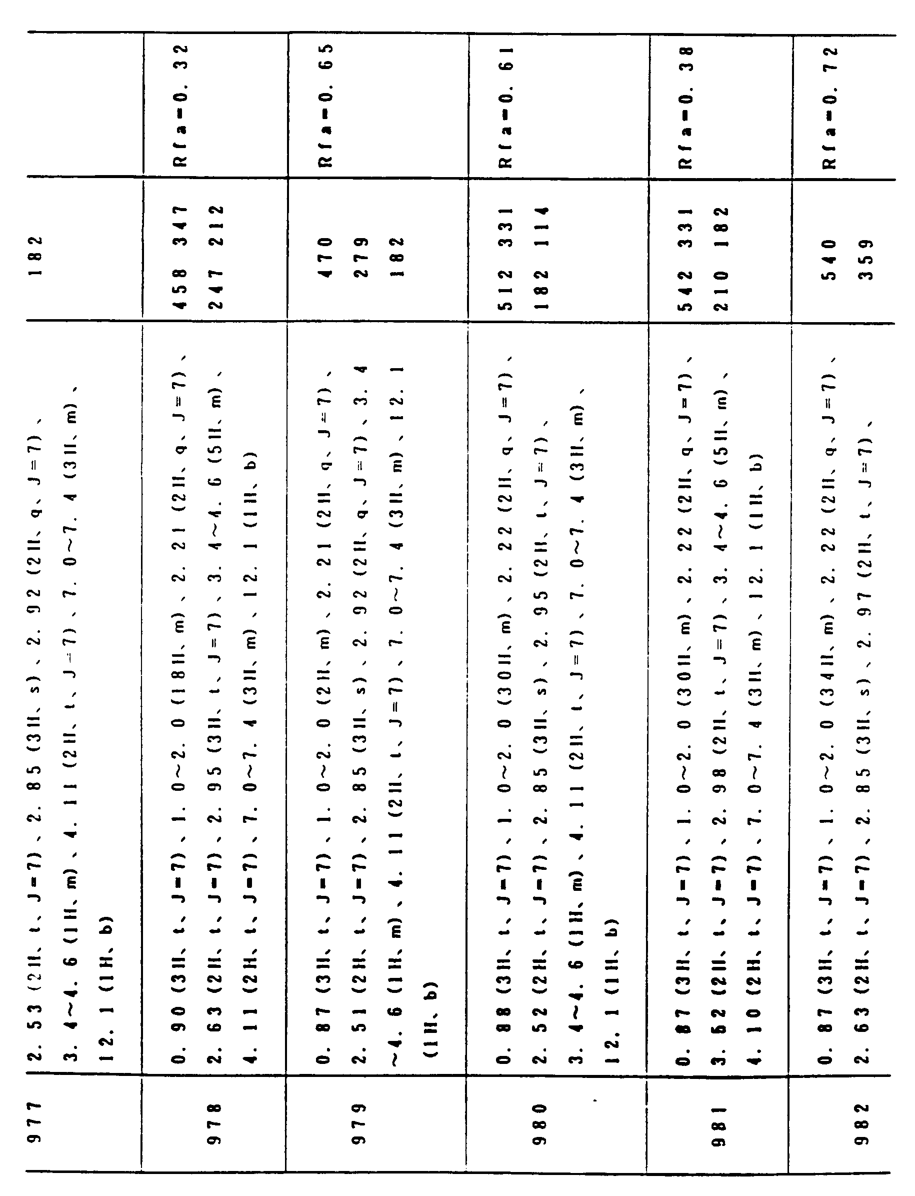

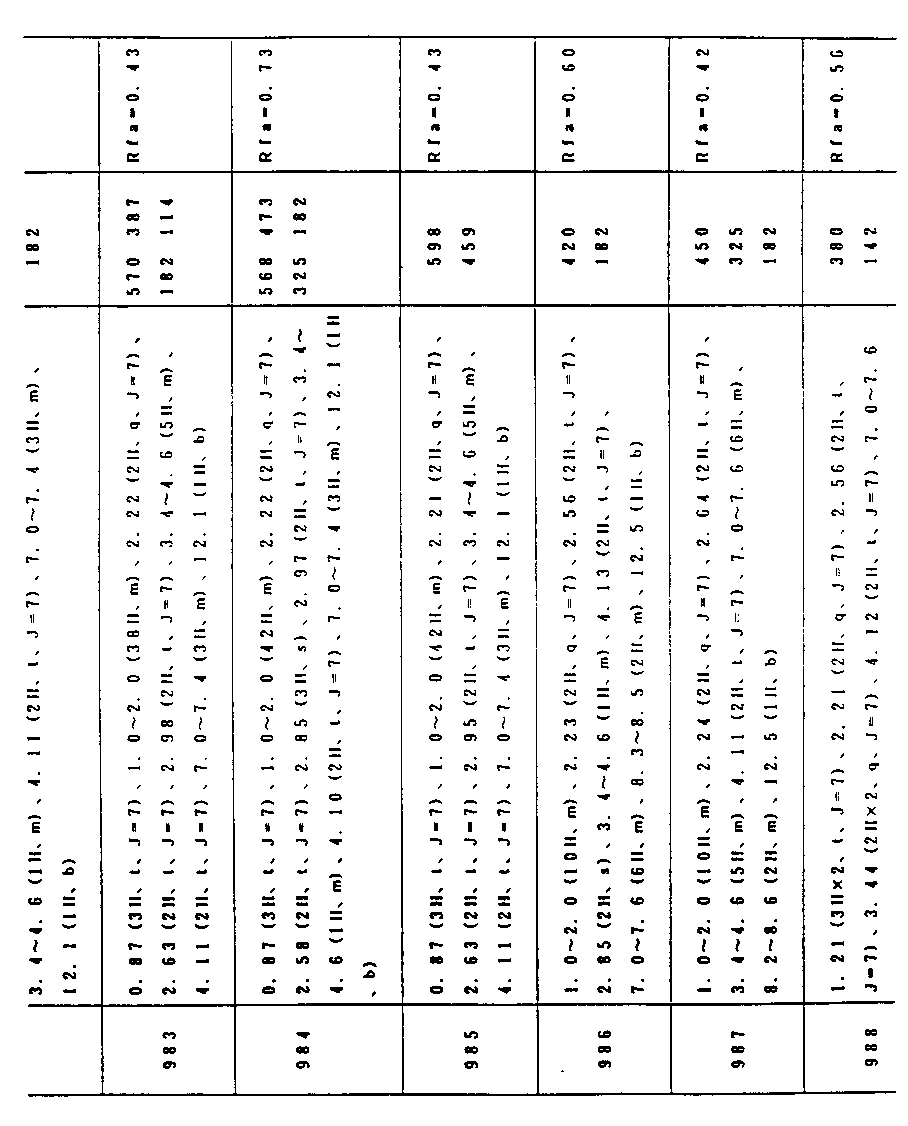

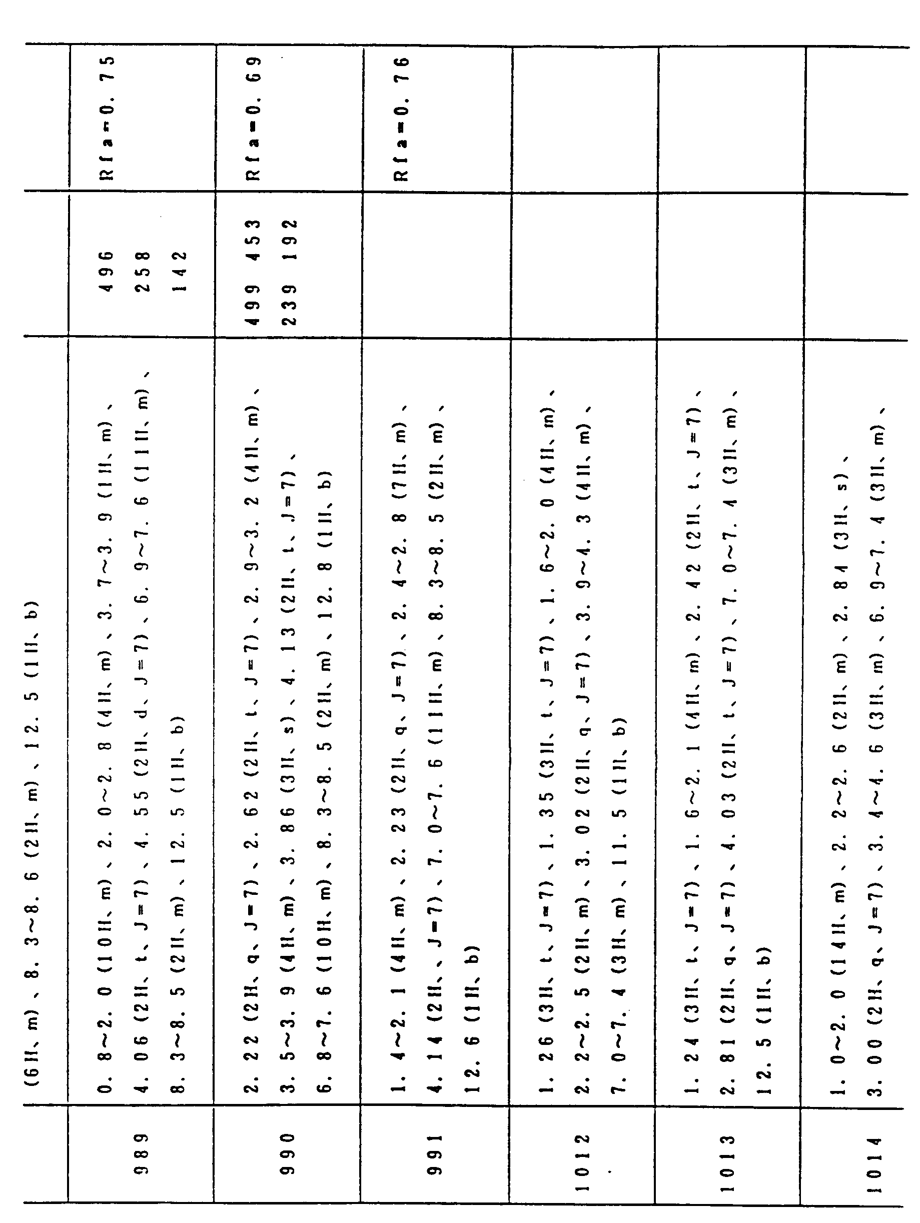

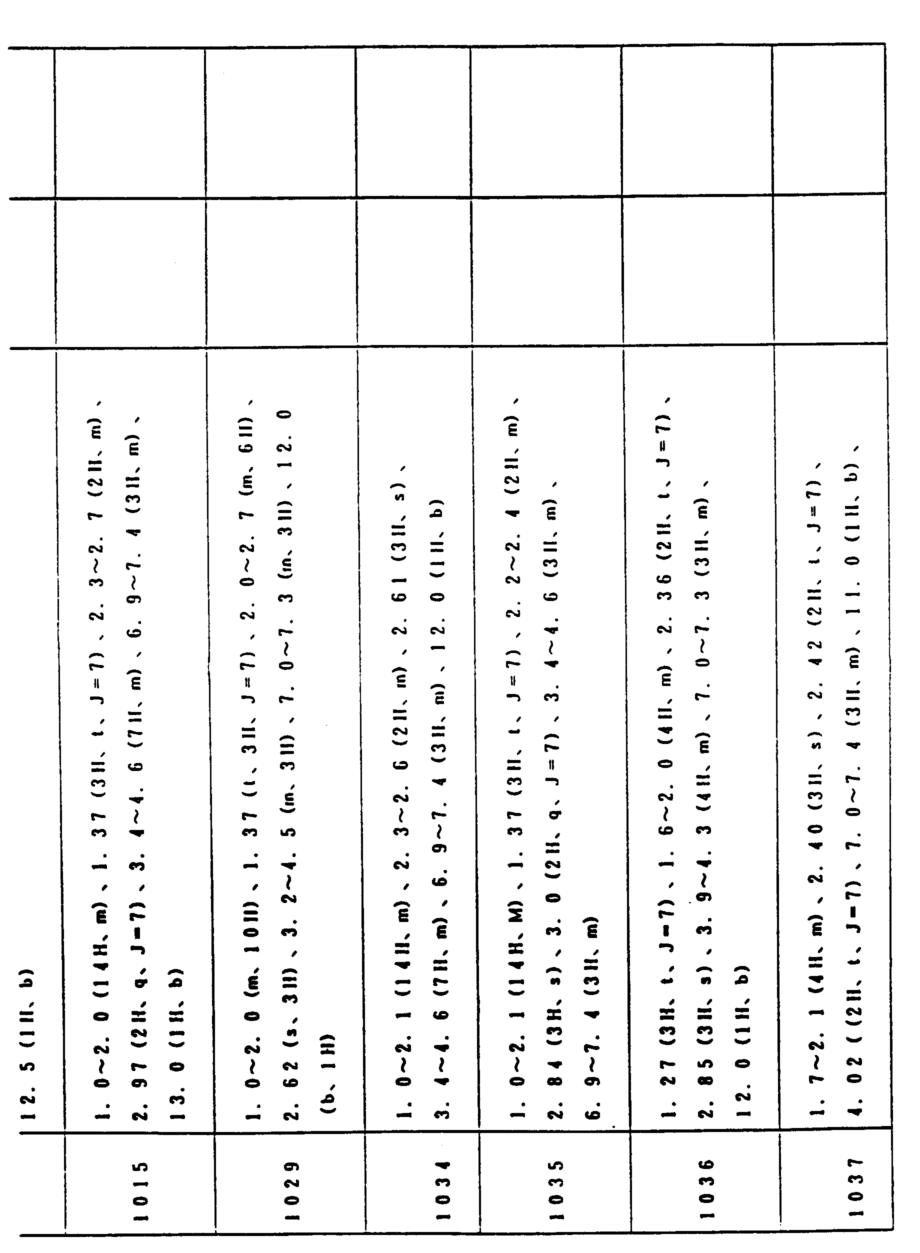

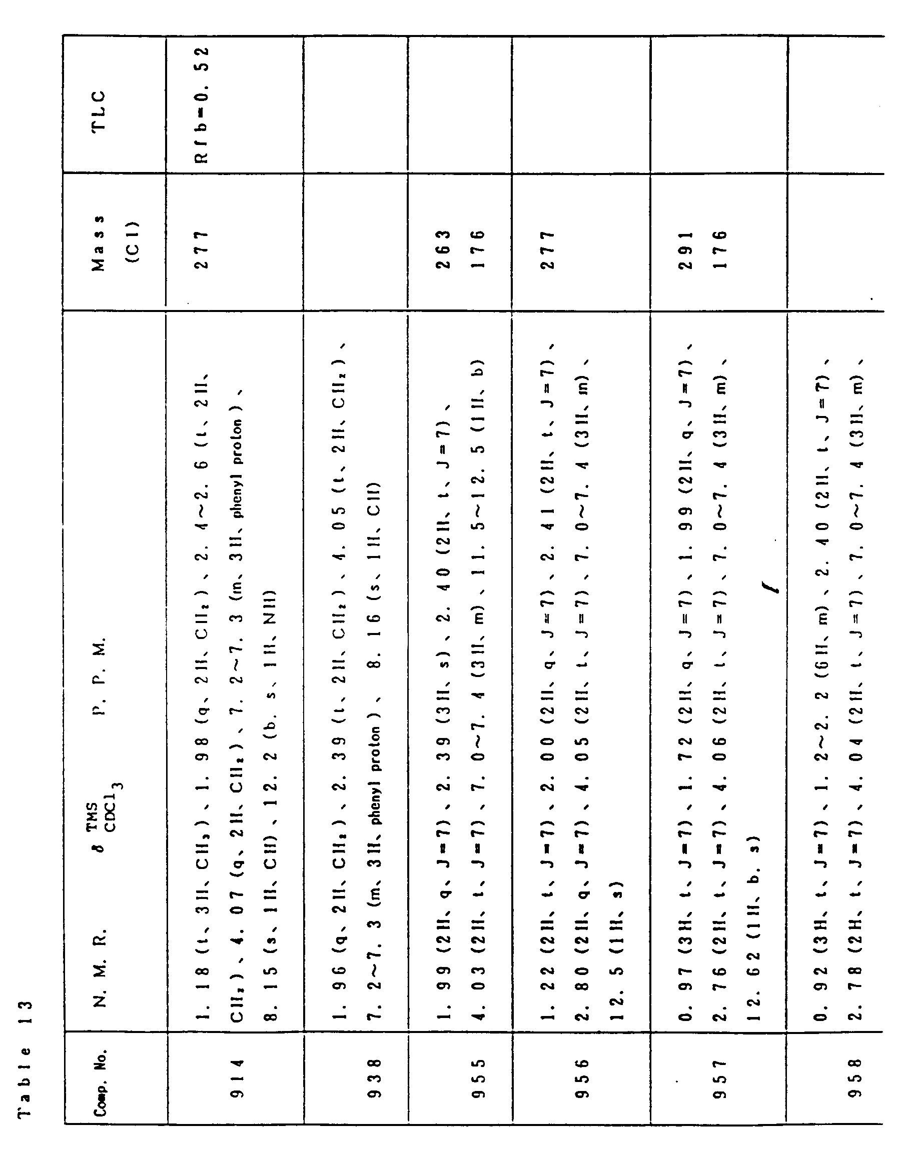

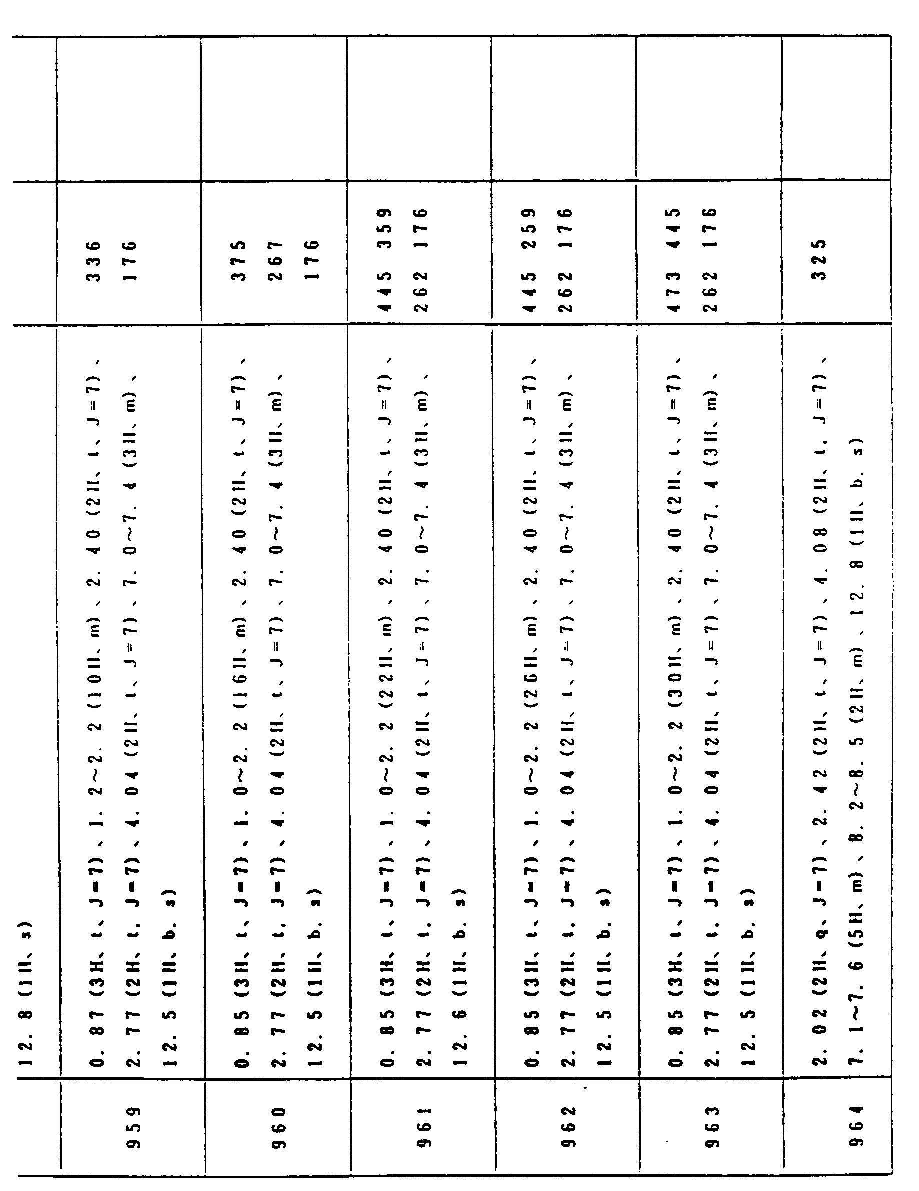

## Claims (Claims for the following Contracting State(s) : CH, DE, FR, GB, IT, LI)

1. A compound which is a 6-substituted alkoxy-2-oxo-1,2-dihydroquinoxaline derivative of formula (I): 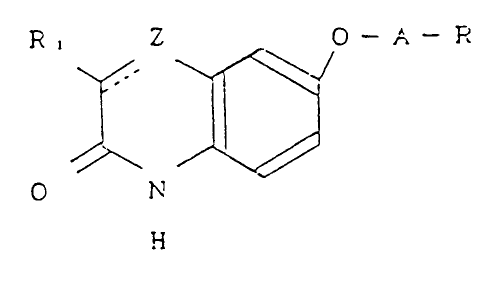 wherein:
Z is N and --- is a double bond or Z is NH and --- is a single bond,
R₁ is hydrogen, branched or unbranched C₁ -₂ₒ alkyl, unsubstituted phenyl or phenyl substituted by at least one substituent selected from C₁₋₃ alkyl, halogen, nitro and C₁-C₆ alkoxy,
A is C₁-C₆ alkylene, and
R is carboxyl, (C₁-C₆ alkoxy)carbonyl, 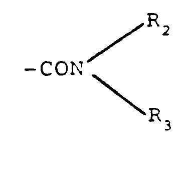 or 1-cycloalkyl-tetrazole-5-yl, wherein R₂ is C₁-C₆ alkyl, hydroxy-C₁-C₆ alkyl or phenyl-C₁-C₆ alkyl, in which the phenyl is unsubstituted or substituted by at least one substituent selected from C₁-C₃ alkyl, halogen, nitro and C₁-C₆ alkoxy, and R₃ is C₁-C₆ alkyl or C₅-C₇ cycloalkyl, or R₂ and R₃, together with the nitrogen to which they are attached, form 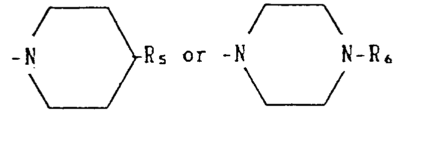 wherein R₅ and R₆ are hydrogen, phenyl or phenyl substituted by at least one substituent selected from C₁-C₃ alkyl, halogen, nitro and C₁-C₆ alkoxy,
or a pharmacologically acceptable non-toxic salt thereof.

2. A compound according to claim 1 wherein R₁ is methyl, ethyl, propyl, isopropyl, butyl, isobutyl, sec-butyl, t-butyl, pentyl, isopentyl, hexyl, heptyl, octyl, nonyl, decyl, undecyl, dodecyl, tridecyl, tetradecyl, pentadecyl or hexadecyl.

3. A compound according claim 1 or 2 wherein R is (C₁-C₄ alkoxy)carbonyl, 1-(C₅₋₇ cycloalkyl)-tetrazole-5-yl or 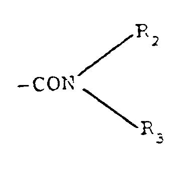 wherein R₂ is C₁-₄ alkyl, hydroxy (C₁-₄ alkyl) or phenyl (C₁-₄ alkyl) wherein the phenyl is unsubstituted or substituted by at least one substituent selected from C₁₋₃ alkyl, halogen, nitro and C₁-C₅ alkoxy and R₃ is C₁₋₄ alkyl or C₅₋₇ cycloalkyl or wherein R₂ and R₃, together with the nitrogen to which they are attached, form 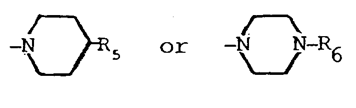 wherein R₅ and R₆ are hydrogen or phenyl unsubstituted or substituted by at least one substituent selected from C₁₋₃ alkyl, halogen, nitro and C₁₋C₆ alkoxy.

4. A compound according to any one of the preceding claims which is in the form of a hydrochloride, sulfate, phosphate, acetate, propionate, butyrate, glycolate, gluconate, malate, tartrate, succinate, mandelate, glutamate, aspartate, methanesulfonate or toluenesulfonate salt.

5. A process for the preparation of a compound as defined in any one of the preceding claims which comprises:
i) when Z is NH, --- is a single bond and R is (Cₗ-C₆ alkoxy)carbonyl or 1-cycloalkyl-tetrazole-5-yl, reducing a compound of formula [2]: 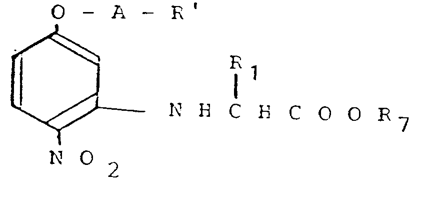 wherein R' is (C₁-C₆ alkoxy)carbonyl or 1-cycloalkyl-tetrazole-5-yl, A and R₁ are as defined in Claim 1 and R₇ is C₁-C₆ alkyl, to convert the nitro group thereof to an amino group, and cyclizing the product thus obtained;
ii) when Z is N, --- is a double bond and R is (Cₗ-C₆alkoxy)carbonyl or 1-cycloalkyl-tetrazole-5-yl, oxidizing a compound of formula [3]: 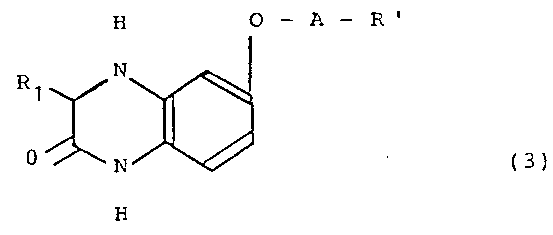 wherein R' is (C₁-C₆alkoxy)carbonyl or 1-cycloalkyl-tetrazole-5-yl and A and R₁ are as defined in claim 1;
iii) when Z is N, --- is a double bond and R is carboxyl, de-esterifying a compound of formula [1 a']: 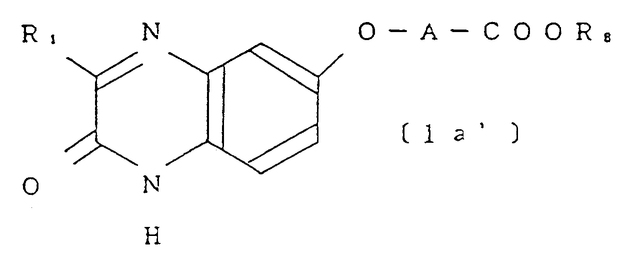 wherein R₈ is C₁-C₅ alkyl and A and R₁ are as defined in claim 1;
iv) when Z is N, --- is a double bond and R is 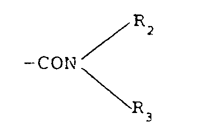 wherein R₂ and R₃ are as defined in claim 1, forming an acid halide or acid anhydride of a compound or formula [1 b]: 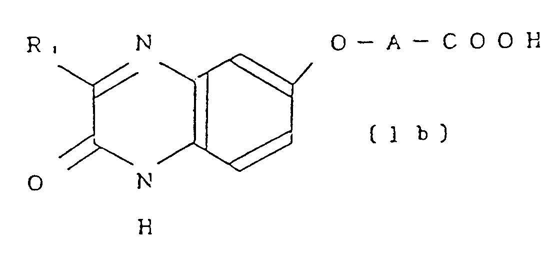 therein A and R₁ are as defined in Claim 1 and reacting the compound thus formed with an amine of formula [6]: 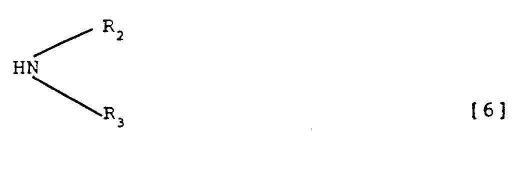 wherein R₂ and R₃ are as defined in Claim 1; or reducing a compound of formula [7]: 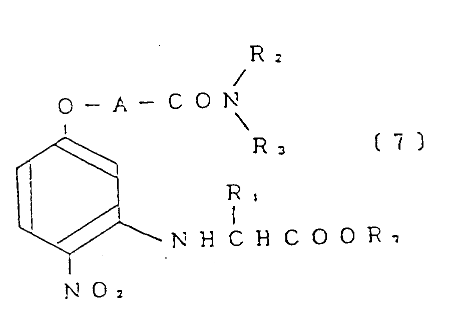 wherein A, R₁, R₂ and R₃ are as defined in Claim 1 and R₇ is C₁-C₆ alkyl, cyclizing the product thus obtained and oxidizing the product obtained by the cyclization;
v) when Z is NH, --- is a single bond and R is carboxyl, de-esterifying a compound of formula [1d']: 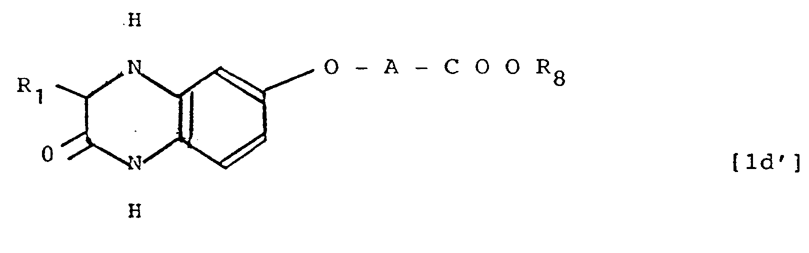 wherein A and R₁ are as defined in Claim 1 and R₈ is C₁-C₆ alkyl; or
vi) when Z is NH, --- is a single bond and R is 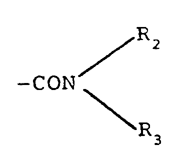 wherein R₂ and R₃ are as defined in Claim 1, forming an acid halide or acid anhydride of a compound of formula [1e]: 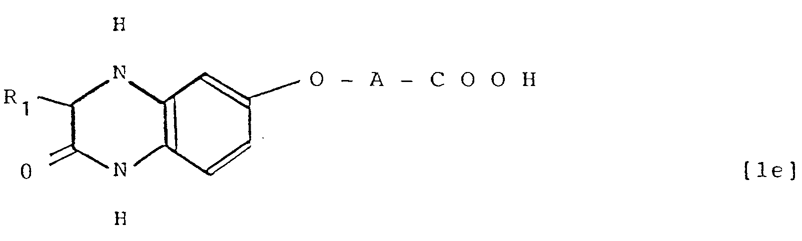 wherein A and R₁ are as defined in Claim 1 and reacting the compound thus formed with an amine of formula [6]: 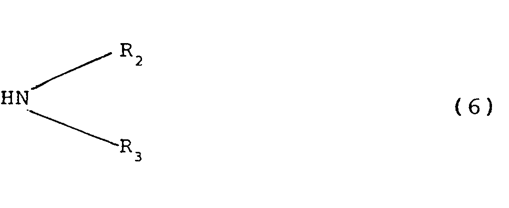 wherein R₂ and R₃ are as defined in Claim 1; or
reducing a compound of formula [7] as defined above and cyclizing the product thus obtained; and if desired converting the resultant compound of formula [I] obtained by any of processes i) to vi) into a pharmacologically acceptable non-toxic salt thereof.

6. A pharmaceutical composition comprising a compound as defined in claim 1 and a pharmaceutically acceptable carrier or diluent.

7. A compound as defined in Claim 1 for use in a method of treatment of the human or animal body by therapy.

8. A compound as defined in Claim 1 for use in a method of treatment of thrombosis or a circulatory condition.

9. Use of a compound as defined in Claim 1 in the manufacture of a medicament for the treatment of thrombosis or a circulatory condition.

## Claims (Claims for the following Contracting State(s) : ES)

1. A process for the preparation of a compound which is a 6-substituted alkoxy-2-oxo-1,2-dihydroquinoxaline derivative of formula (I): 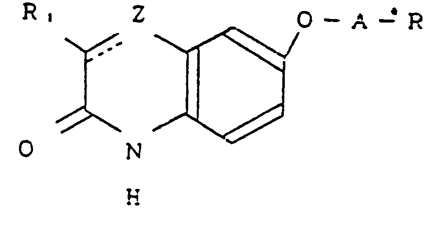 wherein:
Z is N and --- is a double bond or Z is NH and --- is a single bond,
R₁ is hydrogen, branched or unbranched C₁ -₂ₒ alkyl, unsubstituted phenyl or phenyl substituted by at least one substituent selected from C₁₋₃ alkyl, halogen, nitro and C₁-C₆ alkoxy,
A is C₁-C₆ alkylene, and
R is carboxyl, (C₁-C₆ alkoxy)carbonyl, 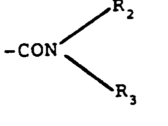 or 1-cycloalkyl-tetrazole-5-yl, wherein R₂ is C₁-C₆ alkyl, hydroxy-C₁-C₆ alkyl or phenyl-C₁-C₆ alkyl, in which the phenyl is unsubstituted or substituted by at least one substituent selected from C₁-C₃ alkyl, halogen, nitro and C₁-C₆ alkoxy, and R₃ is C₁-C₆ alkyl or C₅-C₇ cycloalkyl, or R₂ and R₃, together with the nitrogen to which they are attached, form 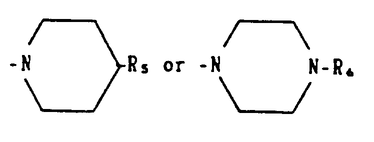 wherein R₅ and R₆ are hydrogen, phenyl or phenyl substituted by at least one substituent selected from C₁-C₃ alkyl, halogen, nitro and C₁-C₆ alkoxy,
or a pharmacologically acceptable non-toxic salt thereof, which process comprises:
i) when Z is NH, --- is a single bond and R is (C₁-C₆ alkoxy)carbonyl or 1-cycloalkyl-tetrazole-5-yl, reducing a compound of formula [2]: 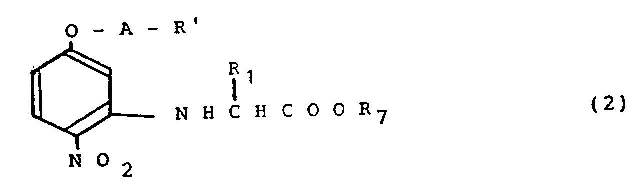 wherein R' is (C₁-C₆ alkoxy)carbonyl or 1-cycloalkyl-tetrazole-5-yl, A and R are as defined above and R₇ is C₁-C₆ alkyl, to convert the nitro group thereof to an amino group and cyclizing the product thus obtained;
ii) when Z is N, --- is a double bond and R is (C₁-C₆ alkoxy)carbonyl or 1-cycloalkyl-tetrazole-5-yl, oxidizing a compound of formula [3]: 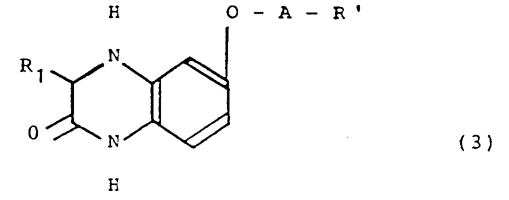 wherein R' is (C₁-C₆ alkoxy)carbonyl or 1-cycloalkyl-tetrazole-5-yl and A and R₁ are as defined above;
iii) when Z is N, --- is a double bond and R is carboxyl, de-esterifying a compound of formula [1 a']: 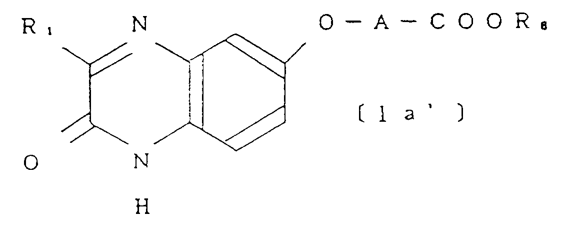 wherein A and R₁ , are as defined above and R₈ is C₁-C₆ alkyl;
iv) when Z is N, --- is a double bond and R is 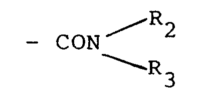 wherein R₂ and R₃ are as defined above forming an acid halide or acid anhydride of a compound of formula [1 b]: 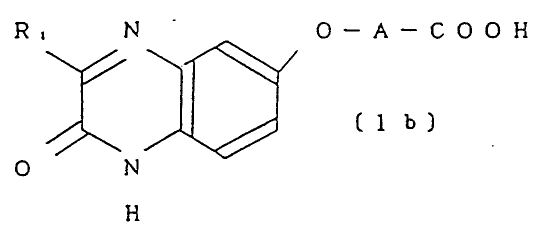 wherein A and R₁ are as defined above and reacting the compound thus formed with an amine of formula [6]: 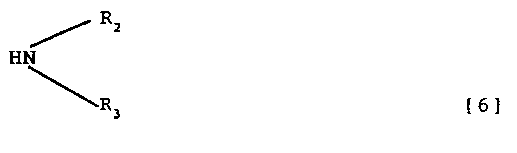 wherein R₂ and R₃ are as defined above; or reducing a compound of formula [7]. 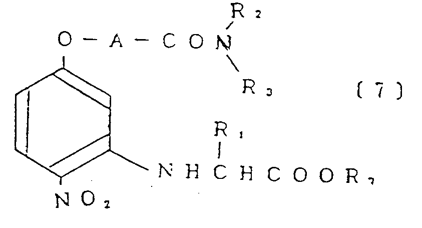 wherein A, Rᵢ, R₂ and R₃ are as defined above and R₇ is C₁-C₆ alkyl, cyclizing the product thus obtained and oxidizing the product obtained by the cyclization;
v) when Z is NH, --- is a single bond and R is carboxyl, de-esterifying a compound of formula [1d']: 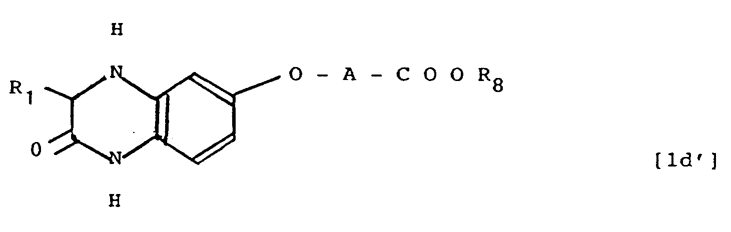 wherein A and R₁ are as defined above and R₈ is C₁-C₆ alkyl; or
vi) when Z is NH, --- is a single bond and R is 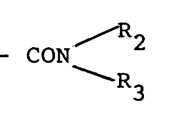 wherein R₂ and R₃ are as defined above, forming an acid halide or acid anhydride of a compound of formula [1 e]: 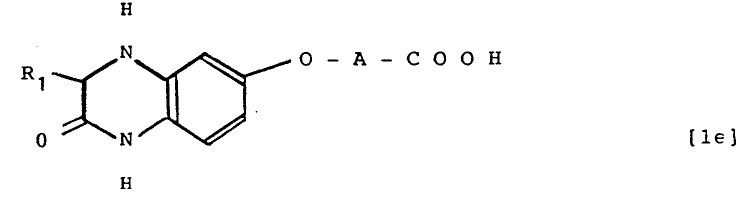 wherein A and R₁ are as defined above and reacting the compound thus formed with an amine of formula [6]: 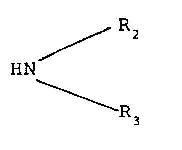 wherein R₂ and R₃ are as defined above; or reducing a compound of formula [7] as defined above and cyclizing the product thus obtained; and if desired converting the resultant compound of formula [1] obtained by any of processes i) to vi) into a pharmacologically acceptable non-toxic salt thereof.

2. A process according to claim 1 (i) which comprises:
i) dissolving the compound of formula [2] in a Cₗ-C₆ alkanol solvent, adding an excess of iron powder to effect the reduction and adding hydrogen chloride with heating to effect the cyclization; or
ii) subjecting the compound of formula [2], dissolved in a C₁-C₆ alkanol solvent, to catalytic hydrogenation using a Pd/C catalyst.

3. A process according to claim 2 (i) which further comprises filtering the reaction product of the cyclization reaction and allowing the ferric chloride produced by the reaction of the iron powder and hydrogen chloride to oxidize the compound of formula [3] as defined in claim 1 produced by the cyclization reaction.

4. A process according to claim 1 (ii) which comprises oxidizing the compound of formula [3] with dichlorodicyanobenzoquinone or with aqueous hydrogen peroxide.

5. A process according to claim 1 (iii) which comprises de-esterifying the compound of formula [1a'], dissolved in a C₁ -C₆ alkanol, with an alkaline hydroxide.

6. A process according to claim 1 (iv) which comprises:
i) reacting the compound of formula [1b] with SOCI₂, PCI₅, POCI₃ or SOBr₂ to form the acid halide thereof or with a carboxylic acid halide in a solvent in the presence of a tertiary organic amine to form a mixed anhydride thereof, and reacting the compound thus formed with the amine of formula [6] at or below room temperature; or
ii) dissolving the compound of formula [7] in a C₁-C₆ alkanol solvent, adding an excess of iron powder to effect the reduction, adding hydrogen chloride whith heating to effect the cyclization and either filtering the reaction product of the cyclization reaction and allowing the ferric chloride produced by the reaction of the iron powder and hydrogen chloride to oxidize the compound produced by the cyclization reaction, or oxidizing the reaction product of the cyclization reaction with dichlorodicyanobenzoquinone or with aqueous hydrogen peroxide.

7. A process according to claim 1 (v) which comprises de-esterifying the compound of formula [1d'], dissolved in a C₁-C₆ alkanol, with an alkaline hydroxide.

8. A process according to claim 1 (vi) which comprises:
i) reacting the compound of formula [1e] with SOCI₂, PCls, POCI₃ or SOBr₂ to form the acid halide thereof or with a carboxylic acid halide in a solvent in the presence of a tertiary organic amine to form a mixed anhydride thereof, and reacting the compound thus formed with the amine of formula [6] at or below room temperature; or
ii) dissolving the compound of formula [7] in a C₁-C₆ alkanol solvent, adding an excess of iron powder to effect the reduction and adding hydrogen chloride with heating to effect the cyclization.

9. A process according to any one of the preceding claims which further comprises mixing the compound of formula [I] or a non-toxic pharmacologically acceptable non-toxic salt thereof with a pharmaceutically acceptable carrier or diluent.

## Patentansprüche (Patentansprüche für folgende(n) Vertragsstaat(en) : CH, DE, FR, GB, IT, LI)

1. Verbindung, bei der es sich um ein 6-substituiertes Alkoxy-2-oxo-1,2-dihydrochinoxalin-Derivat der Formel (I): 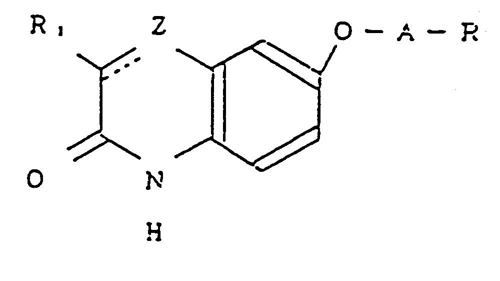 mit den folgenden Bedeutungen handelt:
Z ist N und --- ist eine Doppelbindung oder Z ist NH und --- ist eine Einfachbindung,
R₁ ist Wasserstoff, eine verzweigte oder unverzweigte C₁ -₂₀-Alkylgruppe, eine unsubstituierte Phenylgruppe oder eine Phenylgruppe, die durch mindestens einen Substituenten aus der durch C₁₋₃-Alkylgruppe, Halogenatom, Nitrogruppe und C₁-C₆-Alkoxygruppe gebildeten Gruppe substituiert ist,
A ist eine C₁ -₆-Alkylengruppe und
R ist eine Carboxylgruppe, eine (C₁-₆-Alkoxy)-carbonylgruppe, -CONR2R3 oder 1-Cycloalkyl-tetrazol-5-yl, worin R₂ eine C₁-₆-Alkylgruppe, eine Hydroxy-C₁-₆-alkylgruppe oder eine Phenyl-C₁-₆-alkylgruppe ist, wobei die Phenylgruppe unsubstituiert ist oder durch mindestens einen Substituenten aus der durch C₁₋₃-Alkylgruppe, Halogenatom, Nitrogruppe und C₁₋₆-Alkoxygruppe gebildeten Gruppe substituiert ist, und R₃ eine C₁₋₆Alkylgruppe oder eine C₅₋₇-Cycloalkylgruppe ist oder R₂ und R₃ zusammen mit dem Stickstoffatom, mit dem sie verknüpft sind, 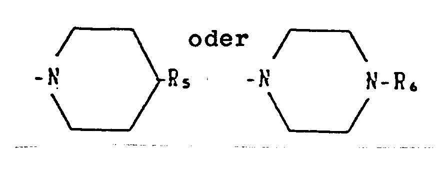 bilden, worin R₅ und R₆ ein Wasserstoffatom, eine Phenylgruppe oder eine Phenylgruppe sind, die durch mindestens einen Substituenten aus der durch C₁₋₃-Alkylgruppe, Halogenatom, Nitrogruppe und C₁ -₆₋Alkoxygruppe gebildeten Gruppe substituiert ist, oder eine pharmakologisch verträgliches nichttoxisches Salz der Verbindung.

2. Verbindung nach Anspruch 1, worin R₁ eine Methyl-, Ethyl-, Propyl-, Isopropyl-, Butyl-, Isobutyl-, sec-Butyl-, t-Butyl-, Pentyl-, Isopentyl-, Hexyl-, Heptyl-, Octyl-, Nonyl-, Decyl-, Undecyl-, Dodecyl-, Tridecyl- , Tetradecyl-, Pentadecyl- oder Hexadecylgruppe ist.

3. Verbindung nach Anspruch 1 oder 2, worin R eine (C₁₋₄-Alkoxy)-carbonylgruppe, 1-(C₅₋₇-Cycloalkyl)-tetrazol-5-yl- oder -CONR₂R₃ ist, worin R₂ eine C₁₋₄-Alkyl-, Hydroxy-(C₁₋₄-alkyl)- oder Phenyl-(C₁₋₄- alkyl)-Gruppe ist, worin die Phenylgruppe unsubstituiert ist oder durch mindestens einen Substituenten aus der durch C₁₋₃-Alkylgruppe, Halogenatom, Nitrogruppe und C₁₋₆-Alkoxygruppe gebildeten Gruppe ist und R₃ eine C₁₋₄-Alkyl- oder C₅₋₇-Cycloalkylgruppe ist oder worin R₂ und R₃ zusammen mit dem Stickstoffatom, mit dem sie verknüpft sind, 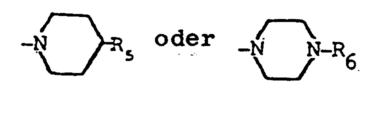 bilden, worin R₅ und R₆ ein Wasserstoffatom oder eine unsubstituierte Phenylgruppe oder eine Phenylgruppe bedeuten, die durch mindestens einen Substituenten aus der durch Cl-3-Alkylgruppe, Halogenatom, Nitrogruppe und C₁₋₆Alkoxygruppe gebildeten Gruppe gewählt sind.

4. Verbindung nach einem der vorhergehenden Ansprüche, wobei die Verbindung in Form eines Hydrochlorids, Sulfats, Phosphats, Acetats, Propionats, Butyrats, Glycolats, Gluconats, Malats, Tartrats, Sukzinats, Mandelats, Glutamats, Aspertats, Methansulfonats oder Toluolsulfonats als Salz vorliegt.

5. Verfahren zur Herstellung einer Verbindung gemäß einem der vorhergehenden Ansprüche, bei dem man
(i) für: Z ist NH, --- ist eine Einfachbindung und R ist eine (C₁₋₆-Alkoxy)-carbonylgruppe oder 1-Cycloalkyl-tetrazol-5-yl; eine Verbindung der Formel [2] 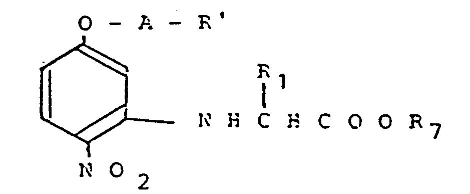 worin R' eine (C₁₋₆-Alkoxy)-carbonylgruppe oder 1-Cycloalkyl-tetrazol-5-yl ist, A und R₁ die Bedeutungen gemäß Anspruch 1 besitzen und R₇ eine C₁₋₆-Alkylgruppe ist, reduziert und die Nitrogruppe in eine Aminogruppe überführt und das zu erhaltende Produkt zyklisiert;
(ii) für: Z ist N, --- ist eine Doppelbindung und R ist eine (C₁₋₆-Alkoxy)-carbonylgruppe oder 1-Cycloalkyl-tetrazol-5-yl; eine Verbindung der Formel [3]: 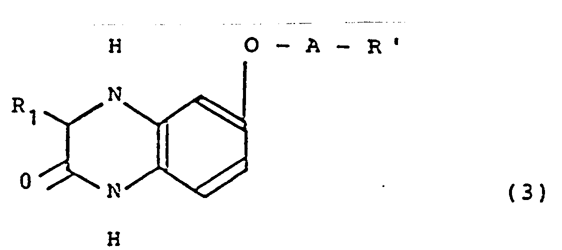 worin R' eine (C₁-₆-Alkoxy)-carbonylgruppe oder 1-Cycloalkyltetrazol-5-yl ist und A und R₁ die Bedeutungen gemäß Anspruch 1 besitzen, oxidiert;
(iii) für: Z ist N, --- ist eine Doppelbindung und R ist eine Carboxylgruppe; eine Verbindung der Formel [1a']: 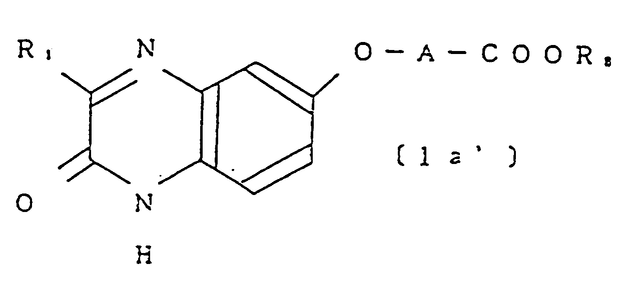 entestert, worin R₈ eine C₁₋₆-Alkylgruppe ist A und R₁ die Bedeutungen gemäß Anspruch 1 besitzen;
(iv) für: Z ist N, --- ist eine Doppelbindung und R ist -CONR₂R₃, worin R₂ und R₃ die Bedeutungen gemäß Anspruch 1 besitzen;
ein Säurehalogenid oder Säureanhydrid einer Verbindung der Formel [1b] bildet: 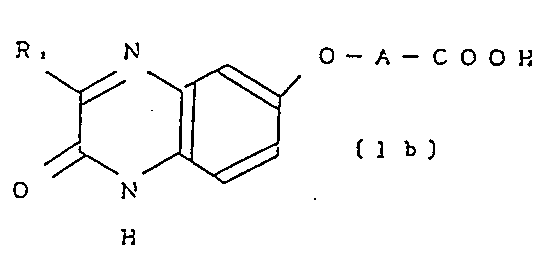 worin A und R₁ die Bedeutungen gemäß Anspruch 1 besitzen, und die so gebildete Verbindung mit einem Amin der Formel [6]: 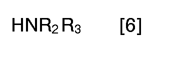 umsetzt, worin R₂ und R₃ die Bedeutungen gemäß Anspruch 1 besitzen; oder eine Verbindung der Formel [7] reduziert: 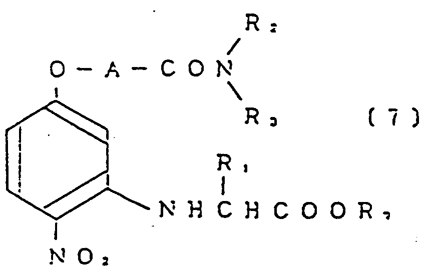 worin A, R₁ , R₂ und R₃ die Bedeutungen gemäß Anspruch 1 besitzen, und R₇ eine Ci-₆-Alkylgruppe ist, das so erhaltene Produkt zyklisiert und das durch Zyklisierung erhaltene Produkt oxydiert;
(v) für: Z ist NH, --- ist eine Einfachbindung und R ist eine Carboxylgruppe; eine Verbindung der Formel [1d']: 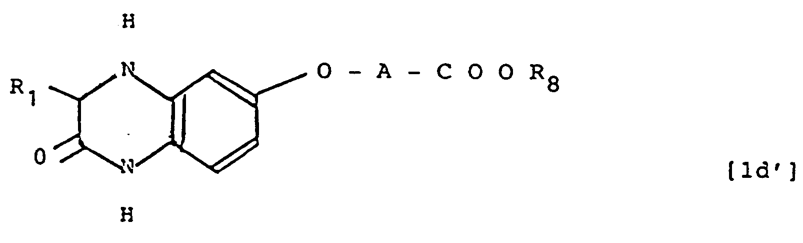 entestert, worin A und R₁ die Bedeutungen gemäß Anspruch 1 besitzen und R₈ eine Ci-₆-Alkylgruppe ist; oder
(vi) für: Z ist NH, --- ist eine Einfachbindung und R ist -CONR₂R₃, worin R₂ und R₃ die Bedeutungen gemäß Anspruchs 1 besitzen;
eine Säurehalogenid oder Säureanhydrid einer Verbindung der Formel [1e] bildet: 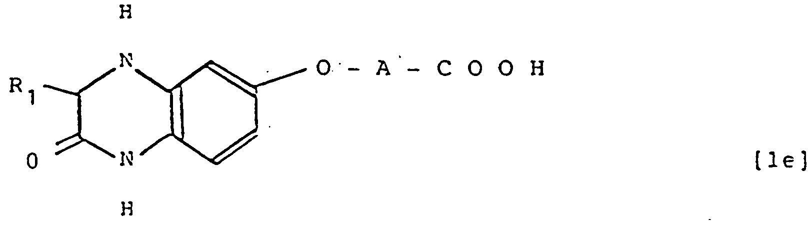 worin A und R₁ die Bedeutungen gemäß Anspruch 1 besitzen, und die so gebildete Verbindung mit einem Amin der Formel [6] umsetzt: 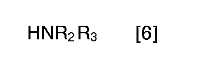 worin R₂ und R₃ die Bedeutungen gemäß Anspruch 1 besitzen; oder eine Verbindung der Formel [7] mit der vorstehenden Kennzeichnung reduziert und das so erhaltene Produkt zyklisiert; und gegebenenfalls
die resultierende Verbindung der Formel [I], die nach einer der Methoden (i) bis (vi) erhalten worden ist, in ein pharmakologisch verträgliches nichttoxisches Salz überführt.

6. Pharmazeutische Zusammensetzung, die eine Verbindung gemäß Anspruch 1 und einen pharmazeutisch verträglichen Träger oder ein pharmazeutisch verträgliches Verdünnungsmittel umfaßt.

7. Verbindung gemäß Anspruch 1 zum Einsatz in einem Verfahren zur therapeutischen Behandlung des menschlichen oder tierischen Körpers.

8. Verbindung gemäß Anspruch 1 zur Verwendung in einem Verfahren zur Behandlung von Thrombose oder des Kreislaufs.

9. Verwendung einer Verbindung gemäß Anspruch 1 bei der Herstellung eines Medikaments zur Behandlung von Thrombose oder des Kreislaufs.

## Patentansprüche (Patentansprüche für folgende(n) Vertragsstaat(en) : ES)

1. Verfahren zur Herstellung einer Verbindung, bei der es sich um ein 6-substituiertes Alkoxy-2-oxo-1,2-dihydrochinoxalin-Derivat der Formel (I): 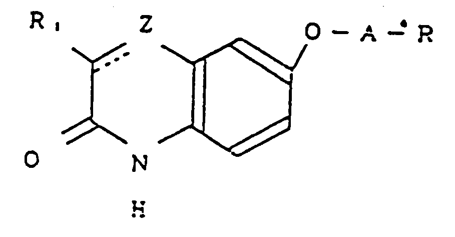 mit den folgenden Bedeutungen handelt:
Z ist N und --- ist eine Doppelbindung oder Z ist NH und --- ist eine Einfachbindung,
R₁ ist Wasserstoff, eine verzweigte oder unverzweigte C₁ -₂₀-Alkylgruppe, eine unsubstituierte Phenylgruppe oder eine Phenylgruppe, die durch mindestens einen Substituenten aus der durch C₁₋₃-Alkylgruppe, Halogenatom, Nitrogruppe und C₁-C₆-Alkoxygruppe gebildeten Gruppe substituiert ist,
A ist eine C₁ -₆-Alkylengruppe und
R ist eine Carboxylgruppe, eine (C₁₋₆-Alkoxy)-carbonylgruppe, -CONR2R3 oder 1-Cycloalkyl-tetrazol-5-yl, worin R₂ eine C₁₋₆-Alkylgruppe, eine Hydroxy-C₁₋₆-alkylgruppe oder eine Phenyl-C₁₋₆-alkylgruppe ist, wobei die Phenylgruppe unsubstituiert ist oder durch mindestens einen Substituenten aus der durch C₁₋₃-Alkylgruppe, Halogenatom, Nitrogruppe und C₁₋₆-Alkoxygruppe gebildeten Gruppe substituiert ist, und R₃ eine C₁₋₆-Alkylgruppe oder eine C₅₋₇-Cycloalkylgruppe ist oder R₂ und R₃ Zusammen mit dem Stickstoffatom, mit dem sie verknüpft sind, 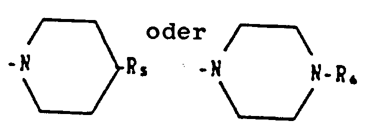 bilden, worin R₅ und R₆ ein Wasserstoffatom, eine Phenylgruppe oder eine Phenylgruppe sind, die durch mindestens einen Substituenten aus der durch C₁₋₃-Alkylgruppe, Halogenatom, Nitrogruppe und C_{1 -6}-Alkoxygruppe gebildeten Gruppe substituiert ist, oder eine pharmakologisch verträgliches nichttoxisches Salz der Verbindung, bei dem man
(i) für: Z ist NH, --- ist eine Einfachbindung und R ist eine (C₁₋₆-Alkoxy)-carbonylgruppe oder 1-Cycloalkyl-tetrazol-5-yl; eine Verbindung der Formel [2] 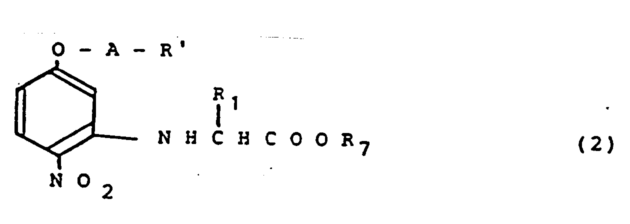 worin R' eine (C₁₋₆-Alkoxy)-carbonylgruppe oder 1-Cycloalkyl-tetrazol-5-yl ist, A und R₁ die vorstehenden Bedeutungen besitzen und R₇ eine C₁₋₆-Alkylgruppe ist, reduziert und die Nitrogruppe in eine Aminogruppe überführt und das zu erhaltende Produkt zyklisiert;
(ii) für: Z ist N, --- ist eine Doppelbindung und R ist eine (C₁₋₆-Alkoxy)-carbonylgruppe oder 1-Cycloalkyl-tetrazol-5-yl; eine Verbindung der Formel [3]: 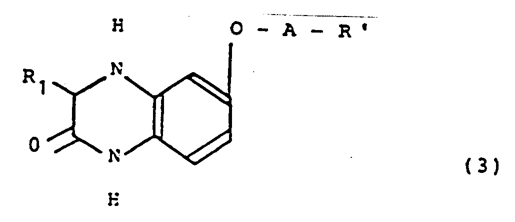 worin R' eine (C₁-₆-Alkoxy)-carbonylgruppe oder 1-Cycloalkyl-tetrazol-5-yl ist und A und R₁ die Bedeutungen gemäß Anspruch 1 besitzen, oxidiert;
(iii) für: Z ist N, --- ist eine Doppelbindung und R ist eine Carboxylgruppe; eine Verbindung der Formel [1a']: 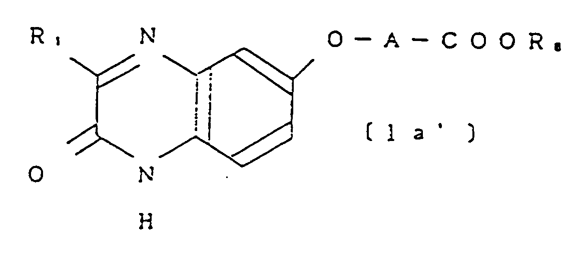 entestert, worin A und R₁ die vorstehenden Bedeutungen besitzen und R₈ eine C₁₋₆-Alkylgruppe ist;
(iv) für: Z ist N, --- ist eine Doppelbindung und R ist -CONR₂R₃, worin R₂ und R₃ die vorstehenden Bedeutungen besitzen; ein Säurehalogenid oder Säureanhydrid einer Verbindung der Formel [1b] bildet: 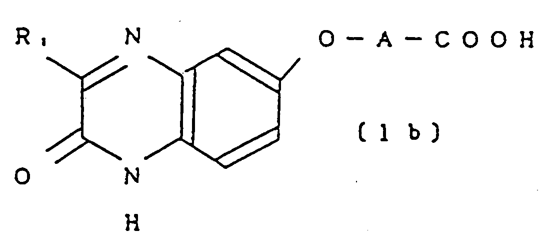 worin A und R₁ die vorstehenden Bedeutungen besitzen und die so gebildete Verbindung mit einem Amin der Formel [6]: 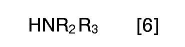 umsetzt, worin R₂ und R₃ die vorstehenden Bedeutungen besitzen; oder eine Verbindung der Formel [7] reduziert: 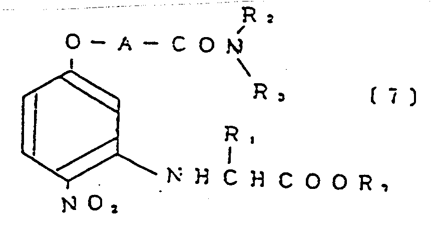 worin A, R₁ , R₂ und R₃ die vorstehenden Bedeutungen besitzen und R₇ eine C₁₋₆-Alkylgruppe ist, das so erhaltene Produkt zyklisiert und das durch Zyklisierung erhaltene Produkt oxydiert;
(v) für: Z ist NH, --- ist eine Einfachbindung und R ist eine Carboxylgruppe; eine Verbindung der Formel [1d']: 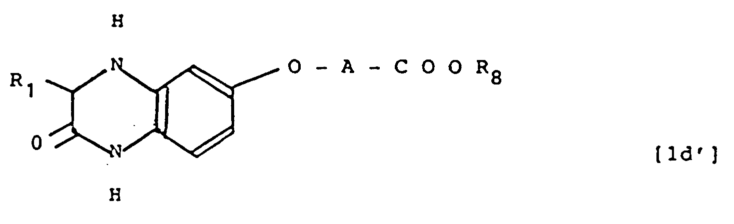 entestert, worin A und R₁ die vorstehenden Bedeutungen besitzen und R₈ eine C₁₋₆-Alkylgruppe ist; oder
(vi) für: Z ist NH, --- ist eine Einfachbindung und R ist -CONR₂R₃, worin R₂ und R₃ die Bedeutungen gemäß Anspruchs 1 besitzen, eine Säurehalogenid oder Säureanhydrid einer Verbindung der Formel [1e] bildet: 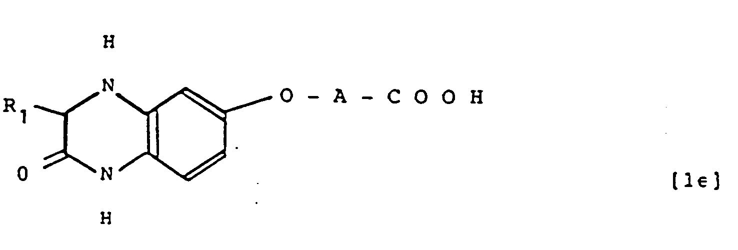 worin A und R₁ die Bedeutungen gemäß Anspruch 1 besitzen, und die so gebildete Verbindung mit einem Amin der Formel [6] umsetzt: 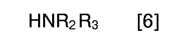 worin R₂ und R₃ die vorstehenden Bedeutungen besitzen; oder eine Verbindung der Formel [7] mit der vorstehenden Kennzeichnung reduziert und das so erhaltene Produkt zyklisiert; und gegebenenfalls
die resultierende Verbindung der Formel [I], die nach einer der Methoden (i) bis (vi) erhalten worden ist, in ein pharmakologisch verträgliches nichttoxisches Salz überführt.

2. Verfahren nach Anspruch 1 (i), bei dem man
(i) die Verbindung der Formel [2] in einem Ci-₆-Alkanol-Lösungsmittel löst, einen Überschuß Eisenpulver zur Reduktion zugibt und Chlorwasserstoff unter Erhitzen zur Zyklisierung zugibt; oder
(ii) die Verbindung der Formel [2], die in einem Ci-₆-Alkanol-Lösungsmittel gelöst ist, einer katalytischen Hydrierung mit einem Pd/C-Katalysator unterwirft.

3. Verfahren nach Anspruch 2 (i), bei dem man ferner das Reaktionsprodukt der Zyklisierungsreaktion abfiltiert und vorsieht, daß das Fe-III-Chlorid, das durch die Umsetzung des Eisenpulvers mit Chlorwasserstoff gebildet wurde, die Verbindung der Formel [3] gemäß Anspruch 1 oxidiert, die durch die Zyklisierungsreaktion gebildet wurde.

4. Verfahren nach Anspruch 1 (ii), bei dem man die Verbindung der Formel [3] mit Dichlordicyanobenzochinon oder mit wässerigem Wasserstoffperoxid oxidiert.

5. Verfahren nach Anspruch 1 (iii), bei dem man die Verbindung der Formel [1a'], die in einem Ci-₆-Alkanol gelöst ist, mit einem alkalischen Hydroxid-entestert.

6. Verfahren nach Anspruch 1 (iv), bei dem man
(i) die Verbindung der Formel [1b] mit SOCI₂, PCI₅, POCI₃ oder SOBr₂ umsetzt und deren Säurehalogenid bildet oder mit einem Carbonsäurehalogenid in einem Lösungsmittel in Gegenwart eines tertiären organischen Amin umsetzt und sein Mischanhydrid bildet und die auf diese Weise gebildete Verbindung mit dem Amin der Formel [6] bei oder unterhalb Raumtemperatur umsetzt; oder
(ii) die Verbindung der Formel [7] in einem C₁-₆-Alkanol-Lösungsmittel löst, einen Überschuß Eisenpulver zur Reduktion zugibt, Chlorwasserstoff unter Erwärmen zur Zyklisierung zugibt und entweder das Reaktionsprodukt der Zyklisierungsreaktion abfiltriert und das Fe-III-Chlorid, das durch die Umsetzung des Eisenpulvers mit dem Chlorwasserstoff gebildet wurde, die Verbindung oxidieren läßt, die durch die Zyklisierungsreaktion gebildet wurde, oder das Reaktionsprodukt der Zyklisierungsreaktion mit Dichlordicyanobenzochinon oder mit wässerigem Wasserstoffperoxid oxidiert.

7. Verfahren nach Anspruch 1 (v), bei dem man die Verbindung der Formel [1d'], die in einem Ci-₆-Alkanol gelöst ist, mit einem alkalischen Hydroxid entestert.

8. Verfahren nach Anspruch 1 (vi), bei dem man
(i) die Verbindung der Formel [1e] mit SOCI₂, PCI₅, POCI₃ oder SOBr₂ zur Bildung seines Säurehalogenids oder mit einem Carbonsäurehalogenid in einem Lösungsmittel in Gegenwart eines tertiären organischen Amins zur Bildung seines Mischanhydrids umsetzt und die so gebildete Verbindung mit dem Amin der Formel [6] bei oder unterhalb Raumtemperatur umsetzt; oder
(ii) die Verbindung der Formel [7] in einem C₁-₆-Alkanol-Lösungsmittel löst, einen Überschuß Eisenpulver zur Reduktion zugibt und Chlorwasserstoff unter Erhitzen zur Zyklisierung zugibt.

9. Verfahren nach einem der vorhergehenden Ansprüche, bei dem man ferner die Verbindung der Formel [I] oder eines ihrer nichttoxischen pharmakologisch verträglichen Salze mit einem pharmazeutisch verträglichen Träger oder einem pharmazeutisch verträglichen Verdünnungsmittel mischt.

## Revendications (Revendications pour l'(les) Etat(s) contractant(s) suivant(s) : suivants : CH, DE, FR, GB, IT, LI)

1. Composé qui est un dérivé de 2-oxo-1,2-dihydroquinoxaline substitué en position 6 par un alcoxy de formule (1) : 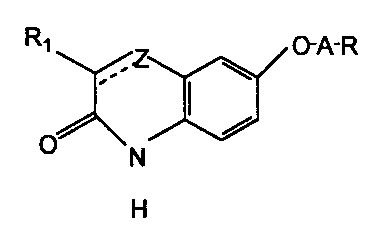 où :
Z est N et --- est une double liaison ou Z est NH et --- est une liaison simple,
R₁ est un hydrogène, un alkyle en C₁₋₂₀ ramifié, un phényle non substitué ou un phényle substitué par au moins un substituant choisi parmi un alkyle en C₁₋₃, un nitro et un alcoxy en C₁₋C₆,
A est un alkylène en C₁-C₆, et
R est un carboxyle, un (alcoxy en C₁-C₆) carbonyle, 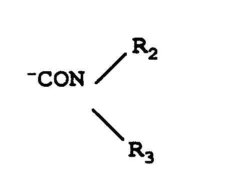 ou un 1-cycloalkyl-tétrazole-5-yl, où R₂ est
un alkyle en C₁-C₆, un hydroxy-alkyle en C₁-C₆, ou un phényl-alkyle en C₁-C₆, dans lequel le phényle est non substitué ou substitué par au moins un substituant choisi parmi un alkyle en C₁-C₃, un halogène, un nitro et un alcoxy en C₁-C₆, et R₃ est un alkyle en Ci-Ce ou un cycloalkyle en C₅-C₇, ou R₂ et R₃, ensemble avec l'azote auquel ils sont attachés, forment 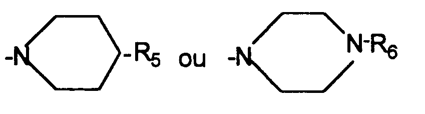 où R₅ et R₆ sont un hydrogène, un phényle ou un phényle substitué par au moins un substituant choisi parmi un alkyle en C₁-C₃, un halogène, un nitro ou un alcoxy en C₁-C₆,
ou un sel non toxique pharmacologiquement acceptable de ceux-ci.

2. Composé selon la revendication 1, dans lequel R₁ est un méthyle, un éthyle, un propyle, un isopropyle, un butyle, un isobutyle, un sec-butyle, un t-butyle, un pentyle, un isopentyle, un hexyle, un heptyle, un octyle, un nonyle, un décyle, un undécyle, un dodécyle, un tridécyle, un tétradécyle, un pentadécyle ou un hexadécyle.

3. Composé selon la revendication 1 ou 2, dans laquelle R est un (alcoxy en C₁-C₄) carbonyle, un 1-(cycloalkyle en C₅₋₇)-tétrazole-5-yle ou 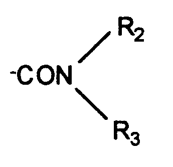 où R₂ est un alkyle en C₁₋₄, un hydroxy (alkyle en C₁₋₄) ou un phényl (alkyle en C₁₋₄) où le phényl est non substitué ou substitué par au moins un substituant choisi parmi un alkyle en C₁₋₃, un halogène, un nitro et un alcoxy en C₁₋C₆ et R₃ est un alkyle en C₁₋₄ ou un cycloalkyle en C₅₋₇ où R₂ et R₃ ensemble avec l'azote auquel ils sont attachés, forment 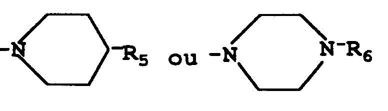 oùRₛ etR₆ sont un hydrogène ou un phényle non substitué ou substitué par au moins un substituant choisi parmi un alkyle en C₁₋₃, un halogène, un nitro, un alcoxy en C₁-C₆.

4. Composé selon l'une quelconque des revendications précédentes qui est sous la forme d'un hydroc- hlorure, d'un sulfate, d'un phosphate, d'un acétate, d'un propionate, d'un butyrate, d'un glycolate, d'un gluconate, d'un malate, d'un tartrate, d'un succinate, d'un mandelate, d'un glutamate, d'un aspartate, d'un méthanesulfonate ou d'un toluènesulfonate.

5. Procédé pour la préparation d'un composé tel que défini dans l'une quelconque des revendications précédentes qui comprend :
i) lorsque Z est NH, --- est une simple liaison et R est un (alcoxy en C₁-C₆) carbonyle ou un 1-cycloalkyl-tétrazole-5-yle, la réduction d'un composé de formule [2] : 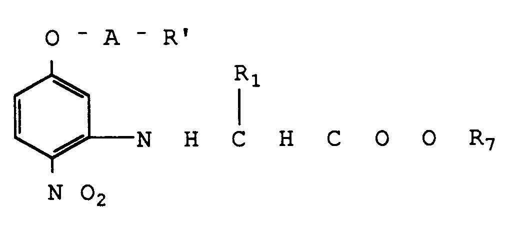 dans laquelle R' est un (alcoxy en C₁-C₆) carbonyle ou un 1-cycloalkyl-tétrazole-5-yle, A et R₁ sont tels que définis en revendication 1 et R₇ est un alkyle en C₁-C₆, pour convertir le groupe nitro de celle-ci en un groupe amino, et la cyclisation du produit ainsi obtenu;
ii) lorsque Z est N, --- est une double liaison et R est un (alcoxy en C₁-C₆) carbonyle ou un 1-cycloakyl-tétrazole-5-yle, l'oxydation d'un composé de formule [3] : 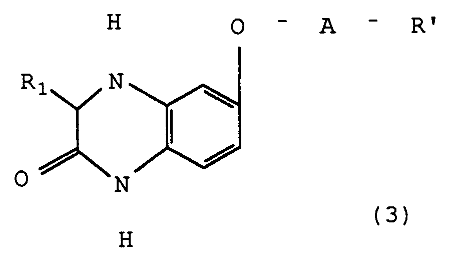 dans laquelle R' est un (alcoxy en C₁-C₆) carbonyle ou un 1-cycloalkyl-tétrazole-5-yle et A et R₁ sont tels que définis en revendication 1;
iii) lorsque Z est N, --- est une double liaison et R est un carboxyle, la dé-esterification du composé de formule [1a'] : 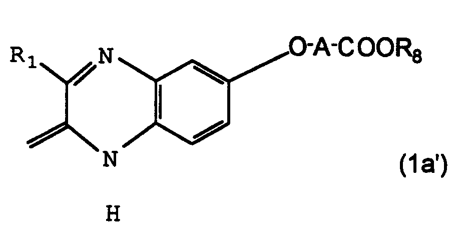 dans laquelle R₈ est un alkyle en C₁-C₆ et A et R₁ sont tels que définis en revendication 1; iv) lorsque Z est N, --- est une double liaison et R est 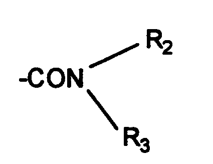 où R₂ et R₃ sont tels que définis en revendication 1, la formation d'un halogénure d'acide ou d'un anhydride d'acide d'un composé de formule [1 b] : 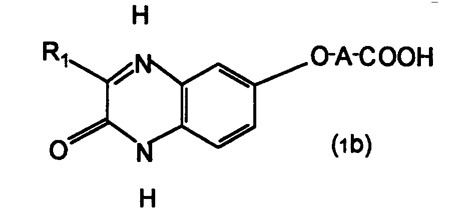 où A et R₁ sont tels que définis en revendication 1 et la réaction du composé ainsi formé avec une amine de formule [6] : 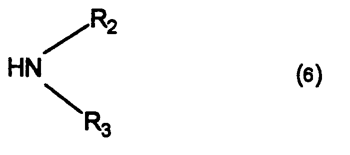 où R₂ et R₃ sont tels que définis en revendication 1; ou la réduction d'un composé de formule [7] : 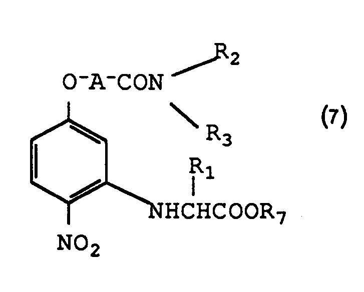 où A, R₁ , R₂ et R₃ sont tels que définis en revendication 1 et R₇ est un alkyle en Ci -C₆, la cyclisation du produit ainsi obtenu et l'oxydation du produit obtenu par la cyclisation;
v) lorsque Z est NH, --- est une simple liaison et R est un carboxyle, la dé-esterification d'un composé de formule [1d'] : 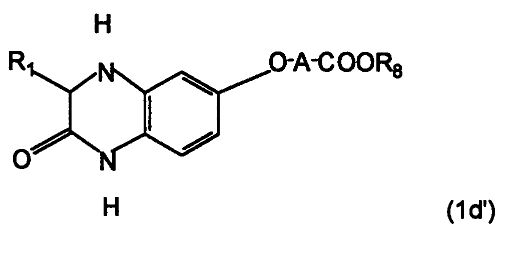 où A et R₁ sont tels que définis en revendication 1 et R₈ est un alkyle en C₁-C₆, ou vi) lorsque Z est NH, --- est une simple liaison et R est 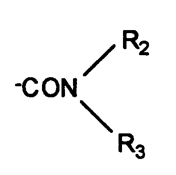 où R₂ et R₃ sont tels que définis en revendication 1, la formation d'un halogénure d'acide ou d'un anhydride d'acide d'un composé de formule [1e] : 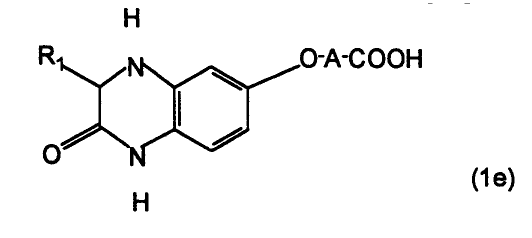 où A et R₁ sont tels que définis en revendication 1 et la réaction du composé ainsi formé avec une amine de formule [6] : 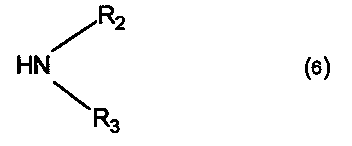 où R₂ et R₃ sont tels que définis en revendication 1; ou
la réduction d'un composé de formule [7] telle que définie ci-dessus et la cyclisation du produit ainsi obtenu; et si désiré la conversion du composé résultant de formule [I] obtenu par l'un des procédés i) à vi) en un sel non toxique pharmacologiquement acceptable de celui-ci.

6. Composition pharmaceutique comprenant un composé tel que défini en revendication 1 et un diluant ou un porteur pharmaceutiquement acceptable.

7. Composé selon la revendication 1 pour utilisation dans une méthode de traitement du corps humain ou animal par thérapie.

8. Composé selon la revendication 1 pour utilisation dans une méthode de traitement de la thrombose ou d'une maladie circulatoire.

9. Utilisation d'un composé selon la revendication 1, dans la fabrication d'un médicament pour le traitement de la thrombose ou d'une maladie circulatoire.

## Revendications (Revendications pour l'(les) Etat(s) contractant(s) suivant(s) : suivant : ES)

1. Procédé pour la préparation d'un composé qui est un dérivé de 2-oxo-1,2-dihydroquinoxaline substitué en position 6 par un alcoxy de formule (l) : 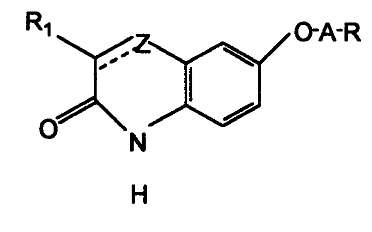 dans laquelle :
Z est N et --- est une double liaison ou Z est NH et --- est une simple liaison,
R₁ est un hydrogène, un alkyle en Ci -₂ₒ ramifié ou non ramifié, un phényle non substitué ou un phényle substitué par au moins un substituant choisi parmi un alkyle en C₁₋₃, un halogène, un nitro, et un alcoxy en Ci -C₆,
A est un alkylène en C₁-C₆, et
R est un carboxyle, un (alcoxy en C₁-C₆) carbonyle, 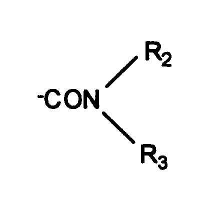 ou un 1-cycloalkyl-tétrazole-5-yle, où R₂ est un alkyle en C₁-C₆, un hydroxy alkyle en Ci-Ce ou un phényl alkyle en C₁-C₆, dans lequel le phényle est non substitué ou substitué par au moins un substituant choisi parmi un alkyle en C₁-C₃,un halogène, un nitro et un alcoxy en C₁-C₆, et R₃ est un alkyle en Ci-Ce ou un cycloalkyle en C₅-C₇, ou R₂ et R₃, ensemble avec l'azote auquel ils sont attachés, forment 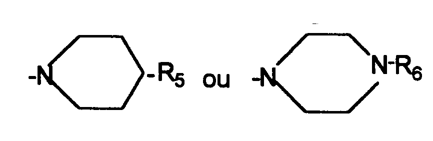 où R₅ et R₆ sont un hydrogène, un phényle ou un phényle substitué par au moins un substituant choisi parmi un alkyle en C₁-C₃, un halogène, un nitro et un alcoxy en C₁-C₆,
ou un sel non toxique pharmacologiquement acceptable de celui-ci, lequel procédé comprend :
i) lorsque Z est NH, --- est une simple liaison et R est un (alcoxy en C₁-C₆) carbonyle ou un 1-cycloalkyl-tétrazole-5-yle, la réduction d'un composé de formule [2] 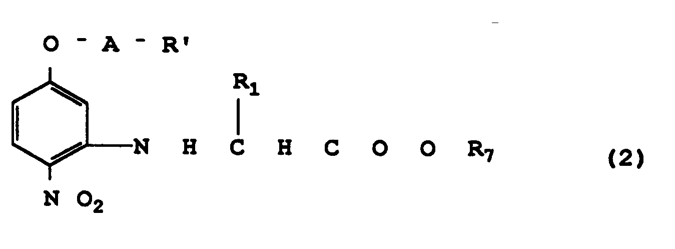 où R' est un (alcoxy en C₁-C₆) carbonyle ou un 1-cycloalkyl-tétrazole-5-yle, A et R sont tels que définis ci-dessus et R₇ est un alkyle en C₁-C₆, pour convertir le groupe nitro de celui-ci en un groupe amino et la cyclisation du produit ainsi obtenu;
ii) lorsque Z est N, --- est une double liaison et R est un (alcoxy en C₁-C₆) carbonyle ou un 1-cycloalkyl-tétrazole-5-yl, l'oxydation d'un composé de formule [3] : 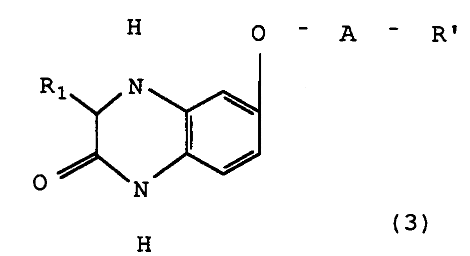 où R' est un (alcoxy en C₁-C₆) carbonyle ou un 1-cycloakyl-tétrazole-5-yle et A et R₁ sont tels que définis ci-dessus;
ii) lorsque Z est N, --- est une double liaison et que R est un carboxyle, la dé-estérification d'un composé de formule [1a'] : 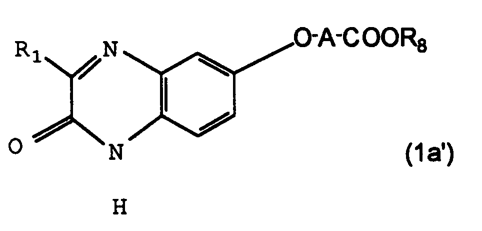 où A et R₁ , sont tels que définis ci-dessus et R₈ est un alkyle en C₁-C₆;
iv) lorsque Z est N, que --- est une double liaison et que R est 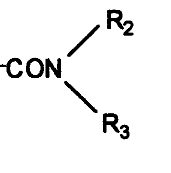 où R₂ et R₃ sont tels que définis ci-dessus la formation d'un halogénure d'acide ou d'un anhydride d'acide d'un composé de la formule [1 b] : 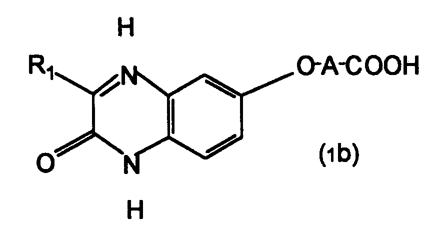 où A et R₁ sont tels que définis ci-dessus et la réaction du composé ainsi formé avec une amine de formule [6] : 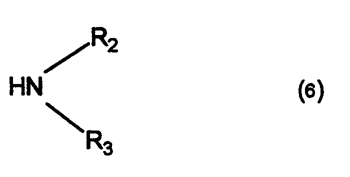 où R₂ et R₃ sont tels que définis ci-dessus; ou la réduction d'un composé de formule [7] : 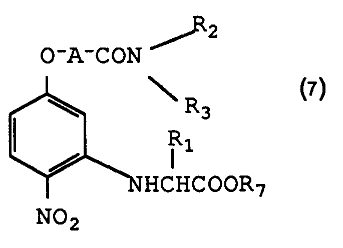 où A, R₁ , R₂ et R₃ sont tels que définis ci-dessus et R₇ est un alkyle en C₁-C₆, la cyclisation du produit ainsi obtenu et l'oxydation du produit obtenu par la cyclisation;
v) lorsque Z est NH, --- est une double liaison et R est un carboxyle, la dé-estérification d'un composé de formule [1d'] : 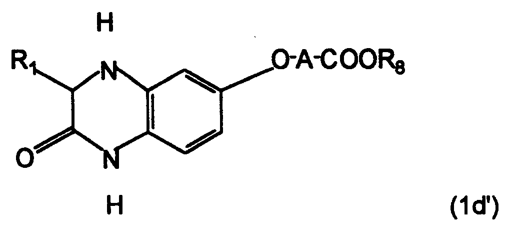 où A et R₁ sont tels que définis ci-dessus et R₈ est un alkyle en C₁-C₆; ou
vi) lorsque Z est NH, --- est une liaison simple et R est 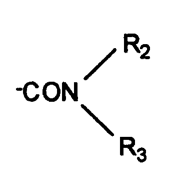 où R₂ et R₃ sont tels que définis ci-dessus, la formation d'un halogénure d'acide ou d'un anhydride d'acide d'un composé de formule [1 e] : 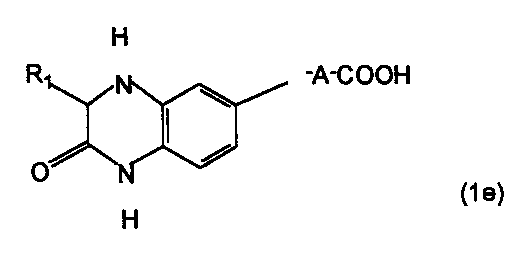 où A et R₁ sont comme définis ci-dessus et la réaction du composé ainsi formé avec une amine de formule [6] : 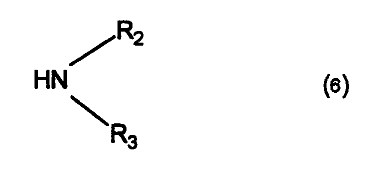 où R₂ et R₃ sont tels que définis ci-dessus; ou la réduction d'un composé de formule [7] tel que défini ci-dessus et la cyclisation du produit ainsi obtenu;
et si souhaité la conversion du composé résultant de formule [1 ] obtenu par l'un quelconque des procédés i) à vi) en un sel non toxique pharmacologiquement acceptable de celui-ci.

2. Procédé selon la revendication 1 (i) qui comprend :
i) la dissolution du composé de formule [2] dans un solvant alcanol en Ci-Ce, l'addition d'un excès de poudre de fer pour effectuer la réduction et l'addition de chlorure d'hydrogène avec chauffage pour effectuer la cyclisation; ou
ii) la soumission du composé de formule [2], dissous dans un solvant alcanol en C₁-C₆, à une hydrogénation catalytique en utilisant un catalyseur Pd/c.

3. Procédé selon la revendication 2 (i) qui comprend de plus les étapes de filtrer le produit de réaction de la réaction de cyclisation et de laisser le chlorure ferrique produit par la réaction de la poudre de fer et du chlorure d'hydrogène oxyder le composé de formule [3] tel que défini en revendication 1 produit par la réaction de cyclisation.

4. Procédé selon la revendication 1 (ii) qui comprend l'oxydation du composé de formule [3] avec du dichlorodicyanobenzoquinone ou avec un peroxyde d'hydrogène aqueux.

5. Procédé selon la revendication 1 (iii) qui comprend la dé-estérification du composé de formule [1a'] dissous dans un alcanol C₁-C₆, avec un hydroxyde alcalin.

6. Procédé selon la revendication 1 (iv) qui comprend :
i) la réaction du composé de formule [1b] avec SOCI₂, PCI₅, POCI₃ ou SOBr₂ pour former l'halogénure d'acide de celui-ci ou avec un halogénure d'acide carboxylique dans un solvant en présence d'une amine organique tertiaire pour former un anhydride mixte de celui-ci, et la réaction du composé ainsi formé avec l'amine de formule [6] à ou en dessous de la température ambiante; ou
ii) la dissolution du composé de formule [7] dans un solvant alcanol en Ci-Ce, l'ajout d'un excès de poudre de fer pour effectuer la réduction, l'ajout de chlorure d'hydrogène avec chauffage pour effectuer la cyclisation et soit filtrer le produit réactionnel de la réaction de cyclisation et laisser le chlorure ferrique produit par la réaction de la poudre de fer et du chlorure d'hydrogène oxyder le composé produit par la réaction de cyclisation, soit oxyder le produit réactionnel de la réaction de cyclisation avec du dichlorodicyanobenzoquinone ou avec du peroxyde d'hydrogène aqueux.

7. Procédé selon la revendication 1 (v) qui comprend la dé-estérification du composé de formule [1d'] dissous dans un alcanol en Ci-Ce, avec un hydroxyde alcalin.

8. Procédé selon la revendication 1 (vi) qui comprend :
i) la réaction du produit de formule [1e] avec SOCI₂, PCls, POCI₃ ou SOBr₂ pour former l'halogénure d'acide de celui-ci ou avec un halogénure d'acide carboxylique dans un solvant en présence d'une amine organique tertiaire pour former un anhydride mixte de celui-ci, et la réaction du composé ainsi formé avec l'amine de formule [6] à ou en dessous de la température ambiante; ou
ii) la dissolution du composé de formule [7] dans un solvant alcanol en Ci-Ce, l'ajout d'un excès de poudre de fer pour effectuer la réduction et l'addition de chlorure d'hydrogène avec chauffage pour effectuer la cyclisation.

9. Procédé selon l'une quelconque des revendications précédentes qui comprend de plus le mélange du composé de formule [I] ou d'un sel non toxique pharmacologiquement acceptable de celui-ci avec un diluant ou porteur pharmaceutiquement acceptable.
